# EUROPEAN PATENT APPLICATION

(11) **EP 4 663 189 A2**
(43) Date of publication of application: **17.12.2025**
(21) Application number: 25206111.4
(22) Date of filing: 03.03.2021
(51) Int. Cl.: A61K 31/424

(54) **FUSED TRICYCLIC COMPOUND AND MEDICINAL USE THEREOF**

(30) Priority: 04.03.2020 JP 2020036931; 07.01.2021 JP 2021001452
(62) Divisional of application: 21764169.5
(71) Applicant: Japan Tobacco Inc., Tokyo 105-6927 (JP)
(72) Inventor: SUZAWA, Koichi, Osaka, 569-1125 (JP); FUJISHIMA, Yuki, Osaka, 569-1125 (JP); YAMAKAWA, Maki, Osaka, 569-1125 (JP); UENO, Hiroshi, Osaka, 569-1125 (JP); MANABE, Tomoyuki, Osaka, 569-1125 (JP)
(74) Representative: Vossius & Partner Patentanwälte Rechtsanwälte mbB

(57) **Abstract**

The present invention provides a compound having a PDHK inhibitory activity and useful for the treatment or prophylaxis of diabetes (type 1 diabetes, type 2 diabetes etc.), insulin resistance syndrome, metabolic syndrome, hyperglycemia, hyperlactacidemia, diabetic complications (diabetic neuropathy, diabetic retinopathy, diabetic nephropathy, cataract etc.), cardiac failure (acute cardiac failure, chronic cardiac failure), cardiomyopathy, myocardial ischemia, myocardial infarction, angina pectoris, dyslipidemia, atherosclerosis, peripheral artery disease, intermittent claudication, chronic obstructive pulmonary disease s, brain ischemia, cerebral apoplexy, mitochondrial disease, mitochondrial encephalomyopathy, cancer, pulmonary hypertension, Alzheimer disease, vascular dementia, glaucoma, diabetic retinopathy, retinopathy of prematurity, retinal vein occlusion, ischemic optic neuropathy or chronic kidney disease. The present invention relates to a compound of the formula [I-a], or a pharmaceutically acceptable salt thereof: 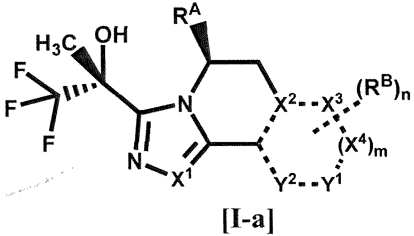 wherein each symbol is as defined in the DESCRIPTION.

## Description

### Technical Field

The present invention relates to a fused tricyclic compound and a pharmaceutical use thereof. More particularly, the present invention relates to a fused tricyclic compound or a pharmaceutically acceptable salt thereof having a pyruvate dehydrogenase kinase (hereinafter to be abbreviated as PDHK) inhibitory activity, a pharmaceutical composition containing the same, a therapeutic or prophylactic agent containing the same for diabetes (type 1 diabetes, type 2 diabetes etc.), insulin resistance syndrome, metabolic syndrome, hyperglycemia, hyperlactacidemia, diabetic complications (diabetic neuropathy, diabetic retinopathy, diabetic nephropathy, cataract etc.), cardiac failure (acute cardiac failure, chronic cardiac failure), cardiomyopathy, myocardial ischemia, myocardial infarction, angina pectoris, dyslipidemia, atherosclerosis, peripheral arterial disease, intermittent claudication, chronic obstructive pulmonary disease, brain ischemia, cerebral apoplexy, mitochondrial disease, mitochondrial encephalomyopathy, cancer, pulmonary hypertension, Alzheimer disease, vascular dementia, glaucoma, diabetic retinopathy, retinopathy of prematurity, retinal vein occlusion, ischemic optic neuropathy, or chronic kidney disease, and the like.

### Background Art

In tissues, for reactions using energy such as biosynthesis, active transport, muscle contraction and the like, the energy is supplied by hydrolysis of adenosine triphosphate (ATP). ATP is produced by oxidation of metabolic fuel which yields much energy, such as glucose and free fatty acids. In oxidative tissues such as muscle, ATP is mostly produced from acetyl-CoA that enters citric acid cycle. Acetyl-CoA is produced by oxidation of glucose via glycolytic pathway or β oxidation of free fatty acid. An enzyme that plays a pivotal role in controlling acetyl-CoA production from glucose is pyruvate dehydrogenase (hereinafter to be abbreviated as PDH). PDH catalyzes reduction of nicotinamide adenine dinucleotide (NAD) to NADH, simultaneously with oxidation of pyruvic acid to acetyl-CoA and carbon dioxide (e.g., non-patent documents 1, 2).

PDH is a multienzyme complex consisting of three enzyme components (E1, E2 and E3) and some subunits localized in mitochondrial matrix. E1, E2 and E3 are responsible for decarboxylation from pyruvic acid, production of acetyl-CoA and reduction of NAD to NADH, respectively.

Two classes of enzyme having regulatory function bind to PDH. One is PDHK, which is a protein kinase having specificity to PDH. The role thereof is to inactivate E1α subunit of the PDH complex by phosphorylation. The other is PDH phosphatase, which is a specific protein phosphatase that activates PDH via dephosphorylation of E1α subunit. The proportion of PDH in its active (dephosphorylated) state is determined by the balance of kinase activity and phosphatase activity. The kinase activity is regulated by the relative concentration of metabolic substrates. For example, the kinase activity is activated by an increase in NADH/NAD, acetyl-CoA/CoA and ATP/adenosine diphosphate (ADP) ratios, and inhibited by pyruvic acid (e.g., non-patent document 3).

In the tissues of mammals, 4 kinds of PDHK isozymes are identified. Particularly, PDHK2 is expressed in a wide range of tissues including the liver, skeletal muscles and adipose tissues involved in glucose metabolism. Furthermore, since PDHK2 shows comparatively high sensitivity to activation by increased NADH/NAD or acetyl-CoA/CoA and inhibition by pyruvic acid, involvement in a short-term regulation of glucose metabolism is suggested (e.g., non-patent document 4).

In addition, PDHK1 is expressed in large amounts in cardiac muscle, skeletal muscle, pancreatic β cell and the like. Furthermore, since expression of PDHK1 is induced via activation of hypoxia inducible factor (HIF) 1 in ischemic state, its involvement in ischemic diseases and cancerous diseases is suggested (e.g., non-patent document 5).

In diseases such as insulin-dependent (type 1) diabetes, non-insulin-dependent (type 2) diabetes and the like, oxidation of lipids is promoted with simultaneous reduction in glucose utilization. This reduction in glucose utilization is one of the factors causing hyperglycemia. When the oxidative glucose metabolism decreases in type 1 and type 2 diabetes and obesity, PDH activity also decreases. It suggests involvement of reduced PDH activity in the reduced glucose utilization in type 1 and type 2 diabetes (e.g., non-patent documents 6, 7).

On the contrary, hepatic gluconeogenesis is enhanced in type 1 and type 2 diabetes, which also forms one factor causing hyperglycemia. The reduced PDH activity increases pyruvic acid concentration, which in turn increases availability of lactic acid as a substrate for hepatic gluconeogenesis. It suggests possible involvement of reduced PDH activity in the enhanced gluconeogenesis in type 1 and type 2 diabetes (e.g., non-patent documents 8, 9).

When PDH is activated by inhibition of PDHK, the rate of glucose oxidation is considered to rise. As a result, glucose utilization in the body is promoted and hepatic gluconeogenesis is suppressed, whereby hyperglycemia in type 1 and type 2 diabetes is expected to be improved (e.g., non-patent documents 10, 11, 12).

Another factor contributing to diabetes is impaired insulin secretion, which is known to be associated with reduced PDH activity in pancreatic β cells, and induction of PDHK1, 2 and 4 (e.g., non-patent documents 13, 14).

In addition, sustained hyperglycemia due to diabetes is known to cause complications such as diabetic neuropathy, diabetic retinopathy, diabetic nephropathy and the like. Thiamine and α-lipoic acid contribute to activation of PDH as coenzymes. Thiamine and α-lipoic acid, or thiamine derivatives and α-lipoic acid derivatives are shown to have a promising effect on the treatment of diabetic complications. Thus, activation of PDH is expected to improve diabetic complications (e.g., non-patent documents 15, 16).

Under ischemic conditions, limited oxygen supply reduces oxidation of both glucose and fatty acid and reduces the amount of ATP produced by oxidative phosphorylation in the tissues. In the absence of sufficient oxygen, ATP level is maintained by promoted anaerobic glycolysis. As a result, lactic acid increases and intracellular pH decreases. Even though the cells try to maintain homeostasis of ion by energy consumption, abnormally low ATP level and disrupted cellular osmolarity lead to cell death. In addition, adenosine monophosphate-activating kinase activated in an ischemic state inactivates acetyl-CoA carboxylase by phosphorylation. The levels of total malonyl-CoA in the tissue drop, carnitine palmitoyltransferase-I activity is therefore increased and fatty acid oxidation is favored over glucose oxidation by allowing the transport of acyl-CoA into mitochondria. Oxidation of glucose is capable of yielding more ATP per molecule of oxygen than is oxidation of fatty acids. Under ischemic conditions, therefore, when energy metabolism becomes glucose oxidation dominant by activation of PDH, the ability to maintain ATP level is considered to be enhanced (e.g., non-patent document 17).

In addition, since activation of PDH causes oxidation of pyruvic acid produced by glycolysis, and reducing production of lactic acid, the net proton burden is considered to be reduced in ischemic tissues. Therefore, PDH activation by inhibition of PDHK is expected to protectively act in ischemic diseases such as cardiac muscle ischemia (e.g., non-patent documents 18, 19).

A drug that activates PDH by inhibition of PDHK is considered to decrease lactate production since it promotes pyruvate metabolism. Hence, such drug is expected to be useful for the treatment of hyperlactacidemia such as mitochondrial disease, mitochondrial encephalomyopathy and sepsis (e.g., non-patent document 20).

In cancer cells, the expression of PDHK1 or 2 increases. In cancer cells, moreover, ATP production by oxidative phosphorylation in mitochondria decreases, and ATP production via the anaerobic glycolysis in cytoplasm increases. PDH activation by inhibition of PDHK is expected to promote oxidative phosphorylation in mitochondria, and increase production of active oxygen, which will induce apoptosis of cancer cells. Therefore, the PDH activation by PDHK inhibition is useful for the treatment of cancerous diseases (e.g., non-patent document 21).

Pulmonary hypertension is characterized by high blood pressure caused by partial narrowing of the pulmonary artery due to promoted cellular proliferation therein. In pulmonary hypertension, therefore, activation of PDH in the pulmonary artery cell is expected to promote oxidative phosphorylation in mitochondria, increase production of active oxygen, and induce apoptosis of the pulmonary artery cells. Therefore, the PDH activation by PDHK inhibition is considered to be useful for the treatment of pulmonary hypertension, for example, pulmonary arterial hypertension (e.g., non-patent document 22).

Energy production and glucose metabolism in the cerebrum decrease in Alzheimer disease, and also, PDH activity declines. When the PDH activity declines, production of acetyl CoA decreases. Acetyl CoA is utilized for ATP production in the electron transport system via the citric acid cycle. Acetyl CoA is also a starting material for synthesizing acetylcholine, which is one of the neurotransmitters. Therefore, reduced brain PDH activity in Alzheimer disease is considered to cause neuronal cell death due to the decreased ATP production. Moreover, it is considered that synthesis of acetylcholine, which is the transmitter for cholinergic nerve, is inhibited to induce deterioration of memory and the like. Activation of PDH in the brain is expected to enhance energy production and acetylcholine synthesis in Alzheimer disease. Therefore, activation of PDH by the inhibition of PDHK is considered to be useful for the treatment of Alzheimer disease (e.g., non-patent documents 23, 24).

Vascular dementia is a disease that is roughly classified into a large-vessel type and a small-vessel type. In the large-vessel type, cerebral infarction including ischemia reperfusion is a factor, and neuronal cell death is induced by an increase in pyruvic acid and lactic acid values due to a decrease in the intracerebral PDH activity, and a decrease in energy production. In the small-vessel type, white matter lesion due to cerebral hypoperfusion is a factor and is considered to cause cognitive dysfunction due to a chronic decrease in glucose metabolism. When PDH in the brain is activated in vascular dementia, a decrease in the lactic acid value and the like and an increase in the energy production are expected in the large-vessel type, and promoted glucose metabolism is expected in the small-vessel type. Therefore, activation of PDH by PDHK inhibitors is considered to be useful for the treatment of vascular dementia (e.g., non-patent documents 28, 29, 30).

It has been shown that dichloroacetic acid, which is a drug having a PDH activating action, provides promising effects for the treatment of diabetes, myocardial ischemia, myocardial infarction, angina pectoris, cardiac failure, hyperlactacidemia, brain ischemia, cerebral apoplexy, peripheral arterial disease, chronic obstructive pulmonary disease, cancerous disease, and pulmonary hypertension (e.g., non-patent documents 10, 18, 20, 22, 25, 26, 27).

It has been shown that a compound having a PDHK inhibitory action has a neuroprotective effect on retinal ischemia-reperfusion injury (non-patent document 31). Retinal ischemia injury is involved in diseases such as glaucoma, diabetic retinopathy, retinopathy of prematurity, retinal vein occlusion and the like.

It has also been shown that in disease model animals exhibiting chronic kidney disease-like renal disorder and decreased renal function, compounds having a PDHK inhibitory action reduce the severity of the diseases (patent document 1).

From the foregoing findings, a PDHK inhibitor is considered to be useful for the treatment or prophylaxis of diseases relating to glucose utilization disorder, for example, diabetes (type 1 diabetes, type 2 diabetes etc.), insulin resistance syndrome, metabolic syndrome, hyperglycemia, hyperlactacidemia, diabetic complications (diabetic neuropathy, diabetic retinopathy, diabetic nephropathy, cataract etc.). Furthermore, a PDHK inhibitor is considered to be useful for the treatment or prophylaxis of diseases caused by limited energy substrate supply to the tissues, for example, cardiac failure (acute cardiac failure, chronic cardiac failure), cardiomyopathy, myocardial ischemia, myocardial infarction, angina pectoris, dyslipidemia, atherosclerosis, peripheral arterial disease, intermittent claudication, chronic obstructive pulmonary disease, brain ischemia, cerebral apoplexy, Alzheimer disease, vascular dementia, glaucoma, diabetic retinopathy, retinopathy of prematurity, retinal vein occlusion, ischemic optic neuropathy and chronic kidney disease. Furthermore, a PDHK inhibitor is considered to be useful for the treatment or prophylaxis of mitochondrial disease, mitochondrial encephalomyopathy, cancer, pulmonary hypertension and the like.

Therefore, a PDHK inhibitor is considered to be useful for the treatment or prophylaxis of diabetes (type 1 diabetes, type 2 diabetes etc.), insulin resistance syndrome, metabolic syndrome, hyperglycemia, hyperlactacidemia, diabetic complications (diabetic neuropathy, diabetic retinopathy, diabetic nephropathy, cataract etc.), cardiac failure (acute cardiac failure, chronic cardiac failure), cardiomyopathy, myocardial ischemia, myocardial infarction, angina pectoris, dyslipidemia, atherosclerosis, peripheral arterial disease, intermittent claudication, chronic obstructive pulmonary disease, brain ischemia, cerebral apoplexy, mitochondrial disease, mitochondrial encephalomyopathy, cancer, pulmonary hypertension, Alzheimer disease, vascular dementia, glaucoma, diabetic retinopathy, retinopathy of prematurity, retinal vein occlusion, ischemic optic neuropathy, or chronic kidney disease.

### Document List

### Patent document

patent document 1: WO 2020/054734

### Non-patent documents

non-patent document 1: Reed LJ, Hackert ML. Structure-function relationships in dihydrolipoamide acyltransferases. J Biol Chem. 1990 Jun 5; 265(16):8971-4.
non-patent document 2: Patel MS, Roche TE. Molecular biology and biochemistry of pyruvate dehydrogenase complexes. FASEB J. 1990 Nov; 4(14):3224-33.
non-patent document 3: Sugden MC, Holness MJ. Recent advances in mechanisms regulating glucose oxidation at the level of the pyruvate dehydrogenase complex by PDKs. Am J Physiol Endocrinol Metab. 2003 May; 284(5):E855-62.
non-patent document 4: Bowker-Kinley MM, Davis WI, Wu P, Harris RA, Popov KM. Evidence for existence of tissue-specific regulation of the mammalian pyruvate dehydrogenase complex. Biochem J. 1998 Jan 1; 329 (Pt 1):191-6.
non-patent document 5: Kim JW, Tchernyshyov I, Semenza GL, Dang CV. HIF-1-mediated expression of pyruvate dehydrogenase kinase: a metabolic switch required for cellular adaptation to hypoxia. Cell Metab. 2006 Mar; 3(3):177-85.
non-patent document 6: Morino K, Petersen KF, Dufour S, Befroy D, Frattini J, Shatzkes N, et al. Reduced mitochondrial density and increased IRS-1 serine phosphorylation in muscle of insulin-resistant offspring of type 2 diabetic parents. J Clin Invest. 2005 Dec; 115(12):3587-93.
non-patent document 7: Caterson ID, Fuller SJ, Randle PJ. Effect of the fatty acid oxidation inhibitor 2-tetradecylglycidic acid on pyruvate dehydrogenase complex activity in starved and alloxan-diabetic rats. Biochem J. 1982 Oct 15; 208(1):53-60.
non-patent document 8: Boden G, Chen X, Stein TP. Gluconeogenesis in moderately and severely hyperglycemic patients with type 2 diabetes mellitus. Am J Physiol Endocrinol Metab. 2001 Jan; 280(1):E23-30.
non-patent document 9: Shangraw RE, Fisher DM. Pharmacokinetics and pharmacodynamics of dichloroacetate in patients with cirrhosis. Clin Pharmacol Ther. 1999 Oct; 66(4):380-90.
non-patent document 10: Stacpoole PW, Moore GW, Kornhauser DM. Metabolic effects of dichloroacetate in patients with diabetes mellitus and hyperlipoproteinemia. NEngl J Med. 1978 Mar 9; 298(10):526-30.
non-patent document 11: Mayers RM, Leighton B, Kilgour E. PDH kinase inhibitors: a novel therapy for Type II diabetes? Biochem Soc Trans. 2005 Apr; 33(Pt 2):367-70.
non-patent document 12: Jeoung NH, Rahimi Y, Wu P, Lee WN, Harris RA. Fasting induces ketoacidosis and hypothermia in PDHK2/PDHK4-double-knockout mice. Biochem J.2012 May 1; 443(3):829-39.
non-patent document 13: Zhou YP, Berggren PO, Grill V. A fatty acid-induced decrease in pyruvate dehydrogenase activity is an important determinant of beta-cell dysfunction in the obese diabetic db/db mouse. Diabetes. 1996 May; 45(5):580-6.
non-patent document 14: Xu J, Han J, Epstein PN, Liu YQ. Regulation of PDK mRNA by high fatty acid and glucose in pancreatic islets. Biochem Biophys Res Commun. 2006 Jun 9; 344(3):827-33.
non-patent document 15: Benfotiamine. Monograph. Altern Med Rev. 2006 Sep; 11(3):238-42.
non-patent document 16: Vallianou N, Evangelopoulos A, Koutalas P. Alpha-lipoic Acid and diabetic neuropathy. Rev Diabet Stud. 2009 Winter; 6(4):230-6.
non-patent document 17: Ussher JR, Lopaschuk GD. The malonyl CoA axis as a potential target for treating ischaemic heart disease. Cardiovasc Res. 2008 Jul 15; 79(2):259-68.
non-patent document 18: Wargovich TJ, MacDonald RG, Hill JA, Feldman RL, StacpoolePW, Pepine CJ. Myocardial metabolic and hemodynamic effects of dichloroacetate in coronary artery disease. Am J Cardiol. 1988 Jan 1; 61(1):65-70.
non-patent document 19: Taniguchi M, Wilson C, Hunter CA, Pehowich DJ, Clanachan AS, Lopaschuk GD. Dichloroacetate improves cardiac efficiency after ischemia independent of changes in mitochondrial proton leak. Am J Physiol Heart Circ Physiol. 2001 Apr; 280(4):H1762-9.
non-patent document 20: Stacpoole PW, Nagaraja NV, Hutson AD. Efficacy of dichloroacetate as a lactate-lowering drug. J Clin Pharmacol. 2003 Jul; 43(7):683-91.
non-patent document 21: Bonnet S, Archer SL, Allalunis-Turner J, Haromy A, Beaulieu C, Thompson R, et al. A mitochondria-K+ channel axis is suppressed in cancer and its normalization promotes apoptosis and inhibits cancer growth. Cancer Cell.2007 Jan; 11(1):37-51.
non-patent document 22: McMurtry MS, Bonnet S, Wu X, Dyck JR, Haromy A, Hashimoto K, et al. Dichloroacetate prevents and reverses pulmonary hypertension by inducing pulmonary artery smooth muscle cell apoptosis. Circ Res. 2004 Oct 15; 95(8):830-40.
non-patent document 23: Saxena U. Bioenergetics breakdown in Alzheimer's disease: targets for new therapies. Int J Physiol Pathophysiol Pharmacol. 2011; 3(2):133-9.
non-patent document 24: Stacpoole PW. The pyruvate dehydrogenase complex as a therapeutic target for age-related diseases. Aging Cell. 2012 Jun; 11(3):371-7.
non-patent document 25: Marangos PJ, Turkel CC, Dziewanowska ZE, Fox AW. Dichloroacetate and cerebral ischaemia therapeutics. Expert Opin Investig Drugs. 1999 Apr; 8(4):373-82.
non-patent document 26: Calvert LD, Shelley R, Singh SJ, Greenhaff PL, Bankart J, Morgan MD, et al. Dichloroacetate enhances performance and reduces blood lactate during maximal cycle exercise in chronic obstructive pulmonary disease. Am J Respir Crit Care Med. 2008 May 15; 177(10):1090-4.
non-patent document 27: Flavin DF. Non-Hodgkin's Lymphoma Reversal with Dichloroacetate. J Oncol. Hindawi Publishing Corporation Journal of Oncology Volume 2010, Article ID 414726, 4 pages doi:10.1155/2010/414726.
non-patent document 28: Froelich L, Goetz ME, Weinmueller M, Youdim MB, Barth N, Dirr A, Gsell W, Jellinger K, Beckmann H, Riederer P. (r)-, but not (s)-alpha lipoic acid stimulates deficient brain pyruvate dehydrogenase complex in vascular dementia, but not in Alzheimer dementia. J Neural Transm (Vienna). 2004 Mar; 111(3):295-310
non-patent document 29: Parnetti L, Reboldi GP, Gallai V. Cerebrospinal fluid pyruvate levels in Alzheimer's disease and vascular dementia. Neurology. 2000 Feb 8; 54(3):735-7.
non-patent document 30: Pascual B, Prieto E, Arbizu J, Marti-Climent J, Olier J, Masdeu JC. Brain glucose metabolism in vascular white matter disease with dementia: differentiation from Alzheimer disease. Stroke. 2010 Dec; 41(12):2889-93.
non-patent document 31: Sato K, Mochida S, Tomimoto D, Konuma T, Kiyota N, Tsuda S, Shiga Y, Omodaka K, Nakazawa T. A pyruvate dehydrogenase kinase inhibitor prevents retinal cell death and improves energy metabolism in rat retinas after ischemia/reperfusion injury. Experimental eye research 2020 Apr; 193: 107997.

### Summary of Invention

The present invention is as follow.
[1] A compound of the formula [I-a], or a pharmaceutically acceptable salt thereof: 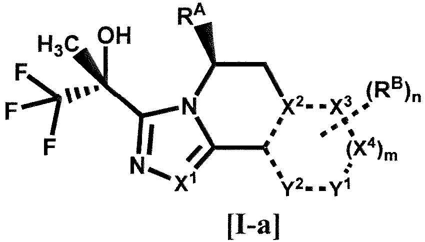 wherein
   a bond in a dotted line is a single bond or a double bond, X¹, X², X³ and X⁴ are each independently C or N, Y¹ and Y² are each independently C, N or O (wherein the total number of N and O for X², X³, X⁴, Y¹ or Y² is 0 to 3),
   R^{A} is C₁₋₄ alkyl,
   R^{B} is
      (1) halogen,
      (2) cyano,
      (3) hydroxy,
      (4) oxo,
      (5) -COR¹ {wherein R¹ is
         (A) hydrogen,
         (B) -OH,
         (C) -NR²R³ (wherein R² and R³ are each independently hydrogen or C₁₋₄ alkyl), or
         (D) a 4- to 6-membered saturated heterocyclyl having one nitrogen atom (wherein the saturated heterocyclyl is optionally substituted by 1 or 2 halogens)},
      (6) C₁₋₈ alkyl {wherein the C₁₋₈ alkyl is optionally substituted by 1 to 8 substituents independently selected from the group consisting of
         (A) halogen,
         (B) hydroxy,
         (C) phenyl optionally substituted by halogen,
         (D) pyridyl optionally substituted by haloC₁₋₄ alkyl, and
         (E) -OR⁴ (wherein R⁴ is
            (a) C₁₋₄ alkyl,
            (b) phenyl optionally substituted by halogen, or
            (c) benzyl optionally substituted by C₁₋₄ alkoxy)),
      (7) C₁₋₈ alkoxy {wherein the C₁₋₈ alkoxy is optionally substituted by 1 to 8 substituents independently selected from the group consisting of
         (A) halogen,
         (B) cyano,
         (C) hydroxy,
         (D) C₁₋₄ alkoxy optionally substituted by 1 to 3 halogens,
         (E) C₁₋₄ alkylsulfonyl,
         (F) C₃₋₆ cycloalkyl optionally substituted by one substituent selected from the group consisting of cyano and cyano C₁₋₄ alkyl,
         (G) phenyl optionally substituted by cyano,
         (H) -COCy¹ (wherein Cy¹ is a 4- to 6-membered saturated heterocyclyl having one nitrogen atom, and the saturated heterocyclyl is optionally substituted by 1 or 2 halogens), and
         (I) a 4- to 6-membered saturated heterocyclyl having 1 or 2 hetero atoms independently selected from a nitrogen atom, an oxygen atom and a sulfur atom (wherein the saturated heterocyclyl is optionally substituted by 1 to 4 substituents independently selected from
            (a) C₁₋₄ alkyl,
            (b) oxo,
            (c) C₁₋₄ alkylcarbonyl,
            (d) benzoyl optionally substituted by halogen, and
            (e) C₁₋₄ alkylsulfonyl,
            when the saturated heterocyclyl is substituted by two C₁₋₄ alkyls, the two C₁₋₄ alkyls are optionally bonded to each other to form a bridged ring together with the atoms bonded thereto)},
      (8) -Cy² {wherein Cy² is
         (A) C₃₋₆ cycloalkyl (wherein the C₃₋₆ cycloalkyl is optionally substituted by 1 or 2 substituents independently selected from
            (a) halogen,
            (b) C₁₋₄ alkyl,
            (c) haloC₁₋₄ alkyl, and
            (d) phenyl optionally substituted by halogen),
         (B) phenyl optionally substituted by 1 or 2 substituents independently selected from the group consisting of halogen, haloC₁₋₄ alkyl, and C₁₋₄ alkoxy, or
         (C) a 4- to 6-membered saturated heterocyclyl having one nitrogen atom or oxygen atom (wherein the saturated heterocyclyl is optionally substituted by one substituent selected from the group consisting of (a) phenyl optionally substituted by halogen and (b) C₁₋₄ alkylcarbonyl)}, or
      (9) -OCy³ {wherein Cy³ is
         (A) a 4- to 6-membered saturated heterocyclyl having one nitrogen atom or oxygen atom (wherein the saturated heterocyclyl is optionally substituted by one substituent selected from the group consisting of (a) benzoyl optionally substituted by halogen and (b) C₁₋₄ alkylcarbonyl), or
         (B) a 6-membered heteroaryl having 1 or 2 nitrogen atoms (wherein the heteroaryl is optionally substituted by 1 or 2 substituents independently selected from cyano, haloC₁₋₄ alkyl, and C₃₋₆ cycloalkyl)},

         m is 0 or 1, and
         n is 0, 1 or 2, when n is 2, each R^{B} may be the same or different.
[2] The compound of [1] or a pharmaceutically acceptable salt thereof, which is a compound of the formula [I-b]: 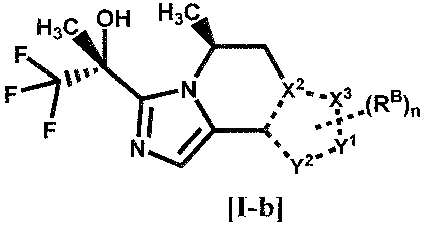 wherein each symbol is as defined in [1], or a pharmaceutically acceptable salt thereof.
[3] The compound of [1] or [2] or a pharmaceutically acceptable salt thereof, which is a compound of the formula [I-c]: 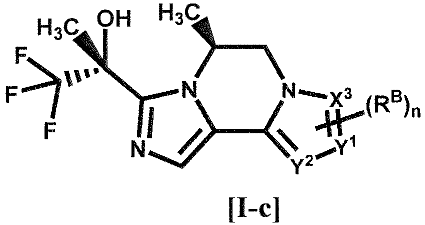 wherein each symbol is as defined in [1], or a pharmaceutically acceptable salt thereof.
[4] The compound of any one of [1] to [3], wherein n is 1, or a pharmaceutically acceptable salt thereof.
[5] The compound of any one of [1] to [4] or a pharmaceutically acceptable salt thereof, which is a compound of the formula [I-d]: 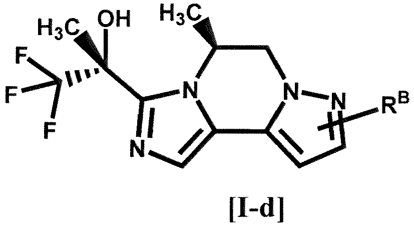 wherein the symbol is as defined in [1], or a pharmaceutically acceptable salt thereof.
[6] The compound of any one of [1] to [4] or a pharmaceutically acceptable salt thereof, which is a compound of the formula [I-e]: 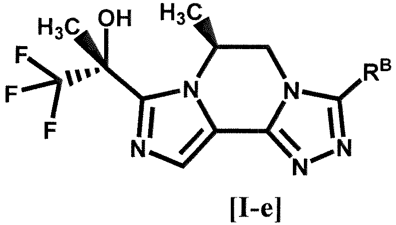 wherein the symbol is as defined in [1], or a pharmaceutically acceptable salt thereof.
[7] The compound of any one of [1] to [6] or a pharmaceutically acceptable salt thereof, wherein R^{B} is (1) C₁₋₈ alkyl {wherein the C₁₋₈ alkyl is optionally substituted by 1 to 8 substituents independently selected from the group consisting of
   (A) halogen,
   (B) hydroxy,
   (C) phenyl optionally substituted by halogen,
   (D) pyridyl optionally substituted by haloC₁₋₄ alkyl, and
   (E) -OR⁴ (wherein R⁴ is
      (a) C₁₋₄ alkyl,
      (b) phenyl optionally substituted by halogen, or
      (c) benzyl optionally substituted by C₁₋₄ alkoxy)}, or (2) C₁₋₈ alkoxy {wherein the C₁₋₈ alkoxy is optionally substituted by 1 to 8 substituents independently selected from the group consisting of
         (A) halogen,
         (B) cyano,
         (C) hydroxy,
         (D) C₁₋₄ alkoxy optionally substituted by 1 to 3 halogens,
         (E) C₁₋₉ alkylsulfonyl,
         (F) C₃₋₆ cycloalkyl optionally substituted by one substituent selected from the group consisting of cyano and cyano C₁₋₄ alkyl,
         (G) phenyl optionally substituted by cyano,
         (H) -COCy¹ (wherein Cy¹ is a 4- to 6-membered saturated heterocyclyl having one nitrogen atom, and the saturated heterocyclyl is optionally substituted by 1 or 2 halogens), and
         (I) a 4- to 6-membered saturated heterocyclyl having 1 or 2 hetero atoms independently selected from a nitrogen atom, an oxygen atom and a sulfur atom (wherein the saturated heterocyclyl is optionally substituted by 1 to 4 substituents independently selected from
            (a) C₁₋₄ alkyl,
            (b) oxo,
            (c) C₁₋₄ alkylcarbonyl,
            (d) benzoyl optionally substituted by halogen, and
            (e) C₁₋₄ alkylsulfonyl,
      when the saturated heterocyclyl is substituted by two C₁₋₄ alkyls, the two C₁₋₄ alkyls are optionally bonded to each other to form a bridged ring together with the atoms bonded thereto)}.
[8] A compound selected from the following formulas: 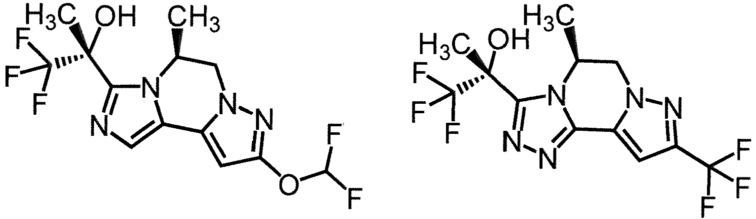 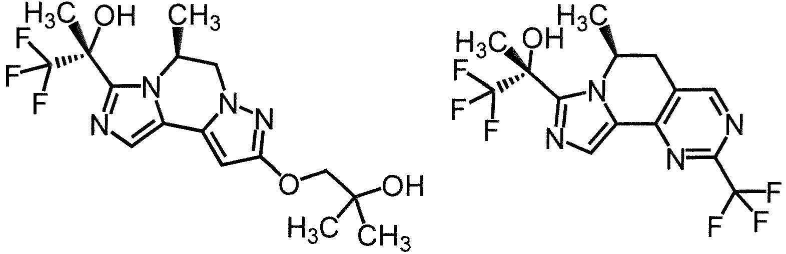 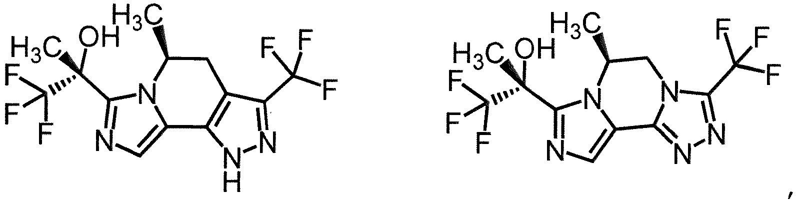 or a pharmaceutically acceptable salt thereof.
[9] A pharmaceutical composition comprising the compound of any one of [1] to [8] or a pharmaceutically acceptable salt thereof, and a pharmaceutically acceptable carrier.
[10] A PDHK inhibitor comprising the compound of any one of [1] to [8] or a pharmaceutically acceptable salt thereof.
[11] A PDHK2 inhibitor comprising the compound of any one of [1] to [8] or a pharmaceutically acceptable salt thereof.
[12] An agent for the treatment or prophylaxis of diabetes, insulin resistance syndrome, metabolic syndrome, hyperglycemia, hyperlactacidemia, diabetic complication, cardiac failure, cardiomyopathy, myocardial ischemia, myocardial infarction, angina pectoris, dyslipidemia, atherosclerosis, peripheral arterial disease, intermittent claudication, chronic obstructive pulmonary disease, brain ischemia, cerebral apoplexy, mitochondrial disease, mitochondrial encephalomyopathy, cancer, pulmonary hypertension, Alzheimer disease, vascular dementia, glaucoma, diabetic retinopathy, retinopathy of prematurity, retinal vein occlusion, ischemic optic neuropathy or chronic kidney disease, the agent comprising the compound of any one of [1] to [8] or a pharmaceutically acceptable salt thereof.
[13] The agent of [12], wherein the diabetes is type 1 diabetes or type 2 diabetes.
[14] The agent of [12], wherein the vascular dementia is a large-vessel type of vascular dementia or a small-vessel type of vascular dementia.
[15] The agent of [12], wherein the cardiac failure is acute cardiac failure or chronic cardiac failure.
[16] The agent of [12], wherein the pulmonary hypertension is pulmonary arterial hypertension.
[17] A method for inhibiting PDHK, comprising administering a therapeutically effective amount of the compound of any one of [1] to [8] or a pharmaceutically acceptable salt thereof to a mammal.
[18] A method for treating or preventing a disease selected from the group consisting of diabetes, insulin resistance syndrome, metabolic syndrome, hyperglycemia, hyperlactacidemia, diabetic complication, cardiac failure, cardiomyopathy, myocardial ischemia, myocardial infarction, angina pectoris, dyslipidemia, atherosclerosis, peripheral arterial disease, intermittent claudication, chronic obstructive pulmonary disease, brain ischemia, cerebral apoplexy, mitochondrial disease, mitochondrial encephalomyopathy, cancer, pulmonary hypertension, Alzheimer disease, vascular dementia, glaucoma, diabetic retinopathy, retinopathy of prematurity, retinal vein occlusion, ischemic optic neuropathy and chronic kidney disease, the method comprising administering a therapeutically effective amount of the compound of any one of [1] to [8] or a pharmaceutically acceptable salt thereof to a mammal.
[19] The method of [18], wherein the diabetes is type 1 diabetes or type 2 diabetes.
[20] The method of [18], wherein the vascular dementia is a large-vessel type of vascular dementia or a small-vessel type of vascular dementia.
[21] The method of [18], wherein the cardiac failure is acute cardiac failure or chronic cardiac failure.
[22] The method of [18], wherein the pulmonary hypertension is pulmonary arterial hypertension.
[23] Use of the compound of any one of [1] to [8] or a pharmaceutically acceptable salt thereof in the manufacture of a PDHK inhibitor.
[24] Use of the compound of any one of [1] to [8] or a pharmaceutically acceptable salt thereof in the manufacture of an agent for the treatment or prophylaxis of a disease selected from the group consisting of diabetes, insulin resistance syndrome, metabolic syndrome, hyperglycemia, hyperlactacidemia, diabetic complication, cardiac failure, cardiomyopathy, myocardial ischemia, myocardial infarction, angina pectoris, dyslipidemia, atherosclerosis, peripheral arterial disease, intermittent claudication, chronic obstructive pulmonary disease, brain ischemia, cerebral apoplexy, mitochondrial disease, mitochondrial encephalomyopathy, cancer, pulmonary hypertension, Alzheimer disease, vascular dementia, glaucoma, diabetic retinopathy, retinopathy of prematurity, retinal vein occlusion, ischemic optic neuropathy and chronic kidney disease.
[25] The use of [24], wherein the diabetes is type 1 diabetes or type 2 diabetes.
[26] The use of [24], wherein the vascular dementia is a large-vessel type of vascular dementia or a small-vessel type of vascular dementia.
[27] The use of [24] wherein the cardiac failure is acute cardiac failure or chronic cardiac failure.
[28] The use of [24] wherein the pulmonary hypertension is pulmonary arterial hypertension.
[29] The compound of any one of [1] to [8] or a pharmaceutically acceptable salt thereof for use in the treatment or prophylaxis of a disease selected from the group consisting of diabetes, insulin resistance syndrome, metabolic syndrome, hyperglycemia, hyperlactacidemia, diabetic complication, cardiac failure, cardiomyopathy, myocardial ischemia, myocardial infarction, angina pectoris, dyslipidemia, atherosclerosis, peripheral arterial disease, intermittent claudication, chronic obstructive pulmonary disease, brain ischemia, cerebral apoplexy, mitochondrial disease, mitochondrial encephalomyopathy, cancer, pulmonary hypertension, Alzheimer disease, vascular dementia, glaucoma, diabetic retinopathy, retinopathy of prematurity, retinal vein occlusion, ischemic optic neuropathy and chronic kidney disease.
[30] The compound of [29] or a pharmaceutically acceptable salt thereof, wherein the diabetes is type 1 diabetes or type 2 diabetes.
[31] The compound of [29] or a pharmaceutically acceptable salt thereof, wherein the vascular dementia is a large-vessel type of vascular dementia or a small-vessel type of vascular dementia.
[32] The compound of [29] or a pharmaceutically acceptable salt thereof, wherein the cardiac failure is acute cardiac failure or chronic cardiac failure.
[33] The compound of [29] or a pharmaceutically acceptable salt thereof, wherein the pulmonary hypertension is pulmonary arterial hypertension.
[34] A method for inhibiting PDHK2, comprising administering a therapeutically effective amount of the compound of any one of [1] to [8] or a pharmaceutically acceptable salt thereof to a mammal.
[35] A commercial package comprising the pharmaceutical composition of [9], and a written matter associated therewith, the written matter stating that the pharmaceutical composition can be used for the treatment or prophylaxis of a disease selected from the group consisting of diabetes, insulin resistance syndrome, metabolic syndrome, hyperglycemia, hyperlactacidemia, diabetic complication, cardiac failure, cardiomyopathy, myocardial ischemia, myocardial infarction, angina pectoris, dyslipidemia, atherosclerosis, peripheral arterial disease, intermittent claudication, chronic obstructive pulmonary disease, brain ischemia, cerebral apoplexy, mitochondrial disease, mitochondrial encephalomyopathy, cancer, pulmonary hypertension, Alzheimer disease, vascular dementia, glaucoma, diabetic retinopathy, retinopathy of prematurity, retinal vein occlusion, ischemic optic neuropathy and chronic kidney disease.
[36] A kit comprising the pharmaceutical composition of [9], and a written matter associated therewith, the written matter stating that the pharmaceutical composition can be used for the treatment or prophylaxis of a disease selected from the group consisting of diabetes, insulin resistance syndrome, metabolic syndrome, hyperglycemia, hyperlactacidemia, diabetic complication, cardiac failure, cardiomyopathy, myocardial ischemia, myocardial infarction, angina pectoris, dyslipidemia, atherosclerosis, peripheral arterial disease, intermittent claudication, chronic obstructive pulmonary disease, brain ischemia, cerebral apoplexy, mitochondrial disease, mitochondrial encephalomyopathy, cancer, pulmonary hypertension, Alzheimer disease, vascular dementia, glaucoma, diabetic retinopathy, retinopathy of prematurity, retinal vein occlusion, ischemic optic neuropathy and chronic kidney disease.

### Description of Embodiments

The definitions of the terms used in the present invention are as follows.

The "halogen" is fluoro, chloro, bromo or iodo. As "halogen", fluoro or chloro is preferred.

The "C₁₋₄ alkyl" means a straight chain or branched chain alkyl having 1 to 4 carbon atoms. Examples thereof include methyl, ethyl, propyl, isopropyl, butyl, isobutyl, sec-butyl and tert-butyl. As "C₁₋₄ alkyl", methyl is preferred.

The "C₁₋₈ alkyl" means a straight chain or branched chain alkyl having 1 to 8 carbon atoms. Examples thereof include methyl, ethyl, propyl, isopropyl, 1,1-dimethylpropyl, 1-ethylpropyl, 1-methyl-1-ethyl-propyl, butyl, isobutyl, sec-butyl, tert-butyl, 1-methyl-1-propyl-butyl, pentyl, isopentyl, neopentyl, hexyl, isohexyl, 1,1-dimethylbutyl, 2,2-dimethylbutyl, 3,3-dimethylbutyl, 2-ethylbutyl and the like.

The "C₁₋₄ alkylcarbonyl" means alkyl-carbonyl in which the alkyl moiety is "C₁₋₄ alkyl" defined above and includes, for example, acetyl, propanoyl, butanoyl, 2-methylpropanoyl, pentanoyl, 3-methylbutanoyl, 2-methylbutanoyl and 2,2-dimethylpropanoyl. As "C₁₋₄ alkylcarbonyl", acetyl is preferred.

The "C₁₋₄ alkylsulfonyl" means alkyl-sulfonyl in which the alkyl moiety is "C₁₋₄ alkyl" defined above and includes, for example, methanesulfonyl, ethylsulfonyl, propylsulfonyl, isopropylsulfonyl, butylsulfonyl, isobutylsulfonyl, sec-butylsulfonyl and tert-butylsulfonyl. As "C₁₋₄ alkylsulfonyl", methanesulfonyl is preferred.

The "haloC₁₋₄ alkyl" means straight chain or branched chain alkyl having 1 to 4 carbon atoms and substituted by 1 to 5 "halogens" defined above. When alkyl is substituted by plural halogens, the halogens may be the same or different. Examples of the "haloC₁₋₄ alkyl" include fluoromethyl, difluoromethyl, trifluoromethyl, 1-fluoro-1-methylethyl, 1,1-difluoroethyl, 2,2-difluoroethyl, 2,2,2-trifluoroethyl, pentafluoroethyl, 1,1-difluoropropyl, 1,1-difluoro-2-methylpropyl and the like. As "haloC₁₋₄ alkyl", C₁₋₄ alkyl substituted by 1 to 3 fluoros is preferred and trifluoromethyl is more preferred.

The "cyano C₁₋₄ alkyl" means "C₁₋₄ alkyl" defined above which is substituted by one cyano. Examples thereof include cyanomethyl, 2-cyanoethyl, 1-cyano-1-methylethyl, 3-cyanopropyl, 4-cyanobutyl and the like.

The "C₁₋₄ alkoxy" means alkyl-oxy in which the alkyl moiety is "C₁₋₄ alkyl" defined above and includes, for example, methoxy, ethoxy, propoxy, isopropoxy, butoxy, isobutoxy, sec-butoxy and tert-butoxy. As "C₁₋₄ alkoxy", methoxy is preferred.

The "C₁₋₈ alkoxy" means alkoxy in which the alkyl moiety is "C₁₋₈ alkyl" defined above. Examples thereof include methoxy, ethoxy, propoxy, isopropoxy, butoxy, isobutoxy, sec-butoxy, tert-butoxy, pentyloxy, isopentyloxy, neopentyloxy, 1,2-dimethylpropyloxy, 1-ethylpropyloxy, hexyloxy, isohexyloxy, 1,2,2-trimethylpropyloxy, 1,1-dimethylbutyloxy, 2,2-dimethylbutyloxy, 3,3-dimethylbutyloxy, 2-ethylbutyloxy and the like.

The "C₃₋₆ cycloalkyl" means a 3- to 6-membered monocyclic hydrocarbon ring group and includes, for example, cyclopropyl, cyclobutyl, cyclopentyl and cyclohexyl. As "C₃₋₆ cycloalkyl", cyclopropyl is preferred.

The "4- to 6-membered saturated heterocyclyl having one nitrogen atom" means a 4- to 6-membered monocyclic saturated heterocyclic group having one nitrogen atom besides carbon atom. Examples of the saturated heterocyclyl include azetidinyl, pyrrolidinyl, and piperidinyl.

The "4- to 6-membered saturated heterocyclyl having 1 or 2 hetero atoms independently selected from the group consisting of a nitrogen atom, an oxygen atom and a sulfur atom" means a 4- to 6-membered monocyclic saturated heterocyclic group having, besides carbon atom, 1 or 2 hetero atoms independently selected from the group consisting of a nitrogen atom, an oxygen atom and a sulfur atom. Examples of the saturated heterocyclyl include oxetanyl, tetrahydrofuranyl, tetrahydropyranyl, azetidinyl, pyrrolidinyl, piperidinyl, piperazinyl, morpholinyl, tetrahydrothiopyranyl, isothiazolidinyl and the like, and oxetanyl, tetrahydrofuranyl, piperidinyl, tetrahydrothiopyranyl, and isothiazolidinyl are preferred.

"When saturated heterocyclyl is substituted by two C₁₋₄ alkyls, the two C₁₋₄ alkyls are optionally bonded to each other to form a bridged ring together with the atoms bonded thereto" means, for example, that the saturated heterocyclyl is the following group:

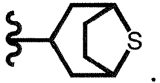

The "4- to 6-membered saturated heterocyclyl having one nitrogen atom or oxygen atom" means a 4- to 6-membered monocyclic saturated heterocyclic group having, besides carbon atom, one hetero atom independently selected from the group consisting of a nitrogen atom and an oxygen atom. Examples of the saturated heterocyclyl include oxetanyl, tetrahydrofuranyl, tetrahydropyranyl, azetidinyl, pyrrolidinyl, piperidinyl and the like, and oxetanyl, tetrahydrofuranyl, tetrahydropyranyl, azetidinyl, and piperidinyl are preferred.

The "6-membered heteroaryl having 1 or 2 nitrogen atoms" means 6-membered monocyclic heteroaryl having 1 or 2 nitrogen atoms besides carbon atom. Examples of the heteroaryl include pyridyl, pyrimidinyl, and pyrazinyl.

A preferred embodiment of the compound of the formula [I-a] is described below.

One of the preferred embodiments of the compound of the formula [I-a] is a compound represented by the formula [I-a1]: 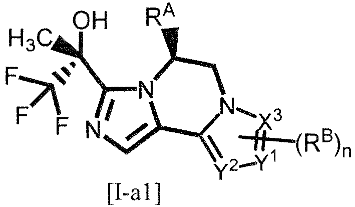 wherein each symbol is as defined in the aforementioned formula [I-a].

One of other preferred embodiments of the formula [I-a] is a compound represented by the formula [I-a2]: 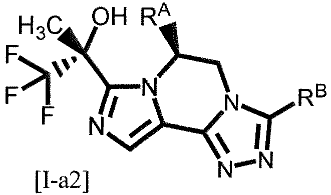 wherein each symbol is as defined in the aforementioned formula [I-a].

One of other preferred embodiments of the formula [I-a] is a compound represented by the formula [I-a3]: 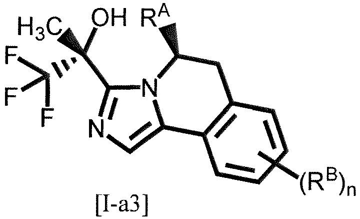 wherein each symbol is as defined in the aforementioned formula [I-a].

One of other preferred embodiments of the formula [I-a] is a compound represented by the formula [I-a4]: 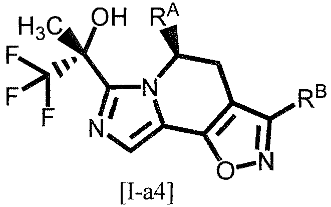 wherein each symbol is as defined in the aforementioned formula [I-a].

One of other preferred embodiments of the formula [I-a] is a compound represented by the formula [I-a5]: 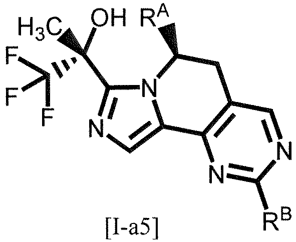 wherein each symbol is as defined in the aforementioned formula [I-a].

One of other preferred embodiments of the formula [I-a] is a compound represented by the formula [I-a6]: 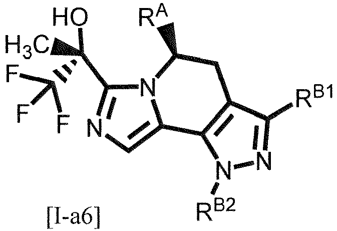 wherein R^{B1} and R^{B2} are each independently as defined for R^{B} in the aforementioned formula [I-a]; and other symbol is as defined in the aforementioned formula [I-a].

One of other preferred embodiments of the formula [I-a] is a compound represented by the formula [I-a7]: 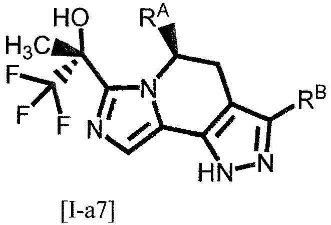 wherein each symbol is as defined in the aforementioned formula [I-a].

One of other preferred embodiments of the formula [I-a] is a compound represented by the formula [I-a8]: 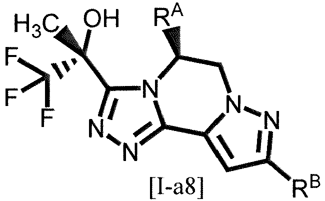 wherein each symbol is as defined in the aforementioned formula [I-a].

One of other preferred embodiments of the formula [I-a] is a compound represented by the formula [I-a10]: 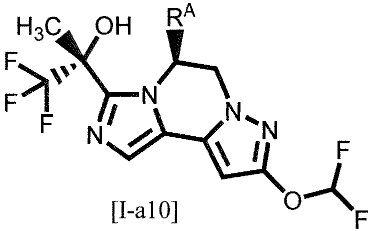 wherein the symbol is as defined in the aforementioned formula [I-a].

In the above-mentioned formulas [I-a] and [I-a1] to [I-a8], R^{A} is preferably methyl.

In the above-mentioned formulas [I-a], [I-a1] and [I-a3], n is preferably 1.

The "pharmaceutically acceptable salt" may be any salt without excessive toxicity known in the art. Specifically, salts with inorganic acids, salts with organic acids, salts with inorganic bases, salts with organic bases and the like can be mentioned. Various forms of pharmaceutically acceptable salts are well known in the art and, for example, they are described in the following reference documents:
(a) Berge et al., J. Pharm. Sci., 66, p1-19(1977),
(b) Stahl et al., "Handbook of Pharmaceutical Salts: Properties, Selection, and Use" (Wiley-VCH, Weinheim, Germany, 2002),
(c) Paulekuhn et al., J. Med. Chem., 50, p6665-6672 (2007).

A pharmaceutically acceptable salt of a compound of the formula [I-a] can be obtained by reacting the compound with an inorganic acid, organic acid, inorganic base or organic base according to a method known per se. A pharmaceutically acceptable salt of the compound of the formula [I-a] may be formed with one half molecule, one molecule or two or more molecules of an acid or base per molecule of the compound of the formula [I-a].

Examples of the salt with inorganic acid include salts with hydrofluoric acid, hydrochloric acid, hydrobromic acid, hydroiodic acid, nitric acid, phosphoric acid and sulfuric acid.

Examples of the salt with organic acid include salts with acetic acid, adipic acid, alginic acid, 4-aminosalicylic acid, anhydromethylenecitric acid, benzoic acid, benzenesulfonic acid, calcium edetate, camphoric acid, camphor-10-sulfonic acid, carbonic acid, citric acid, edetic acid, ethane-1,2-disulfonic acid, dodecylsulfuric acid, ethanesulfonic acid, fumaric acid, glucoheptonic acid, gluconic acid, glucuronic acid, glycolylarsanilic acid, hexylresorcylic acid, hydroxy-naphthoic acid, 2-hydroxy-1-ethanesulfonic acid, lactic acid, lactobionic acid, malic acid, maleic acid, mandelic acid, methanesulfonic acid, methylsulfuric acid, methylnitric acid, methylenebis(salicylic acid), galactaric acid, naphthalene-2-sulfonic acid, 2-naphthoic acid, 1,5-naphthalenedisulfonic acid, oleic acid, oxalic acid, pamoic acid, pantothenic acid, pectic acid, picric acid, propionic acid, polygalacturonic acid, salicylic acid, stearic acid, succinic acid, tannic acid, tartaric acid, teoclic acid, thiocyanic acid, trifluoroacetic acid, p-toluenesulfonic acid, undecanoic acid, aspartic acid and glutamic acid.

Examples of the salt with inorganic base include a salt with lithium, sodium, potassium, magnesium, calcium, barium, aluminum, zinc, bismuth or ammonium.

Examples of the salt with organic base include a salt with arecoline, betaine, choline, clemizole, ethylenediamine, N-methylglucamine, N-benzylphenethylamine, tris(hydroxymethyl)methylamine, arginine or lysine.

A preferred embodiment of the "pharmaceutically acceptable salt" is as described below.

Examples of the salt with inorganic acid include salts with hydrochloric acid, nitric acid, sulfuric acid, phosphoric acid and hydrobromic acid.

Examples of the salt with organic acid include salts with oxalic acid, maleic acid, citric acid, fumaric acid, lactic acid, malic acid, succinic acid, tartaric acid, acetic acid, trifluoroacetic acid, benzoic acid, glucuronic acid, oleic acid, pamoic acid, methanesulfonic acid, benzenesulfonic acid, p-toluenesulfonic acid and 2-hydroxy-1-ethanesulfonic acid.

Examples of the salt with inorganic base include salts with sodium, potassium, calcium, magnesium and zinc.

Examples of the salt with organic base include salts with tris(hydroxymethyl)methylamine, N-methylglucamine and lysine.

The compound of the formula [I-a] or a pharmaceutically acceptable salt thereof may exist as a solvate. The term "solvate" refers to the compound of the formula [I-a] or a pharmaceutically acceptable salt thereof with which a solvent molecule is associated, and also includes hydrates. Such solvates are preferably pharmaceutically acceptable solvates and include, for example, hydrate, ethanol solvate, dimethyl sulfoxide-solvate and the like of the compound of the formula [I-a] or a pharmaceutically acceptable salt thereof.

Specific examples include hemihydrate, monohydrate, dihydrate or mono(ethanol)solvate of the compound of the formula [I-a] or a monohydrate of hydrochloride of the compound of the formula [I-a], dihydrate of hydrochloride of the same and the like. Such solvates can be produced according to conventional methods.

The compound of the formula [I-a] may exist as a stereoisomer that should be recognized as a cis/trans isomer. In this case, the compound of the formula [I-a] may exist as a cis isomer, a trans isomer, or a mixture of a cis isomer and a trans isomer.

The compound of the formula [I-a] may exist as a tautomer. In this case, the compound of the formula [I-a] may exist as an individual tautomer or a mixture of tautomers.

The compound of the formula [I-a] may contain one or more asymmetric carbons. In this case, the compound of the formula [I-a] may exist as a single enantiomer, a single diastereomer, a mixture of enantiomers or a mixture of diastereomers.

The compound of the formula [I-a] may exist as an atropisomer. In this case, the compound of the formula [I-a] may exist as an individual atropisomer or a mixture of atropisomers.

The compound of the formula [I-a] may simultaneously contain plural structural characteristics that produce the above-mentioned isomers. Moreover, the compound of the formula [I-a] may contain the above-mentioned isomers at any ratio.

In the absence of other reference such as annotation and the like, the formulas, chemical structures and compound names indicated in the present specification without specifying the stereochemistry thereof encompass all the above-mentioned isomers that may exist.

A diastereomeric mixture can be separated into each diastereomer by conventional methods such as chromatography, crystallization and the like. In addition, each diastereomer can also be formed by using a stereochemically single starting material, or by a synthesis method using a stereoselective reaction.

An enantiomeric mixture can be separated into each single enantiomer by a method well known in the art.

For example, a mixture of enantiomers may be reacted with a substantially pure enantiomer which is known as a chiral auxiliary to form a mixture of diastereomers, which may be then isolated into a diastereomer with an enhanced isomeric ratio or a substantially pure single diastereomer by a common method such as fractionated crystallization or chromatography. The added chiral auxiliary may be removed from the isolated diastereomer by a cleavage reaction to give a desirable enantiomer.

In addition, a mixture of enantiomers of a compound can also be directly separated by a chromatography method using a chiral solid phase well known in the art. Alternatively, one of the enantiomers can also be obtained by using a substantially pure optically active starting material or stereoselective synthesis (asymmetric induction) of a prochiral intermediate using a chiral auxiliary or an asymmetric catalyst.

The absolute steric configuration can be determined based on the X-ray crystal analysis of the resultant crystalline product or intermediate. In this case, a resultant crystalline product or intermediate derivatized with a reagent having an asymmetric center with a known steric configuration may be used where necessary.

The compound of the formula [I-a] may be labeled with an isotope (²H, ³H, ¹⁴C, ³⁵S and the like).

A compound of the formula [I-a] or a pharmaceutically acceptable salt thereof is preferably a substantially purified compound of the formula [I-a] or a pharmaceutically acceptable salt thereof. Further preferably, it is a compound of the formula [I-a] or a pharmaceutically acceptable salt thereof that is purified to a purity of not less than 80%.

The pharmaceutical composition of the present invention may be produced by appropriately admixing a suitable amount of a compound of the formula [I-a] or a pharmaceutically acceptable salt thereof with at least one kind of a pharmaceutically acceptable carrier according to a method known in the art of pharmaceutical preparations. The content of the compound of the formula [I-a] or a pharmaceutically acceptable salt thereof in the pharmaceutical composition varies depending on the dosage form, the dose and the like. It is, for example, 0.1 to 100 wt% of the whole composition.

A dosage form of the compound of formula [I-a] or a pharmaceutically acceptable salt thereof includes an oral preparation such as a tablet, a capsule, a granule, a powder, a lozenge, a syrup, an emulsion, and a suspension or a parenteral preparation such as an external preparation, a suppository, an injection, an eye drop, a nasal preparation, and a pulmonary preparation.

Examples of the "pharmaceutically acceptable carrier" include various organic or inorganic carrier substances conventionally used as preparation materials, and include excipient, disintegrant, binder, fluidizer, lubricant and the like for solid preparations, and solvent, solubilizing agent, suspending agent, isotonic agent, buffering agent, soothing agent and the like for liquid preparations and base, emulsifier, wetting agent, stabilizer, stabilizing agent, dispersing agent, plasticizer, pH adjuster, absorption promoter, gelling agent, preservative, filler, dissolving agent, solubilizing agents, suspending agent and the like for semisolid preparation s. Where necessary, moreover, additives such as preservative, antioxidant, colorant, sweetening agent and the like may also be used.

Examples of the "excipient" include lactose, sucrose, D-mannitol, D-sorbitol, cornstarch, dextrin, crystalline cellulose, crystalline cellulose, carmellose, carmellose calcium, sodium carboxymethyl starch, low-substituted hydroxypropylcellulose, gum arabic and the like.

Examples of the "disintegrant" include carmellose, carmellose calcium, carmellose sodium, sodium carboxymethyl starch, croscarmellose sodium, crospovidone, low-substituted hydroxypropylcellulose, hydroxypropylmethylcellulose, crystalline cellulose and the like.

Examples of the "binder" include hydroxypropylcellulose, hydroxypropylmethylcellulose, povidone, crystalline cellulose, sucrose, dextrin, starch, gelatin, carmellose sodium, gum arabic and the like.

Examples of the "fluidizer" include light anhydrous silicic acid, magnesium stearate and the like.

Examples of the "lubricant" include magnesium stearate, calcium stearate, talc and the like.

Examples of the "solvent" include purified water, ethanol, propylene glycol, macrogol, sesame oil, corn oil, olive oil and the like.

Examples of the "solubilizing agents" include propylene glycol, D-mannitol, benzyl benzoate, ethanol, triethanolamine, sodium carbonate, sodium citrate and the like.

Examples of the "suspending agent" include benzalkonium chloride, carmellose, hydroxypropylcellulose, propylene glycol, povidone, methylcellulose, glycerol monostearate and the like.

Examples of the "isotonic agent" include glucose, D-sorbitol, sodium chloride, D-mannitol and the like.

Examples of the "buffering agent" include sodium hydrogenphosphate, sodium acetate, sodium carbonate, sodium citrate and the like.

Examples of the "soothing agent" include benzyl alcohol and the like.

Examples of the "base" include water, animal and vegetable oils (olive oil, corn oil, peanut oil, sesame oil, castor oil and the like), lower alcohols (ethanol, propanol, propylene glycol, 1,3-butyleneglycol, phenol and the like), higher fatty acid and ester thereof, waxes, higher alcohol, polyhydric alcohol, hydrocarbons (white petrolatum, liquid paraffin, paraffin and the like), hydrophilic petrolatum, purified lanolin, water absorption ointment, hydrous lanolin, hydrophilic ointment, starch, pullulan, gum arabic, gum tragacanth, gelatin, dextran, cellulose derivative (methylcellulose, carboxymethylcellulose, hydroxyethylcellulose, hydroxypropylcellulose and the like), synthetic polymer (carboxyvinyl polymer, sodium polyacrylate, poly(vinyl alcohol), polyvinylpyrrolidone and the like), propylene glycol, macrogol (macrogol 200 - 600 and the like), and a combination of two or more kinds thereof.

Examples of the "preservative" include ethyl paraoxybenzoate, chlorobutanol, benzyl alcohol, sodium dehydroacetate, sorbic acid and the like.

Examples of the "antioxidant" include sodium sulfite, ascorbic acid and the like.

Examples of the "colorant" include food colors (e.g., Food Color Red No. 2 or 3, Food Color yellow No. 4 or 5 etc.), β-carotene and the like.

Examples of the "sweetening agent" include saccharin sodium, dipotassium glycyrrhizinate, aspartame and the like.

The pharmaceutical composition of the present invention can be administered orally or parenterally (topical, rectal, intravenous administration, intramuscular, subcutaneous, and the like) to mammals other than human (e.g., mouse, rat, hamster, guinea pig, rabbit, cat, dog, swine, bovine, horse, sheep, monkey and the like) and human. The dose varies depending on the subject of administration, disease, symptom, dosage form, administration route and the like. For example, the daily dose for oral administration to an adult patient is generally within the range of about 0.01 mg to 1 g, based on the compound of the formula [I-a] as the active ingredient. This amount can be administered in one to several portions.

The compound of the formula [I-a] or a pharmaceutically acceptable salt thereof has a PDHK inhibitory action, and is useful for the treatment and/or prophylaxis of various diseases or conditions expected to be improved by controlling PDHK activity. Examples of various diseases or conditions expected to be improved by controlling PDHK activity include diseases such as diabetes (type 1 diabetes, type 2 diabetes), insulin resistance syndrome, metabolic syndrome, hyperglycemia, hyperlactacidemia, diabetic complications (diabetic neuropathy, diabetic retinopathy, diabetic nephropathy, cataract), cardiac failure (acute cardiac failure, chronic cardiac failure), cardiomyopathy, myocardial ischemia, myocardial infarction, angina pectoris, dyslipidemia, atherosclerosis, peripheral arterial disease, intermittent claudication, chronic obstructive pulmonary diseases, brain ischemia, cerebral apoplexy, mitochondrial disease, mitochondrial encephalomyopathy, cancer, pulmonary hypertension (pulmonary arterial hypertension), Alzheimer disease, vascular dementia (large-vessel type or small-vessel type vascular dementia), glaucoma, diabetic retinopathy, retinopathy of prematurity, retinal vein occlusion, ischemic optic neuropathy, chronic kidney disease and the like.

The symptoms of Alzheimer disease include a decline in cognitive function, psychological symptoms and behavioral disorder and the like.

To "inhibit PDHK" means to eliminate or attenuate the activity of PDHK by inhibit the function thereof. For example, it means to inhibit the function as PDHK based on the conditions in the below-mentioned Experimental Example 1. To "inhibit PDHK", human PDHK is preferably inhibited. To "inhibit PDHK", preferably, "PDHK2 is inhibited".

The "PDHK inhibitor" means a substance that binds to PDHK and inhibits the function of PDHK. As the "PDHK inhibitor", preferred is a "human PDHK inhibitor". As the "PDHK inhibitor", preferred is an "inhibitor of PDHK2".

In the present specification, the "treatment" also includes improvement of symptoms, prevention of severity, maintenance of remission, prevention of exacerbation, and further, prevention of recurrence.

In the present specification, the "prevention" or "prophylaxis" means to suppress the onset of symptoms.

In the present specification, presentation of preferred embodiments and options of the compound, method, use and composition of the present invention also includes combinations of preferred embodiments and options as long as they can be combined and are free of inconsistency.

The production methods of the compound of the formula [I-a] or a pharmaceutically acceptable salt thereof are explained in the following. However, the production method of the compound of the formula [I-a] or a pharmaceutically acceptable salt thereof is not limited to such production methods.

The compound obtained in each step can be isolated or purified as necessary by conventional methods such as distillation, recrystallization, column chromatography and the like. In some cases, the next step may be performed without isolation or purification. When the reaction to be performed in each step is an anhydrous reaction, it is preferably performed in an inert gas atmosphere of argon, nitrogen and the like.

### [Production Method 1]

The compound of the formula [I-a1] can be obtained by Production Method 1 shown by the following scheme. 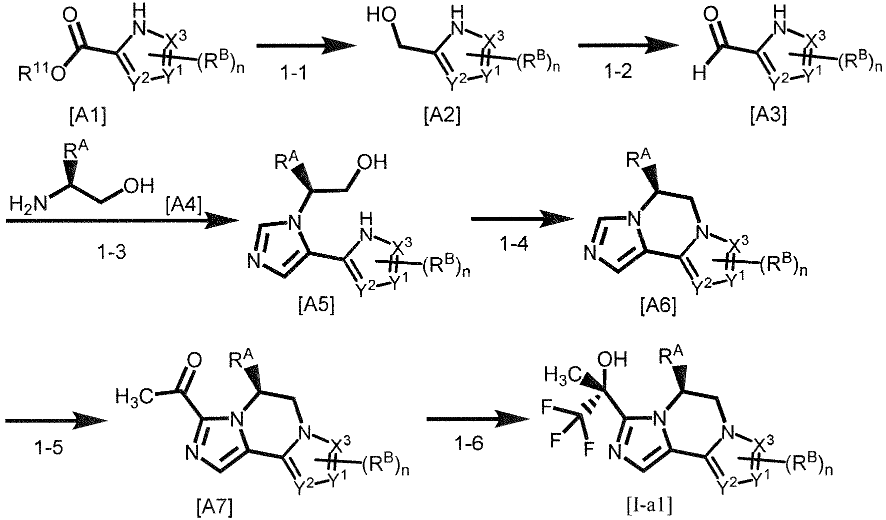 wherein R¹¹ is C₁₋₄ alkyl; and each other symbol is as defined in the aforementioned formula [I-a].

### Step 1-1

Compound [A2] can be obtained by reduction of an ester group of compound [A1]. For example, compound [A2] can be obtained by reacting compound [A1] with a reducing agent in a solvent at -40°C to room temperature.

Examples of the reducing agent include lithium aluminum hydride, diisobutylaluminum hydride and lithium borohydride.

Examples of the solvent include tetrahydrofuran, diethyl ether and cyclopentyl methyl ether.

Compound [A1] may be a commercially available product, or may be obtained by appropriately converting a commercially available product by a method well known to those of ordinary skill in the art.

### Step 1-2

Compound [A3] can be obtained by oxidation of a hydroxy group of compound [A2]. For example, compound [A3] can be obtained by reacting compound [A2] with an oxidizing agent in a solvent under ice-cooling to room temperature.

Examples of the oxidizing agent include manganese dioxide, Dess-Martin periodinane and sulfur trioxide-pyridine complex.

Examples of the solvent include tetrahydrofuran, dimethoxyethane, toluene, dimethyl sulfoxide, chloroform and dichloromethane.

### Step 1-3

Compound [A5] can be obtained by an imination reaction of compound [A3] and compound [A4], and a cyclization reaction using p-toluenesulfonylmethyl isocyanide. For example, an imination reaction of compound [A3] and compound [A4] is performed in a solvent at room temperature to 60°C. Successively, the resultant product is reacted with p-toluenesulfonylmethyl isocyanide in a solvent in the presence of a base under ice-cooling to room temperature to give compound [A5].

Examples of the solvent of the imination reaction include methanol and dimethylformamide.

Examples of the base include potassium carbonate.

Examples of the solvent of the cyclization reaction include dimethoxyethane.

Compounds [A3] and [A4] may be commercially available products, or may be obtained by appropriately converting a commercially available product by a method well known to those of ordinary skill in the art.

### Step 1-4

Compound [A6] can be obtained by an intramolecular Mitsunobu reaction of compound [A5]. For example, compound [A6] can be obtained by reacting compound [A5] with phosphine and azodicarboxylic acid diester in a solvent at room temperature to 100°C.

Examples of the phosphine include trioctylphosphine, tributylphosphine and triphenylphosphine.

Examples of the azodicarboxylic acid diester include diisopropyl azodicarboxylate and di-tert-butyl azodicarboxylate.

Examples of the solvent include toluene, tetrahydrofuran and 2-methyltetrahydrofuran.

### Step 1-5

Compound [A7] can be obtained by reacting compound [A6] with N-methoxy-N-methylacetamide. For example, compound [A7] can be obtained by reacting compound [A6] with N-methoxy-N-methylacetamide in a solvent at -78°C to room temperature in the presence of a base.

Examples of the base include n-butyllithium and lithium diisopropylamide.

Examples of the solvent include cyclopentyl methyl ether, tetrahydrofuran and toluene.

### Step 1-6

Compound [I-a1] can be obtained by reacting compound [A7] with (trifluoromethyl)trimethylsilane. For example, compound [I-a1] can be obtained by reacting compound [A7] with (trifluoromethyl)trimethylsilane in a solvent in the presence of an additive under ice-cooling to room temperature.

Examples of the additive include tetra-n-butylammonium fluoride, lithium acetate, potassium carbonate and cesium fluoride.

Examples of the solvent include tetrahydrofuran, dimethylformamide and dimethylacetamide.

The R^{A} group of compound [A7] becomes a steric hindrance, and the reaction proceeds in a diastereoselective manner. The steric configuration of compound [I-a1] can be assumed from the reaction mechanism and can be confirmed by X-ray crystal structure analysis.

### [Production Method 2]

The compound of the formula [I-a2] can be obtained by Production Method 2 shown by the following scheme. 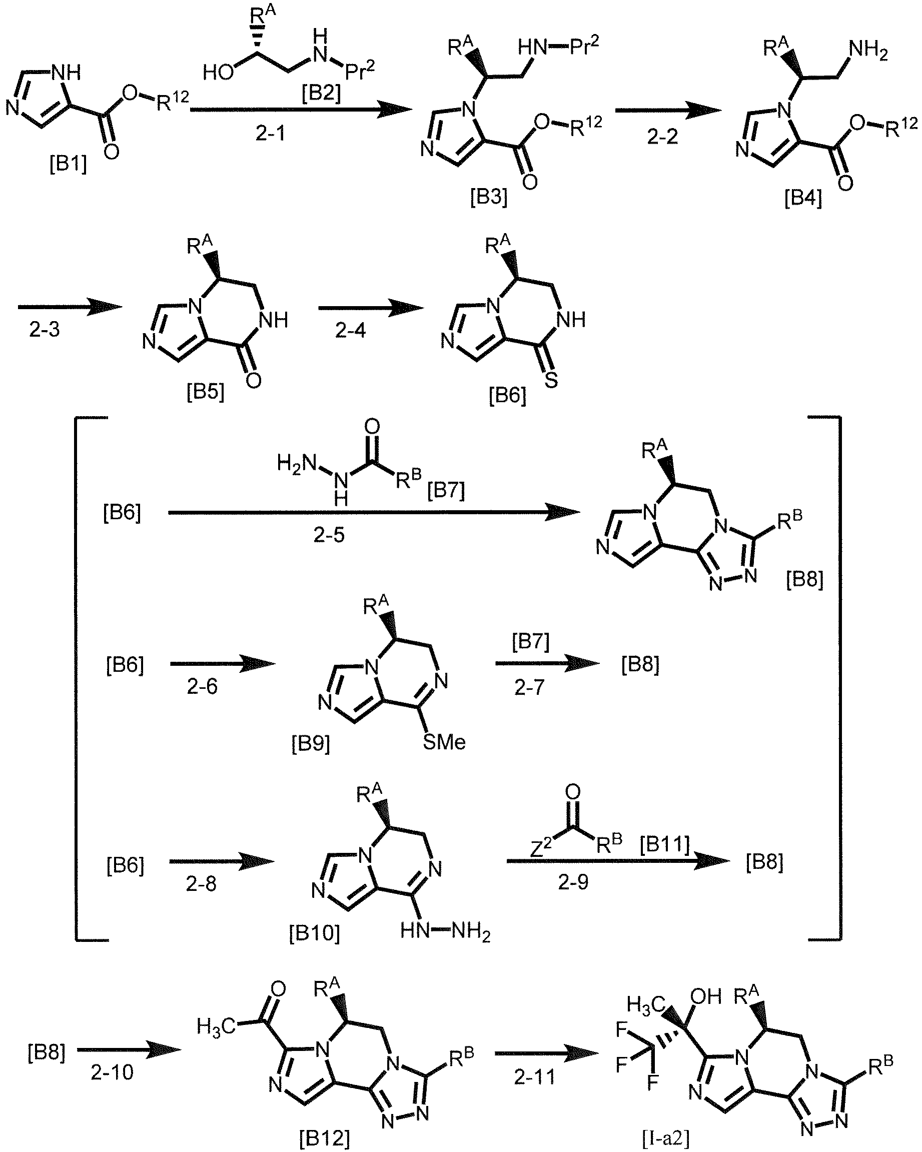
wherein R¹² is C₁₋₄ alkyl;
Pr² is an amino-protecting group such as tert-butoxycarbonyl and the like;
Z² is R^{B}C(O)O- or chloro; and
each other symbol is as defined in the aforementioned formula [I-a].

### Step 2-1

Compound [B3] can be obtained by a Mitsunobu reaction of compound [B1] and compound [B2]. For example, compound [B3] can be obtained by reacting compound [B1] with compound [B2], phosphine and azodicarboxylic acid diester in a solvent at room temperature to 100°C.

Examples of the phosphine include trioctylphosphine, tributylphosphine and triphenylphosphine.

Examples of the azodicarboxylic acid diester include diisopropyl azodicarboxylate and di-tert-butyl azodicarboxylate.

Examples of the solvent include toluene and tetrahydrofuran.

Compound [B1] and compound [B2] may be commercially available products, or may be obtained by appropriately converting a commercially available product by a method well known to those of ordinary skill in the art.

### Step 2-2

Compound [B4] can be obtained by deprotection of an amino group of compound [B3]. For example, when Pr² is tert-butoxycarbonyl, compound [B4] can be obtained by treating compound [B3] with an acid in a solvent under ice-cooling to room temperature. Compound [B4] may be obtained as a salt with the acid used in this reaction.

Examples of the acid include trifluoroacetic acid and hydrochloric acid.

Examples of the solvent include tetrahydrofuran and ethyl acetate.

### Step 2-3

Compound [B5] can be obtained by lactamization of compound [B4]. For example, compound [B5] can be obtained by reacting compound [B4] with a base in a solvent under ice-cooling to room temperature.

Examples of the base include sodium hydrogen carbonate.

Examples of the solvent include methanol and water.

### Step 2-4

Compound [B6] can be obtained by reacting compound [B5] with a sulfur reagent. For example, compound [B6] can be obtained by reacting compound [B5] with a sulfur reagent in a solvent at room temperature to 110°C.

Examples of the sulfur reagent include Lawesson's reagent (2,4-bis(4-methoxyphenyl)-1,3,2,4-dithiadiphosphetane-2,4-disulfide).

Examples of the solvent include toluene and pyridine.

### Step 2-5

Compound [B8] can be obtained by a cyclization reaction of compound [B6] and compound [B7]. For example, compound [B8] can be obtained by reacting compound [B6] with compound [B7] in a solvent at 100°C to 200°C. Where necessary, a microwave apparatus may also be used.

Examples of the solvent include n-butanol and N-methylpyrrolidone.

Compound [B7] may be a commercially available product, or may be obtained by appropriately converting a commercially available product by a method well known to those of ordinary skill in the art.

### Steps 2-6 and 2-7

Compound [B9] is obtained by methylation of compound [B6], and then compound [B8] can also be obtained by reacting compound [B9] with compound [B7]. The methylation of compound [B6] can be performed, for example, by reacting compound [B6] with a methylating agent in a solvent under ice-cooling to room temperature. Compound [B9] may also be obtained as a salt such as hydrogen iodide salt or the like.

Examples of the methylating agent include methyl iodide.

Examples of the solvent include dimethylformamide and acetone.

The reaction of compound [B9] and compound [B7] can be performed, for example, by an operation similar to that in Step 2-5.

Compound [B7] may be a commercially available product, or may be obtained by appropriately converting a commercially available product by a method well known to those of ordinary skill in the art.

### Steps 2-8 and 2-9

Compound [B10] is obtained by reacting compound [B6] with hydrazine, and then compound [B8] can also be obtained by reacting compound [B10] with compound [B11]. The reaction of compound [B6] and hydrazine can be performed, for example, by reacting compound [B6] with hydrazine in a solvent at room temperature to 80°C.

Examples of the solvent include ethanol and isopropanol.

The reaction of compound [B10] and compound [B11] can be performed by reacting compound [B10] with compound [B11] in a solvent in the presence of an acid under ice-cooling to room temperature.

Examples of the acid include trifluoroacetic acid.

Examples of the solvent include chloroform.

Compound [B11] may be a commercially available product, or may be obtained by appropriately converting a commercially available product by a method well known to those of ordinary skill in the art.

### Step 2-10

Compound [B12] can be obtained by reacting compound [B8] with N-methoxy-N-methylacetamide. For example, compound [B12] can be obtained by an operation similar to that in Step 1-5.

### Step 2-11

Compound [I-a2] can be obtained by reacting compound [B12] with (trifluoromethyl)trimethylsilane. For example, compound [I-a2] can be obtained by an operation similar to that in Step 1-6.

### [Production Method 3]

The compound of the formula [I-a3] can be obtained by Production Method 3 shown by the following scheme. 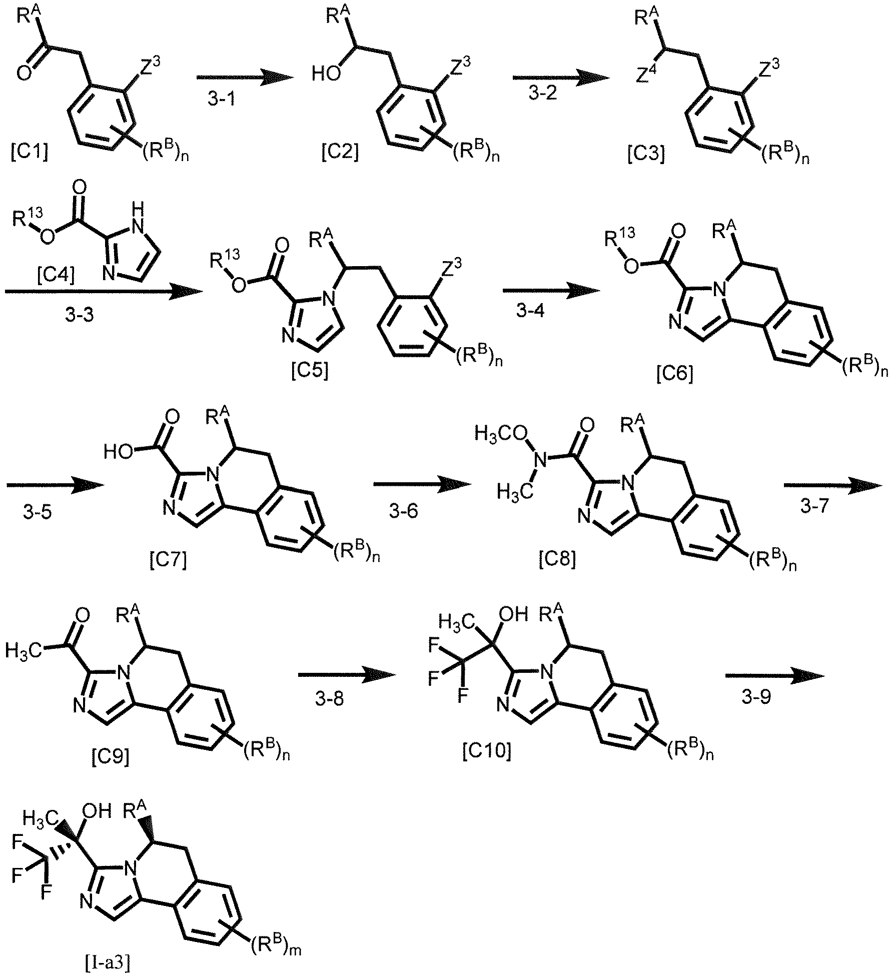
wherein R¹³ is C₁₋₄ alkyl;
Z³ is a leaving group such as bromo, iodo,
trifluoromethanesulfonyloxy or the like;
Z⁴ is a leaving group such as chloro, bromo, methanesulfonyloxy or the like; and
each other symbol is as defined in the aforementioned formula [I-a].

### Step 3-1

Compound [C2] can be obtained by reduction of a carbonyl group of compound [C1]. For example, compound [C2] can be obtained by reacting compound [C1] with a reducing agent in a solvent under ice-cooling to room temperature.

Examples of the reducing agent include sodium borohydride.

Examples of the solvent include tetrahydrofuran and methanol.

Compound [C1] may be a commercially available product, or may be obtained by appropriately converting a commercially available product by a method well known to those of ordinary skill in the art.

### Step 3-2

Compound [C3] can be obtained by conversion of a hydroxy group of compound [C2] to a leaving group. For example, when Z⁴ is methanesulfonyloxy, compound [C3] can be obtained by reacting compound [C2] with methanesulfonic anhydride in a solvent in the presence of a base under ice-cooling.

Examples of the base include triethylamine.

Examples of the solvent include tetrahydrofuran, chloroform and dichloromethane.

### Step 3-3

Compound [C5] can be obtained by reacting compound [C3] with compound [C4]. For example, compound [C5] can be obtained by reacting compound [C3] with compound [C4] in a solvent in the presence of a base at room temperature to 80°C.

Examples of the base include cesium carbonate.

Examples of the solvent include dimethylformamide.

Compound [C4] may be a commercially available product, or may be obtained by appropriately converting a commercially available product by a method well known to those of ordinary skill in the art.

### Step 3-4

Compound [C6] can be obtained by an intramolecular cyclization reaction of compound [C5]. For example, compound [C6] can be obtained by reacting compound [C5] in a solvent in the presence of a metal catalyst, a ligand and a base at 120°C.

Examples of the metal catalyst include palladium(II) acetate.

Examples of the ligand include di-1-adamantyl-n-butylphosphine and 2-dicyclohexylphosphino-2',6'-dimethoxybiphenyl.

Examples of the base include potassium carbonate.

Examples of the solvent include dimethylacetamide.

### Step 3-5

Compound [C7] can be obtained by hydrolysis of an ester group of compound [C6]. For example, compound [C7] can be obtained by treating compound [C6] with an alkali in a solvent under ice-cooling to 60°C.

Examples of the alkali include lithium hydroxide and sodium hydroxide.

Examples of the solvent include methanol, ethanol and water.

### Step 3-6

Compound [C8] can be obtained by an amidation reaction of compound [C7] and N,O-dimethylhydroxylamine. For example, compound [C8] can be obtained by reacting compound [C7] with N,O-dimethylhydroxylamine in a solvent in the presence of a base and a condensing agent under ice-cooling to room temperature.

Examples of the base include diisopropylethylamine and triethylamine.

Examples of the condensing agent include HATU.

Examples of the solvent include dimethylformamide.

### Step 3-7

Compound [C9] can be obtained by reacting compound [C8] with methylmagnesium halide. For example, compound [C9] can be obtained by reacting compound [C8] with methylmagnesium halide in a solvent at 0°C to room temperature.

Examples of the methylmagnesium halide include methylmagnesium bromide.

Examples of the solvent include tetrahydrofuran and diethyl ether.

### Step 3-8

Compound [C10] can be obtained by reacting compound [C9] with (trifluoromethyl)trimethylsilane. For example, compound [C10] can be obtained by an operation similar to that in Step 1-6.

### Step 3-9

Compound [I-a3] can be obtained by purifying compound [C10] by chiral column chromatography. The steric configuration of compound [I-a3] can be determined, for example, by X-ray crystal structure analysis.

### [Production Method 4]

The compound of the formula [I-a4] can be obtained by Production Method 4 shown by the following scheme. 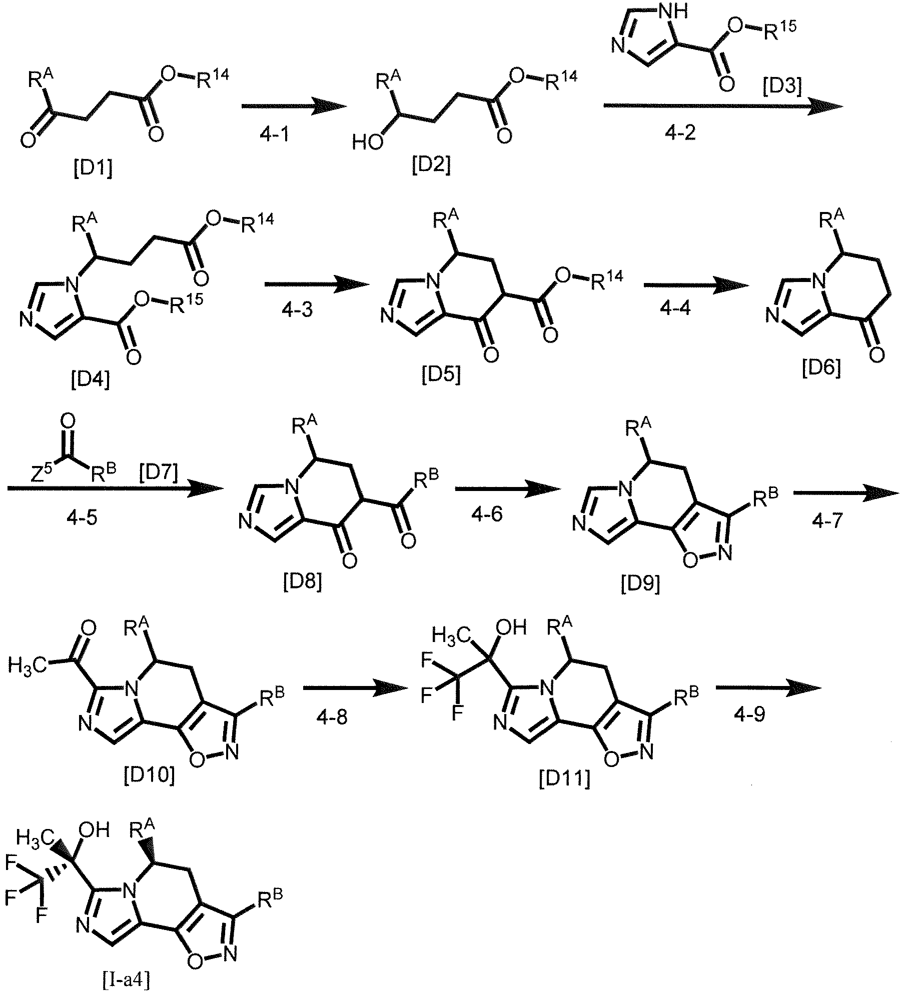
wherein R¹⁴ and R¹⁵ are each independently C₁₋₄ alkyl;
Z⁵ is C₁₋₄ alkoxy or chloro; and
each other symbol is as defined in the aforementioned formula [I-a].

### Step 4-1

Compound [D2] can be obtained by reduction of a carbonyl group of compound [D1]. For example, compound [D2] can be obtained by reacting compound [D1] with a reducing agent in a solvent at -78°C to room temperature.

Examples of the reducing agent include sodium borohydride.

Examples of the solvent include methanol and tetrahydrofuran.

Compound [D1] may be a commercially available product, or may be obtained by appropriately converting a commercially available product by a method well known to those of ordinary skill in the art.

### Step 4-2

Compound [D4] can be obtained by a Mitsunobu reaction of compound [D2] and compound [D3]. For example, compound [D4] can be obtained by an operation similar to that in Step 2-1.

Compound [D3] may be a commercially available product, or may be obtained by appropriately converting a commercially available product by a method well known to those of ordinary skill in the art.

### Step 4-3

Compound [D5] can be obtained by an intramolecular Claisen condensation of compound [D4]. For example, compound [D5] can be obtained by treating compound [D4] with a base in a solvent at room temperature to 110°C.

Examples of the base include potassium tert-butoxide.

Examples of the solvent include toluene and tetrahydrofuran.

### Step 4-4

Compound [D6] can be obtained by decarboxylation of an ester group of compound [D5]. For example, compound [D6] can be obtained by treating compound [D5] with an acid or sodium chloride in a solvent at 100°C to 160°C.

Examples of the acid include hydrochloric acid.

Examples of the solvent include water and dimethyl sulfoxide.

### Steps 4-5 and 4-6

Compound [D9] can be obtained by reacting compound [D6] with compound [D7] to give compound [D8], and subjecting compound [D8] and hydroxylamine to a cyclization reaction. Compound [D8] can be obtained, for example, by reacting compound [D6] with compound [D7] in a solvent in the presence of a base at 0°C to 70°C.

Examples of the base include sodium hydride and lithium bis(trimethylsilyl)amide.

Examples of the solvent include tetrahydrofuran.

Compound [D7] may be a commercially available product, or may be obtained by appropriately converting a commercially available product by a method well known to those of ordinary skill in the art.

Compound [D9] can be obtained, for example, by reacting compound [D8] with hydroxylamine in a solvent at 70°C to 110°C, and an acid may be used where necessary.

Examples of the acid include concentrated sulfuric acid.

Examples of the solvent include acetic acid and methanol.

### Step 4-7

Compound [D10] can be obtained by reacting compound [D9] with N-methoxy-N-methylacetamide. For example, compound [D10] can be obtained by an operation similar to that in Step 1-5.

### Step 4-8

Compound [D11] can be obtained by reacting compound [D10] with (trifluoromethyl)trimethylsilane. For example, compound [D11] can be obtained by an operation similar to that in Step 1-6.

### Step 4-9

Compound [I-a4] can be obtained by purifying compound [D11] by chiral column chromatography. The steric configuration of compound [I-a4] can be determined, for example, by X-ray crystal structure analysis.

### [Production Method 4-a]

In Step 4-6 of Production Method 4, a compound [D12] represented by the formula: 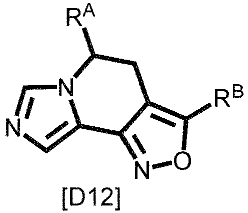 can be obtained along with compound [D9]. Compound [I-a16] represented by the formula: 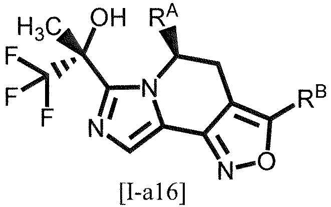 can be obtained by reactions similar to those in Step 4-7 to Step 4-9 and using compound [D12]. The steric configuration of compound [I-a16] can be determined, for example, by X-ray crystal structure analysis.

### [Production Method 5]

The compound of the formula [I-a5] can be obtained by Production Method 5 shown by the following scheme. 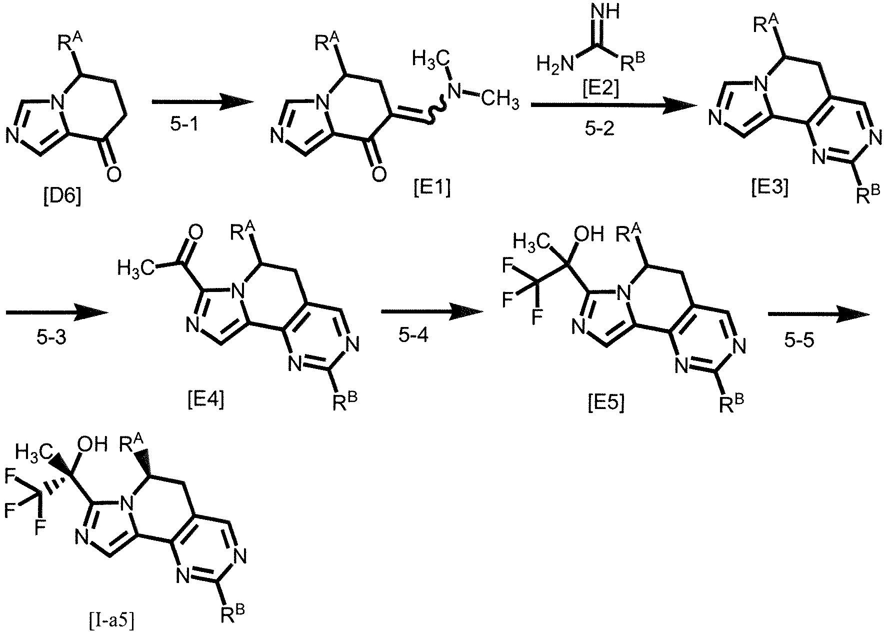 wherein each symbol is as defined in the aforementioned formula [I-a].

### Step 5-1

Compound [E1] can be obtained by reacting compound [D6] with tert-butoxy bis(dimethylamino)methane. For example, compound [E1] can be obtained by reacting compound [D6] with tert-butoxy bis(dimethylamino)methane in a solvent at room temperature to 110°C.

Examples of the solvent include dioxane.

### Step 5-2

Compound [E3] can be obtained by a cyclization reaction of compound [E1] and compound [E2]. For example, compound [E3] can be obtained by reacting compound [E1] with compound [E2] in a solvent in the presence of a base at room temperature to 78°C.

Examples of the base include sodium ethoxide.

Examples of the solvent include ethanol.

Compound [E2] may be a commercially available product, or may be obtained by appropriately converting a commercially available product by a method well known to those of ordinary skill in the art.

### Step 5-3

Compound [E4] can be obtained by reacting compound [E3] with N-methoxy-N-methylacetamide. For example, compound [E4] can be obtained by an operation similar to that in Step 1-5.

### Step 5-4

Compound [E5] can be obtained by reacting compound [E4] with (trifluoromethyl)trimethylsilane. For example, compound [E5] can be obtained by an operation similar to that in Step 1-6.

### Step 5-5

Compound [I-a5] can be obtained by purifying compound [E5] by chiral column chromatography. The steric configuration of compound [I-a5] can be determined, for example, by X-ray crystal structure analysis.

### [Production Method 6]

The compound of the formula [I-a6] can be obtained by Production Method 6 shown by the following scheme. 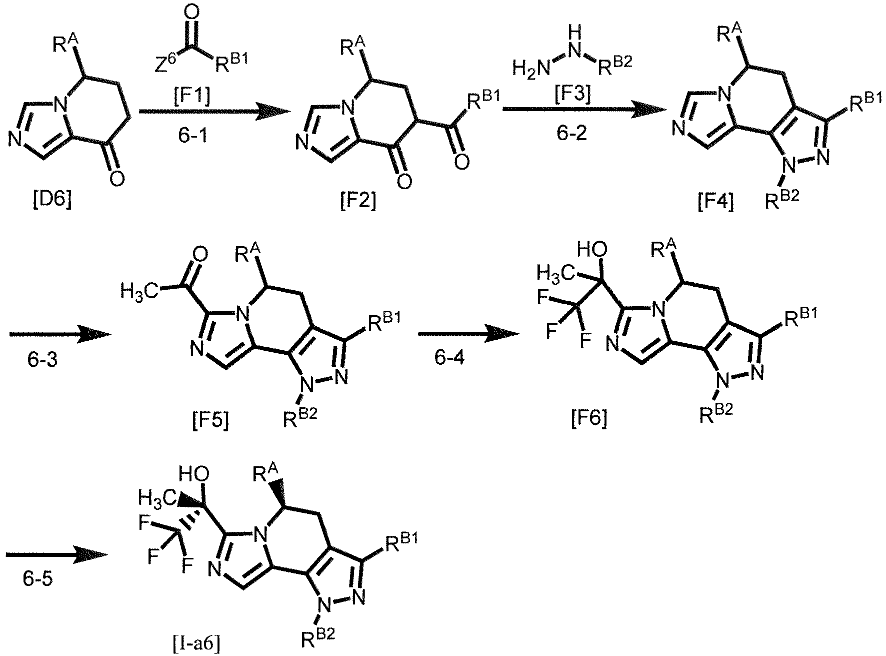
wherein R^{B1} and R^{B2} are each independently as defined for R^{B} in the aforementioned formula [I-a];
Z⁶ is C₁₋₄ alkoxy or chloro; and
each other symbol is as defined in the aforementioned formula [I-a].

### Step 6-1

Compound [F2] can be obtained by reacting compound [D6] with compound [F1]. For example, compound [F2] can be obtained by an operation similar to that in Step 4-5.

Compound [F1] may be a commercially available product, or may be obtained by appropriately converting a commercially available product by a method well known to those of ordinary skill in the art.

### Step 6-2

Compound [F4] can be obtained by a pyrazole cyclization reaction of compound [F2] and compound [F3]. For example, compound [F4] can be obtained by reacting compound [F2] with compound [F3] in a solvent at room temperature to 100°C.

Examples of the solvent include acetic acid and ethanol.

Compound [F3] may be a commercially available product, or may be obtained by appropriately converting a commercially available product by a method well known to those of ordinary skill in the art.

### Step 6-3

Compound [F5] can be obtained by reacting compound [F4] with N-methoxy-N-methylacetamide. For example, compound [F5] can be obtained by an operation similar to that in Step 1-5.

### Step 6-4

Compound [F6] can be obtained by reacting compound [F5] with (trifluoromethyl)trimethylsilane. For example, compound [F6] can be obtained by an operation similar to that in Step 1-6.

### Step 6-5

Compound [I-a6] can be obtained by purifying compound [F6] by chiral column chromatography. The steric configuration of compound [I-a6] can be determined, for example, by X-ray crystal structure analysis.

### [Production Method 7]

The compound of the formula [I-a7] can be obtained by Production Method 7 shown by the following scheme. 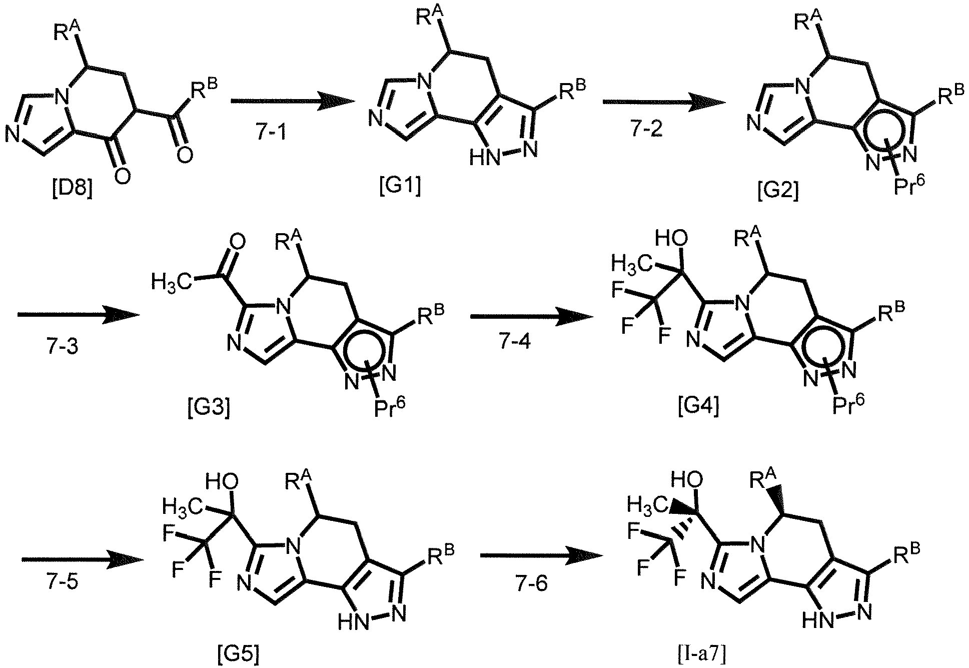
wherein Pr⁶ is a pyrazole-protecting group such as p-methoxybenzyl and the like; and
each other symbol is as defined in the aforementioned formula [I-a].

### Step 7-1

Compound [G1] can be obtained by a pyrazole cyclization reaction of compound [D8] and hydrazine. For example, compound [G1] can be obtained by an operation similar to that in Step 6-2.

### Step 7-2

Compound [G2] can be obtained by introduction of a protecting group into pyrazole of compound [G1]. For example, when Pr⁶ is a p-methoxybenzyl group, compound [G2] can be obtained by reacting compound [G1] with p-methoxybenzyl chloride in a solvent in the presence of a base under ice-cooling to room temperature. The protecting group Pr⁶ may be bonded to either of the two nitrogen atoms of pyrazole. Examples of the solvent include tetrahydrofuran. Examples of the base include sodium hydride.

### Step 7-3

Compound [G3] can be obtained by reacting compound [G2] with N-methoxy-N-methylacetamide. For example, compound [G3] can be obtained by an operation similar to that in Step 1-5.

### Step 7-4

Compound [G4] can be obtained by reacting compound [G3] with (trifluoromethyl)trimethylsilane. For example, compound [G4] can be obtained by an operation similar to that in Step 1-6.

### Step 7-5

Compound [G5] can be obtained by deprotection of pyrazole of compound [G4]. For example, when Pr⁶ is a p-methoxybenzyl group, compound [G5] can be obtained by treating compound [G4] with an acid in a solvent at 60°C to 80°C.

Examples of the acid include trifluoroacetic acid.

Examples of the solvent include dichloromethane.

### Step 7-6

Compound [I-a7] can be obtained by purifying compound [G5] by chiral column chromatography. The steric configuration of compound [I-a7] can be determined, for example, by X-ray crystal structure analysis.

### [Production Method 8]

The compound of the formula [I-a8] can be obtained by Production Method 8 shown by the following scheme. 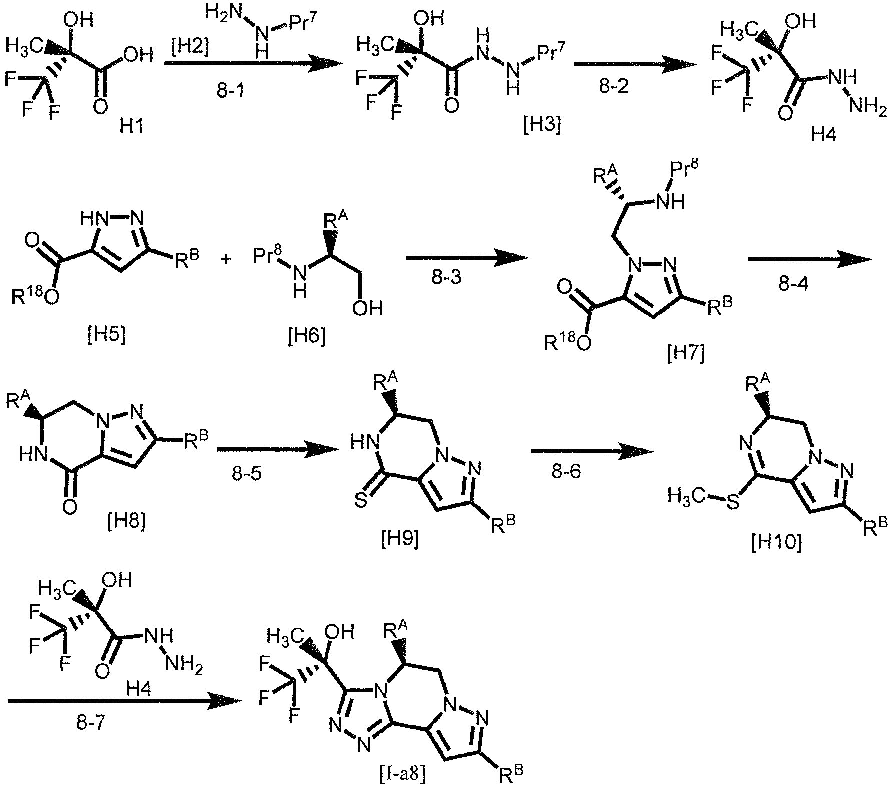
wherein R¹⁸ is C₁₋₄ alkyl;
Pr⁷ and Pr⁸ are each an amino-protecting group such as tert-butoxycarbonyl and the like; and
each other symbol is as defined in the aforementioned formula [I-a].

### Step 8-1

Compound [H3] can be obtained by reacting (R)-3,3,3-trifluoro-2-hydroxy-2-methylpropionic acid (compound H1) and compound [H2]. For example, compound [H3] can be obtained by reacting compound H1 with compound [H2] in a solvent in the presence of a condensing agent under ice-cooling to room temperature.

Examples of the condensing agent include 1-(3-dimethylaminopropyl)-3-ethylcarbodiimide hydrochloride and 1-hydroxybenzotriazole monohydrate.

Examples of the solvent include acetonitrile and dimethylformamide.

Compound [H2] may be a commercially available product, or may be obtained by appropriately converting a commercially available product by a method well known to those of ordinary skill in the art.

### Step 8-2

(R)-3,3,3-Trifluoro-2-hydroxy-2-methylpropanehydrazine (compound H4) can be obtained by deprotection of compound [H3]. For example, when Pr⁷ is tert-butoxycarbonyl, compound H4 can be obtained by treating compound [H3] with an acid in a solvent under ice-cooling to room temperature. Compound H4 may be obtained as a salt with the acid used in this reaction.

Examples of the acid include trifluoroacetic acid and hydrochloric acid.

Examples of the solvent include tetrahydrofuran, ethyl acetate and chloroform.

### Step 8-3

Compound [H7] can be obtained by a Mitsunobu reaction of compound [H5] and compound [H6]. For example, compound [H7] can be obtained by an operation similar to that in Step 2-1.

Compound [H5] and compound [H6] may be commercially available products, or may be obtained by appropriately converting a commercially available product by a method well known to those of ordinary skill in the art.

### Step 8-4

Compound [H8] can be obtained by deprotection of an amino group of compound [H7] and lactamization. The deprotection of the amino group can be performed, for example, by an operation similar to that in Step 2-2. The lactamization can be performed, for example, by an operation similar to that in Step 2-3.

### Step 8-5

Compound [H9] can be obtained by reacting compound [H8] with a sulfur reagent. For example, compound [H9] can be obtained by an operation similar to that in Step 2-4.

### Step 8-6

Compound [H10] can be obtained by methylation of compound [H9]. For example, compound [H10] can be obtained by an operation similar to that in Step 2-6.

### Step 8-7

Compound [I-a8] can be obtained by a cyclization reaction of compound [H10] and compound H4. For example, compound [I-a8] can be obtained by reacting compound [H10] with compound H4 in a solvent in the presence of an acid at 60°C to 120°C.

Examples of the acid include acetic acid.

Examples of the solvent include isopropanol and water.

### [Production Method 9]

A compound having a desired R^{B} can be obtained by functional group conversion in an appropriate stage in Production Methods 1 to 8. For example, compounds [I-a10] to [I-a15] can be obtained by converting compound [I-a9] obtained in any of Production Methods 1 to 8 by Production Method 9 shown by the following scheme. 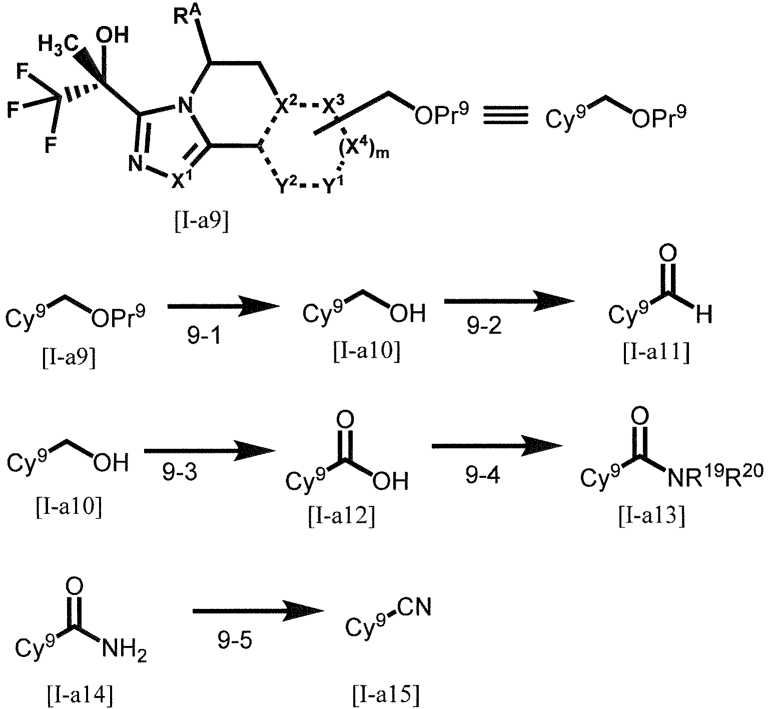
wherein Pr⁹ is a hydroxy-protecting group such as p-methoxybenzyl and the like;
R¹⁹ and R²⁰ are each independently hydrogen or C₁₋₄ alkyl; and each other symbol is as defined in the aforementioned formula [I-a].

### Step 9-1

Compound [I-a10] can be obtained by deprotection of a hydroxy group of compound [I-a9]. For example, compound [I-a10] can be obtained by treating compound [I-a9] with an acid in a solvent at room temperature.

Examples of the acid include trifluoroacetic acid.

Examples of the solvent include dichloromethane.

### Step 9-2

Compound [I-a11] can be obtained by oxidation of a hydroxy group of compound [I-a10]. For example, compound [I-a11] can be obtained by an operation similar to that in Step 1-2.

### Step 9-3

Compound [I-a12] can be obtained by oxidation of a hydroxy group of compound [I-a10]. For example, compound [I-a12] can be obtained by reacting compound [I-a10] with an oxidizing agent in a solvent at room temperature.

Examples of the oxidizing agent include potassium permanganate.

Examples of the solvent include acetone.

### Step 9-4

Compound [I-a13] can be obtained by amidation of compound [I-a12] and HNR¹⁹R²⁰. For example, compound [I-a13] can be obtained by reacting compound [I-a12] with HNR¹⁹R²⁰ in a solvent in the presence of a base and a condensing agent under ice-cooling to room temperature.

Examples of the base include diisopropylethylamine and triethylamine.

Examples of the condensing agent include HATU.

Examples of the solvent include dimethylformamide.

When R¹⁹ and R²⁰ of HNR¹⁹R²⁰ are each a hydrogen in this Step, compound [I-a14] can be obtained.

### Step 9-5

Compound [I-a15] can be obtained by a cyanation reaction of compound [I-a14]. For example, compound [I-a15] can be obtained by reacting compound [I-a14] with an acid anhydride in a solvent in the presence of a base under ice-cooling to room temperature.

Examples of the base include pyridine.

Examples of the acid anhydride include trifluoroacetic anhydride.

Examples of the solvent include 1,4-dioxane.

### [Production Method 10]

The compound of the formula [I-a10] can be obtained by Production Method 10 shown by the following scheme. 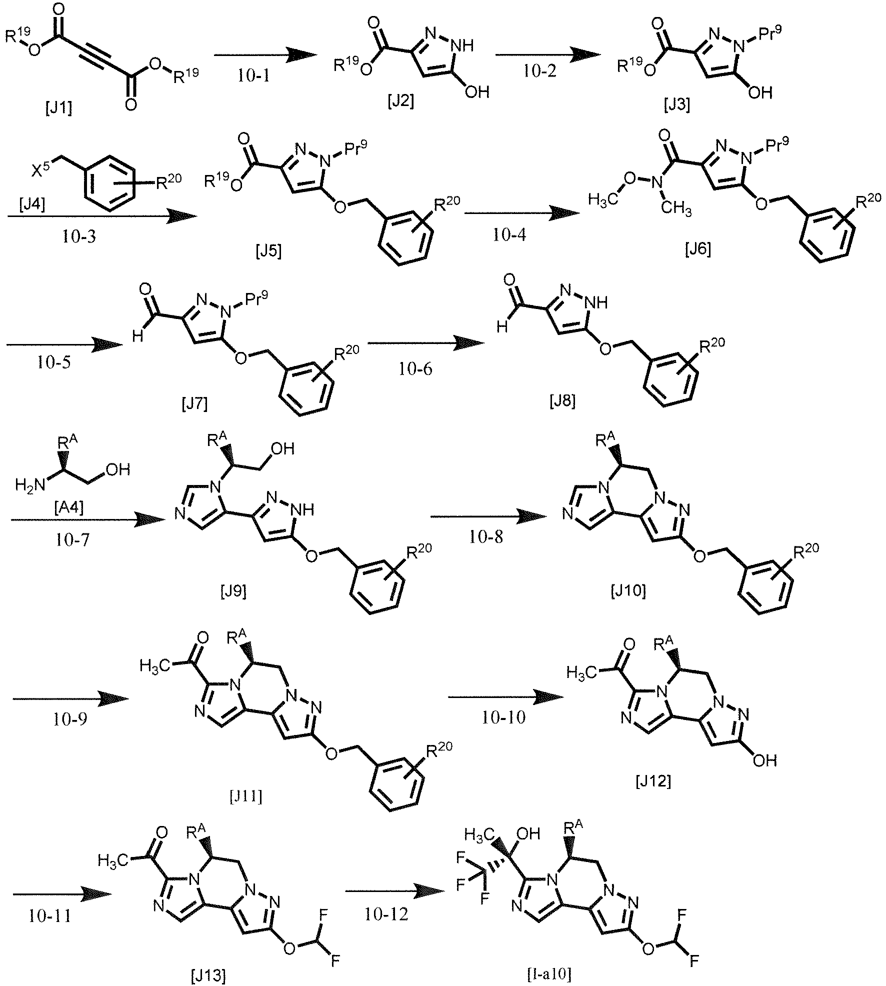
wherein R¹⁹ is C₁₋₄ alkyl;
R²⁰ is hydrogen, halogen, C₁₋₄ alkyl or nitro;
Pr⁹ is a pyrazole-protecting group such as 2-tetrahydropyranyl and the like;
X⁵ is a leaving group such as chloro, bromo, methanesulfonyloxy and the like; and
each other symbol is as defined in the aforementioned formula [I-a].

### Step 10-1

Compound [J2] can be obtained by reacting compound [J1] with hydrazine. For example, compound [J2] can be obtained by reacting compound [J1] with hydrazine in a solvent at room temperature. Where necessary, the reaction may be performed in the presence of an acid.

Examples of the solvent include acetonitrile, toluene and ethanol.

Examples of the acid include acetic acid.

Compound [J1] may be a commercially available product, or may be obtained by appropriately converting a commercially available product by a method well known to those of ordinary skill in the art.

### Step 10-2

Compound [J3] can be obtained by introduction of a protecting group into pyrazole of compound [J2]. For example, when Pr⁹ is a 2-tetrahydropyranyl group, compound [J3] can be obtained by reacting compound [J2] with 3,4-dihydro-2H-pyran in a solvent in the presence of an acid at room temperature.

Examples of the solvent include acetonitrile and N,N-dimethylformamide.

Examples of the acid include pyridinium p-toluenesulfonate and p-toluenesulfonic acid.

### Step 10-3

Compound [J5] can be obtained by reacting compound [J3] with compound [J4]. For example, compound [J5] can be obtained by reacting compound [J3] with compound [J4] in a solvent in the presence of a base at room temperature.

Examples of the solvent include N-methylpyrrolidone, N,N-dimethylformamide, acetonitrile, toluene, isopropyl acetate, tetrahydrofuran and dimethyl sulfoxide.

Examples of the base include potassium carbonate, sodium carbonate, cesium carbonate, lithium carbonate, potassium tert-butoxide, potassium acetate, potassium phosphate, 1,8-diazabicyclo[5.4.0]undec-7-ene (DBU), N,N-diisopropylethylamine.

Compound [J4] may be a commercially available product, or may be obtained by appropriately converting a commercially available product by a method well known to those of ordinary skill in the art.

### Step 10-4

Compound [J6] can be obtained by hydrolyzing ester of compound [J5], and then performing an amidation reaction with N,O-dimethylhydroxylamine. For example, compound [J6] can be obtained by reacting compound [J5] with an alkali to perform hydrolysis of ester in a solvent and reacting the obtained compound with N,O-dimethylhydroxylamine in the presence of a condensing agent at room temperature.

Examples of the alkali include sodium hydroxide.

Examples of the condensing agent include a combination of WSC-HCl and HOBt.

Examples of the solvent include 1,2-dimethoxyethane.

### Step 10-5

Compound [J7] can be obtained by reduction of compound [J6]. For example, compound [J7] can be obtained by reacting compound [J6] with a reducing agent in a solvent under ice-cooling.

Examples of the reducing agent include sodium bis(2-methoxyethoxy) aluminum hydride.

Examples of the solvent include toluene and 1,2-dimethoxyethane.

### Step 10-6

Compound [J8] can be obtained by deprotection of the protecting group of pyrazole of compound [J7]. For example, when Pr⁹ is a 2-tetrahydropyranyl group, compound [J8] can be obtained by treating compound [J7] with an acid in a solvent at room temperature.

Examples of the acid include hydrochloric acid, methanesulfonic acid, sulfuric acid and phosphoric acid.

Examples of the solvent include 1,2-dimethoxyethane.

### Step 10-7

Compound [J9] can be obtained by reacting compound [J8] with compound [A4]. For example, compound [J9] can be obtained by an operation similar to that in Step 1-3.

### Step 10-8

Compound [J10] can be obtained by an intramolecular Mitsunobu reaction of compound [J9]. For example, compound [J10] can be obtained by an operation similar to that in Step 1-4. Compound [J10] may be obtained as a salt with an acid such as hydrochloric acid and the like.

### Step 10-9

Compound [J11] can be obtained by reacting compound [J10] with N-methoxy-N-methylacetamide. For example, compound [J11] can be obtained by an operation similar to that in Step 1-5.

### Step 10-10

Compound [J12] can be obtained by deprotection of compound [J11]. For example, compound [J12] can be obtained by treating compound [J11] with an acid at room temperature to 50°C. Compound [J12] may be obtained as a salt with an acid used.

Examples of the acid include concentrated hydrochloric acid.

### Step 10-11

Compound [J13] can be obtained by reacting compound [J12] with diethyl (bromodifluoromethyl)phosphonate. For example, compound [J13] can be obtained by reacting compound [J12] with diethyl (bromodifluoromethyl)phosphonate in a solvent in the presence of a base at room temperature.

Examples of the solvent include acetonitrile, 1,2-dimethoxyethane and tetrahydrofuran.

Examples of the base include potassium hydroxide, lithium hydroxide and tetrabutylammonium hydroxide.

### Step 10-12

Compound [I-a10] can be obtained by reacting compound [J13] with (trifluoromethyl)trimethylsilane. For example, compound [I-a10] can be obtained by an operation similar to that in Step 1-6. The R^{A} group of compound [J13] becomes a steric hindrance, and the reaction proceeds in a diastereoselective manner. The steric configuration of compound [I-a10] can be assumed from the reaction mechanism and can be confirmed by X-ray crystal structure analysis.

### Examples

The production method of the compound of the formula [I-a] or a pharmaceutically acceptable salt thereof of the present invention is specifically explained by way of the following Production Examples. However, the production method of the compound of the formula [I-a] or a pharmaceutically acceptable salt thereof is not limited by the Production Examples.

Unless otherwise specified, % shows wt%. Unless otherwise specified, the ratio of a mixed solvent is a volume mixing ratio.

In the Examples, abbreviations mean the following.
DMSO: dimethyl sulfoxide
M: mol/L
N: normality
HATU: O-(7-azabenzotriazol-1-yl)-N,N,N',N'-tetramethyluronium hexafluorophosphate
WSC-HCl: 1-ethyl-3-(3-dimethylaminopropyl)carbodiimide hydrochloride
HOBt-H₂O: 1-hydroxybenzotriazole monohydrate

The measurement results of ¹H-NMR are indicated using the following abbreviations.
s: singlet, d: doublet, dd: double doublet, dt: double triplet,
t: triplet, q: quartet, dq: double quartet, m: multiplet, brs: broad singlet, brm: broad multiplet, J: coupling constant, Hz: Hertz

¹H-NMR spectrum was measured in CDCl₃ or DMSO-D₆ using tetramethylsilane as an internal standard, and all δ values are shown in ppm.

### Production Example 1

### Synthesis of (R)-2-((S)-9-(difluoromethoxy)-5-methyl-5,6-dihydroimidazo[1,5-a]pyrazolo[5,1-c]pyrazin-3-yl)-1,1,1-trifluoropropan-2-ol (compound of Example 23)

### Step 1

### ethyl 3-(benzyloxy)-1H-pyrazole-5-carboxylate

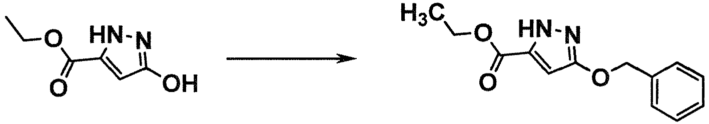

Ethyl 3-hydroxy-1H-pyrazole-5-carboxylate (10 g) was mixed with tetrahydrofuran (100 ml). To the mixture were added benzyl alcohol (7.99 ml) and triphenylphosphine (18.48 g). Under ice-cooling, diisopropyl azodicarboxylate (13.7 ml) was added dropwise, and the mixture was stirred at room temperature overnight. The reaction mixture was concentrated under reduced pressure, ethyl acetate (70 ml) and hexane (140 ml) were added, and the mixture was stirred at room temperature. The precipitated solid was filtered off, and the filtrate was concentrated under reduced pressure. The obtained residue was purified by silica gel column chromatography (hexane:ethyl acetate=92:8 to hexane:ethyl acetate=47:53) to give the title compound as a crude product (20 g). The crude product was used for the next step without further purification.

### Step 2

### (3-(benzyloxy)-1H-pyrazol-5-yl)methanol

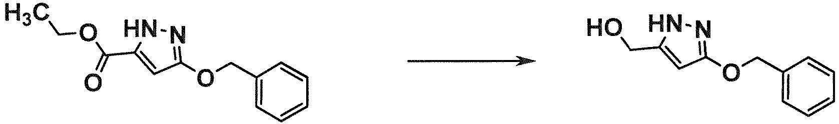

The crude product (19 g) of ethyl 3-(benzyloxy)-1H-pyrazole-5-carboxylate obtained in the previous step was dissolved in cyclopentyl methyl ether (70 ml). Under ice-cooling, the solution was added dropwise to a solution of lithium borohydride (4.17 g) in cyclopentyl methyl ether (150 ml). The reaction mixture was stirred at 0°C for 5 min. Under ice-cooling, a solution of methanol (8 ml) in cyclopentyl methyl ether (50 ml), methanol (8 ml), and methanol (8 ml) were successively added dropwise. The mixture was stirred at 0°C for 10 min. To the reaction mixture were added saturated aqueous ammonium chloride solution and 1N aqueous hydrochloric acid solution, and the mixture was extracted with ethyl acetate. The organic layer was washed with saturated aqueous sodium chloride solution and dried over sodium sulfate. Sodium sulfate was filtered off, and the filtrate was concentrated under reduced pressure. The obtained residue was purified by silica gel column chromatography (ethyl acetate:hexane=16:84 to ethyl acetate, thereafter ethyl acetate:methanol=82:18) to give the title compound (8.2 g).
¹H-NMR (400 MHz, CDCl₃) 4.66 (s, 2H), 5.19 (s, 2H), 5.64 (s, 1H), 7.26 - 7.47 (m, 5H)

### Step 3

### 3-(benzyloxy)-1H-pyrazole-5-carbaldehyde

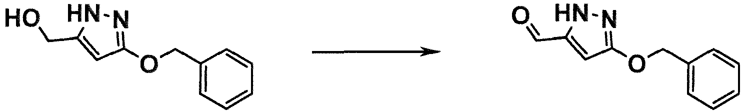

(3-(Benzyloxy)-1H-pyrazol-5-yl)methanol (8.2 g) obtained in the previous step was dissolved in 1,2-dimethoxyethane (164 ml). Manganese dioxide (41 g) was added at room temperature, and the mixture was stirred at 80°C for 1.5 hr. After allowing to cool, the mixture was filtered through celite and the filtrate was concentrated under reduced pressure to give the title compound as a crude product (5.4 g). The crude product was used for the next step without further purification.

### Step 4

### (S)-2-(5-(3-(benzyloxy)-1H-pyrazol-5-yl)-1H-imidazol-1-yl)propan-1-ol

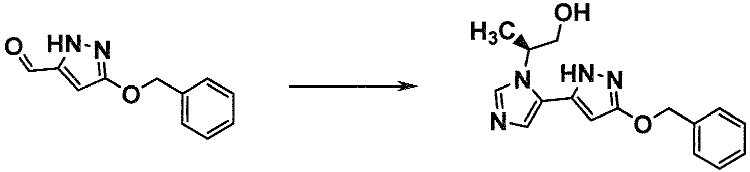

The crude product (5.4 g) of 3-(benzyloxy)-1H-pyrazole-5-carbaldehyde obtained in the previous step was mixed with methanol (54 ml). (S)-2-aminopropan-1-ol (2 g) was added at room temperature, and the mixture was stirred at room temperature overnight. Under ice-cooling, 1,2-dimethoxyethane (164 ml), p-toluenesulfonylmethyl isocyanide (7.82 g) and potassium carbonate (11.07 g) were added, and the mixture was stirred at room temperature overnight. The reaction mixture was filtered through celite, and the filtrate was concentrated under reduced pressure. To the obtained residue was added water, and the mixture was extracted with ethyl acetate. The organic layer was washed with saturated aqueous sodium chloride solution and dried over sodium sulfate. Sodium sulfate was filtered off, and the filtrate was concentrated under reduced pressure. The obtained residue was purified by silica gel column chromatography (ethyl acetate:hexane=25:75 to ethyl acetate, thereafter ethyl acetate:methanol=60:40) to give the title compound as a crude product (4.1 g).

### Step 5

### (S)-9-(benzyloxy)-5-methyl-5,6-dihydroimidazo[1,5-a]pyrazolo[5,1-c]pyrazine

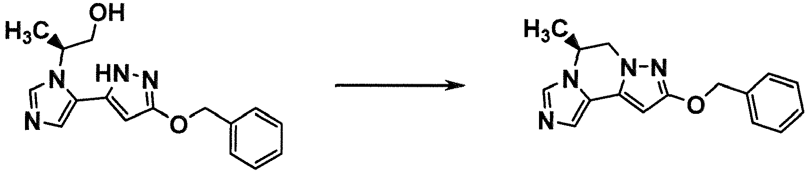

The crude product (4.1 g) of (S)-2-(5-(3-(benzyloxy)-1H-pyrazol-5-yl)-1H-imidazol-1-yl)propan-1-ol obtained in the previous step and triphenylphosphine (4.69 g) were mixed with tetrahydrofuran (123 ml). Under ice-cooling, di-tert-butyl azodicarboxylate (4.11 g) was added, and the mixture was stirred at room temperature overnight. The reaction mixture was concentrated under reduced pressure and purified by silica gel column chromatography (ethyl acetate:hexane=25:75 to ethyl acetate, thereafter ethyl acetate:methanol=60:40) to give the title compound (2.98 g).
¹H-NMR (400 MHz, CDCl₃) 1.64 (d, J=6.70Hz, 3H), 3.97 (dd, J=12.95, 8.09Hz, 1H), 4.31 (dd, J=12.95, 4.39Hz, 1H), 4.54 - 4.65 (m, 1H), 5.21 (s, 2H), 5.86 (s, 1H), 7.26 (s, 1H), 7.29 - 7.49 (m, 5H), 7.59 (s, 1H)

### Step 6

### (S)-1-(9-(benzyloxy)-5-methyl-5,6-dihydroimidazo[1,5-a]pyrazolo[5,1-c]pyrazin-3-yl)ethan-1-one

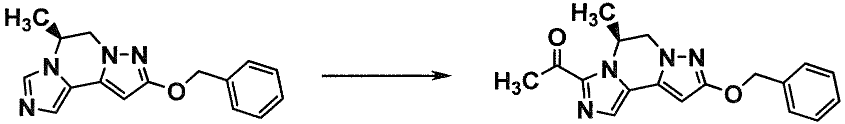

(S)-9-(Benzyloxy)-5-methyl-5,6-dihydroimidazo[1,5-a]pyrazolo[5,1-c]pyrazine (2.98 g) obtained in the previous step was mixed with tetrahydrofuran (29.8 ml). The mixture was cooled to -78°C, and 2M lithium diisopropylamide/tetrahydrofuran-heptane-ethylbenzene solution (13.29 ml) was added dropwise. The mixture was stirred at - 78°C for 30 min. To the mixture was added N-methoxy-N-methylacetamide (5.25 ml), and the mixture was stirred at -78°C for 1 hr. To the reaction mixture were added saturated aqueous ammonium chloride solution and ethyl acetate, and the precipitated solid was collected by filtration. The filtrate was partitioned, and the organic layer was washed with saturated aqueous sodium chloride solution and dried over sodium sulfate. Sodium sulfate was filtered off, and the filtrate was concentrated under reduced pressure. To the obtained residue was added ethyl acetate/hexane=1/1 (10 ml), and the mixture was stirred at room temperature. The precipitated solid was collected by filtration, combined with the solid collected earlier by filtration, mixed with ethyl acetate, and concentrated to give the title compound (2 g). ¹H-NMR (400 MHz, CDCl₃) 1.44 (d, J=6.58Hz, 3H), 2.69 (s, 3H), 4.19 (dd, J=13.45, 1.20Hz, 1H), 4.29 (dd, J=13.45, 4.63Hz, 1H), 5.24 (s, 2H), 5.75 - 5.84 (m, 1H), 5.98 (s, 1H), 7.32 - 7.50 (m, 6H)

### Step 7

### (R)-2-((S)-9-(benzyloxy)-5-methyl-5,6-dihydroimidazo[1,5-a]pyrazolo[5,1-c]pyrazin-3-yl)-1,1,1-trifluoropropan-2-ol

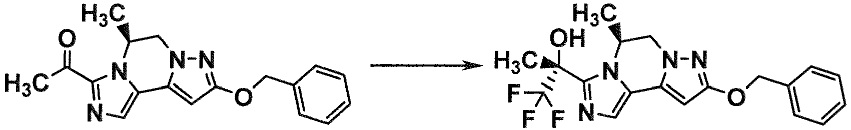

(S)-1-(9-(Benzyloxy)-5-methyl-5,6-dihydroimidazo[1,5-a]pyrazolo[5,1-c]pyrazin-3-yl)ethan-1-one (2 g) obtained in the previous step was mixed with tetrahydrofuran (29.8 ml). Under ice-cooling, to the mixture was added cesium fluoride (0.195 g), and (trifluoromethyl)trimethylsilane (1.088 ml) was added dropwise. The reaction mixture was stirred at room temperature for 20 min. Under ice-cooling, methanol (24.84 ml) and potassium carbonate (1.065 g) were added, and the mixture was stirred at room temperature for 45 min. The reaction mixture was concentrated under reduced pressure, water was added, and the mixture was extracted with ethyl acetate. The organic layer was washed with saturated aqueous sodium chloride solution and dried over sodium sulfate. Sodium sulfate was filtered off, and the filtrate was concentrated under reduced pressure. The obtained residue was purified by silica gel column chromatography (ethyl acetate:hexane=12:88 to 4:96) to give the title compound (2.2 g).
¹H-NMR (400 MHz, DMSO-D₆) 1.28 (d, J=6.70Hz, 3H), 1.80 (s, 3H), 4.12 - 4.24 (m, 2H), 5.15 (s, 2H), 5.27 - 5.36 (m, 1H), 6.04 (s, 1H), 7.23 (s, 1H), 7.28 (s, 1H), 7.29 - 7.46 (m, 5H)

### Step 8

### (S)-5-methyl-3-((R)-1,1,1-trifluoro-2-hydroxypropan-2-yl)-5,6-dihydroimidazo[1,5-a]pyrazolo[5,1-c]pyrazin-9-ol

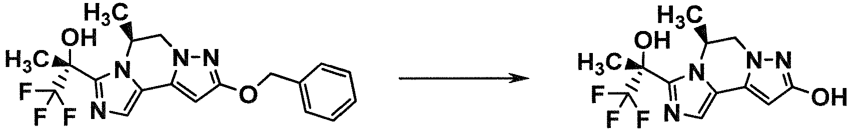

(R)-2-((S)-9-(Benzyloxy)-5-methyl-5,6-dihydroimidazo[1,5-a]pyrazolo[5,1-c]pyrazin-3-yl)-1,1,1-trifluoropropan-2-ol (2.2 g) obtained in the previous step and 10 % palladium carbon (0.44 g) were mixed with ethanol (44 g). Under a hydrogen atmosphere, the mixture was stirred at room temperature for 1.5 hr. The reaction mixture was filtered through celite, and the filtrate was concentrated under reduced pressure. The obtained residue was purified by silica gel column chromatography (ethyl acetate:hexane=16:84 to ethyl acetate, thereafter ethyl acetate:methanol=80:20). To the obtained crude product were successively added ethyl acetate (6 ml) and hexane (6 ml), and the mixture was stirred at room temperature. The precipitated solid was collected by filtration to give the title compound (1.82 g). The steric configuration of the title compound was determined by X-ray crystal structure analysis. ¹H-NMR (400 MHz, DMSO-D₆) 1.28 (d, J=6.58Hz, 3H), 1.81 (s, 3H), 4.07 - 4.11 (m, 2H), 5.23 - 5.36 (m, 1H), 5.75 (s, 1H), 7.20 (s, 1H), 7.26 (brs, 1H), 9.89 (brs, 1H)

### Step 9

### (R)-2-((S)-9-(difluoromethoxy)-5-methyl-5,6-dihydroimidazo[1,5-a]pyrazolo[5,1-c]pyrazin-3-yl)-1,1,1-trifluoropropan-2-ol

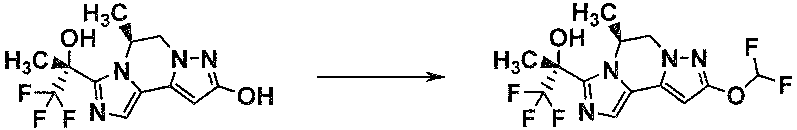

(S)-5-Methyl-3-((R)-1,1,1-trifluoro-2-hydroxypropan-2-yl)-5,6-dihydroimidazo[1,5-a]pyrazolo[5,1-c]pyrazin-9-ol (1 g) obtained in the previous step, potassium carbonate (0.914 g) and sodium chlorodifluoroacetate (1.009 g) were mixed with dimethylformamide (6 ml), and the mixture was stirred at 100°C for 3 hr. To the reaction mixture was added saturated aqueous ammonium chloride solution, and the mixture was extracted twice with ethyl acetate. The organic layer was washed successively with water and saturated aqueous sodium chloride solution and dried over sodium sulfate. Sodium sulfate was filtered off, and the filtrate was concentrated under reduced pressure. The obtained residue was purified by silica gel column chromatography (hexane:ethyl acetate=88:12 to ethyl acetate). To the obtained crude product was added ethyl acetate/hexane=1/2 (1.5 ml), and the mixture was stirred at room temperature. The precipitated solid was collected by filtration to give the title compound (0.315 g).
¹H-NMR (400 MHz, DMSO-D₆) 1.27 (d, J=6.70Hz, 3H), 1.81 (s, 3H), 4.20 - 4.30 (m, 2H), 5.31 - 5.40 (m, 1H), 6.31 (s, 1H), 7.27 (t, J=73.29Hz, 1H), 7.31 (s, 1H), 7.32 (s, 1H)

### Production Example 2

### Synthesis of (R)-1,1,1-trifluoro-2-((S)-9-(2-hydroxy-2-methylpropoxy)-5-methyl-5,6-dihydroimidazo[1,5-a]pyrazolo[5,1-c]pyrazin-3-yl)propan-2-ol (compound of Example 52)

### Step 1

### methyl 2-(((S)-5-methyl-3-((R)-1,1,1-trifluoro-2-hydroxypropan-2-yl)-5,6-dihydroimidazo[1,5-a]pyrazolo[5,1-c]pyrazin-9-yl)oxy)acetate

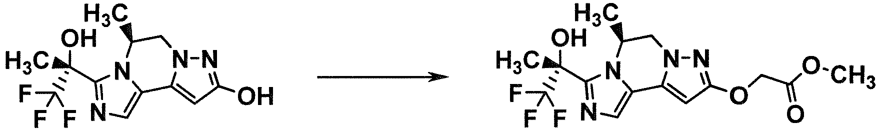

(S)-5-Methyl-3-((R)-1,1,1-trifluoro-2-hydroxypropan-2-yl)-5,6-dihydroimidazo[1,5-a]pyrazolo[5,1-c]pyrazin-9-ol (0.5 g) and potassium carbonate (0.288 g) were mixed with dimethylformamide (5 ml). Under ice-cooling, methyl bromoacetate (0.154 ml) was added, and the mixture was stirred at room temperature for 2 hr. To the reaction mixture was added saturated aqueous ammonium chloride solution, and the mixture was extracted with ethyl acetate. The organic layer was washed successively with water and saturated aqueous sodium chloride solution and dried over sodium sulfate. Sodium sulfate was filtered off, and the filtrate was concentrated under reduced pressure. The obtained residue was successively purified by silica gel chromatography (hexane:ethyl acetate=88:12 to ethyl acetate) and amino silica gel chromatography (ethyl acetate:hexane=12:88 to ethyl acetate, thereafter ethyl acetate:methanol=90:10) to give the title compound (0.411 g).
¹H-NMR (400 MHz, DMSO-D₆) 1.26 (d, J=6.47Hz, 3H), 1.80 (s, 3H), 3.67 (s, 3H), 4.08 - 4.21 (m, 2H), 4.78 (s, 2H), 5.24 - 5.36 (m, 1H), 6.03 (s, 1H), 7.25 (s, 1H), 7.27 (s, 1H)

### Step 2

### (R)-1,1,1-trifluoro-2-((S)-9-(2-hydroxy-2-methylpropoxy)-5-methyl-5,6-dihydroimidazo[1,5-a]pyrazolo[5,1-c]pyrazin-3-yl)propan-2-ol

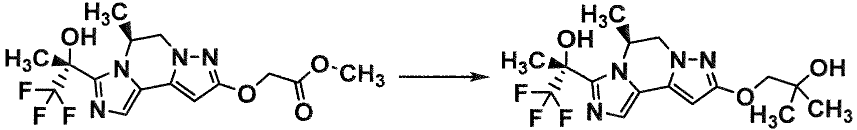

Methyl 2-(((S)-5-methyl-3-((R)-1,1,1-trifluoro-2-hydroxypropan-2-yl)-5,6-dihydroimidazo[1,5-a]pyrazolo[5,1-c]pyrazin-9-yl)oxy)acetate (0.411 g) obtained in the previous step was mixed with tetrahydrofuran (2 ml). Under ice-cooling, 1.08M methylmagnesium bromide/tetrahydrofuran solution (4 ml) was added, and the mixture was stirred at room temperature for 2 hr. To the reaction mixture was added saturated aqueous ammonium chloride solution, and the mixture was extracted twice with ethyl acetate. The organic layer was washed with saturated aqueous sodium chloride solution and dried over sodium sulfate. Sodium sulfate was filtered off, and the filtrate was concentrated under reduced pressure. The obtained residue was purified by silica gel chromatography (hexane:ethyl acetate=88:12 to ethyl acetate) and amino silica gel chromatography (ethyl acetate:hexane=24:76 to ethyl acetate). To the obtained crude product were successively added ethyl acetate (0.7 ml) and hexane (1.4 ml), and the mixture was stirred at room temperature. The precipitated solid was collected by filtration to give the title compound (0.251 g).
¹H-NMR (400 MHz, DMSO-D₆) 1.16 (s, 6H), 1.27 (d, J=6.47Hz, 3H), 1.80 (s, 3H), 3.82 (s, 2H), 4.09 - 4.19 (m, 2H), 4.57 (s, 1H), 5.25 - 5.36 (m, 1H), 5.98 (s, 1H), 7.22 (s, 1H), 7.26 (s, 1H)

### Production Example 3

### Synthesis of (R)-1,1,1-trifluoro-2-((S)-6-methyl-3-(trifluoromethyl)-5,6-dihydroimidazo[1,5-a][1,2,4]triazolo[3,4-c]pyrazin-8-yl)propan-2-ol (compound of Example 2)

### Step 1

### methyl (S)-1-(1-((tert-butoxycarbonyl)amino)propan-2-yl)-1H-imidazole-5-carboxylate

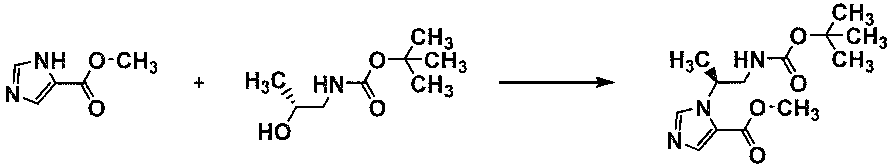

Methyl 1H-imidazole-5-carboxylate (250 g), tert-butyl (R)-(2-hydroxypropyl)carbamate (521 g) and trioctylphosphine (1505 ml) were mixed with toluene (1750 ml). The reaction solution was heated to 80°C, and 40% diisopropyl azodicarboxylate/toluene solution (1599 ml) was added dropwise. The reaction solution was stirred at 80°C for 1 hr. The mixture was extracted twice with 1N potassium hydrogen sulfate aqueous solution. The aqueous layer was washed with ethyl acetate:hexane=1:1. Under ice-cooling, to the aqueous layer was added 1.4M sodium carbonate aqueous solution (1L) by small portions, and the mixture was extracted twice with ethyl acetate. Sodium sulfate and silica gel were added, and the mixture was stirred at room temperature for 30 min. Sodium sulfate and silica gel were filtered off, and the filtrate was concentrated under reduced pressure. To the obtained residue was added ethyl acetate (250 ml), and the mixture was stirred at 80°C for 10 min. Hexane (1750 ml) was slowly added, and the mixture was stirred while allowing to cool to room temperature. The precipitated solid was collected by filtration to give the title compound (432.4 g).
¹H-NMR (400 MHz, CDCl₃) 1.39 (s, 9H), 1.54 (d, J=6.88Hz, 3H), 3.48 (t, J=6.28Hz, 2H), 3.85 (s, 3H), 4.63 (brs, 1H), 5.17 - 5.32 (m, 1H), 7.72 - 7.77 (m, 2H)

### Step 2

### methyl (S)-1-(1-aminopropan-2-yl)-1H-imidazole-5-carboxylate dihydrochloride

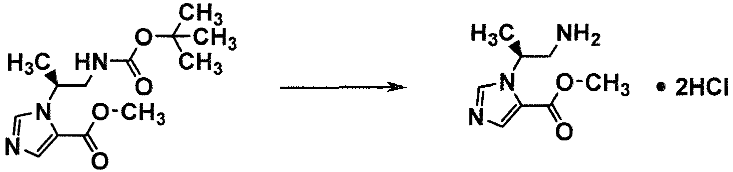

Methyl (S)-1-(1-((tert-butoxycarbonyl)amino)propan-2-yl)-1H-imidazole-5-carboxylate (432.4 g) obtained in the previous step was mixed with ethyl acetate (865 ml). Under ice-cooling, 4N hydrochloric acid/ethyl acetate (1526 ml) was added dropwise. The reaction mixture was stirred at room temperature for 4 hr. Under ice-cooling, 4N hydrochloric acid/ethyl acetate (382 ml) was added dropwise. The reaction mixture was stirred at room temperature for 1 hr. Under ice-cooling, 4N hydrochloric acid/ethyl acetate (38.2 ml) was added dropwise. The reaction mixture was stirred at room temperature for 1 hr. The precipitated solid was collected by filtration to give the title compound (388.89 g).
¹H-NMR (400 MHz, DMSO-D₆) 1.51 (d, J=6.94Hz, 3H), 3.20 - 3.36 (m, 1H), 3.42 - 3.57 (m, 1H), 3.85 (d, J=4.62Hz, 3H), 5.29 - 5.43 (m, 1H), 8.09 (s, 1H), 8.21 (brs, 3H), 8.95 (s, 1H)

### Step 3

### (S)-5-methyl-6,7-dihydroimidazo[1,5-a]pyrazin-8(5H)-one

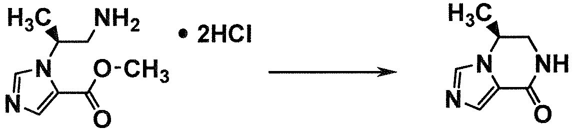

Methyl (S)-1-(1-aminopropan-2-yl)-1H-imidazole-5-carboxylate dihydrochloride (388.89 g) obtained in the previous step was mixed with methanol (1944 ml) and water (117 ml). To the mixture was added sodium carbonate (644 g), and the mixture was stirred at 90°C for 2 hr. Tetrahydrofuran (1944 ml) was added at room temperature, and the reaction mixture was filtered through celite. The filtrate was concentrated under reduced pressure, and azeotroped twice with toluene. To the obtained residue was added toluene, and the mixture was stirred at room temperature for 1 hr. The precipitated solid was collected by filtration to give the title compound (278.65 g).
¹H-NMR (400 MHz, DMSO-D₆) 1.46 (d, J=6.28Hz, 3H), 3.19 - 3.28 (m, 1H), 3.46 - 3.58 (m, 1H), 4.37 - 4.48 (m, 1H), 7.45 (s, 1H), 7.83 - 7.94 (m, 2H)

### Step 4

### (S)-5-methyl-6,7-dihydroimidazo[1,5-a]pyrazine-8(5H)-thione

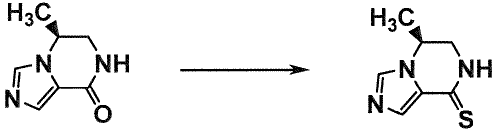

(S)-5-Methyl-6,7-dihydroimidazo[1,5-a]pyrazin-8(5H)-one (62.2 g) was mixed with toluene (600 ml) and pyridine (200 ml). To the mixture was added Lawesson's reagent (50 g), and the mixture was stirred at 110°C overnight. After allowing to cool, the solvent was decanted, and the gum-like substance was washed with toluene. To the obtained gum-like substance was added methanol (187 ml), and the mixture was stirred at room temperature. The precipitated solid was collected by filtration to give the title compound (39.9 g). The title compound (20.7 g) was obtained by a similar method.
¹H-NMR (400 MHz, DMSO-D₆) 1.43 (d, J=6.47Hz, 3H), 3.23 - 3.31 (m, 1H), 3.58 (dt, J=13.56, 4.10Hz, 1H), 4.39 - 4.52 (m, 1H), 7.61 (s, 1H), 7.96 (s, 1H), 10.03 (brs, 1H)

### Step 5

### (S)-6-methyl-3-(trifluoromethyl)-5,6-dihydroimidazo[1,5-a][1,2,4]triazolo[3,4-c]pyrazine

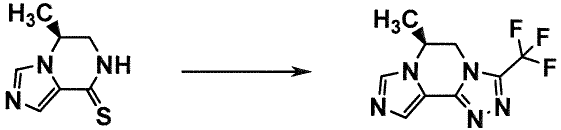

(S)-5-Methyl-6,7-dihydroimidazo[1,5-a]pyrazine-8(5H)-thione (3 g) obtained in the previous step was mixed with isopropyl alcohol (30 ml). Hydrazine hydrate (2.62 ml) was added at room temperature, and the mixture was stirred at 80°C for 3 hr. After allowing to cool, the reaction mixture was concentrated under reduced pressure, and azeotroped once with ethanol and twice with toluene. To the obtained residue were added trifluoroacetic acid (23.68 ml) and trifluoroacetic anhydride (12.65 ml), and the mixture was stirred at room temperature overnight. The reaction mixture was concentrated under reduced pressure and azeotroped twice with toluene. The obtained residue was purified by amino silica gel column chromatography (ethyl acetate:hexane=75:25 to ethyl acetate, thereafter ethyl acetate:methanol=80:20) to give the title compound (3.96 g).
¹H-NMR (400 MHz, DMSO-D₆) 1.57 (d, J=6.58Hz, 3H), 4.26 (dd, J=13.15, 8.67Hz, 1H), 4.64 (dd, J=13.15, 4.48Hz, 1H), 4.74 - 4.85 (m, 1H), 7.72 (s, 1H), 8.13 (s, 1H)

### Step 6

### (S)-1-(6-methyl-3-(trifluoromethyl)-5,6-dihydroimidazo[1,5-a][1,2,4]triazolo[3,4-c]pyrazin-8-yl)ethan-1-one

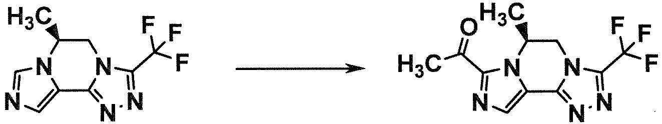

(S)-6-Methyl-3-(trifluoromethyl)-5,6-dihydroimidazo[1,5-a][1,2,4]triazolo[3,4-c]pyrazine (3.96 g) obtained in the previous step was mixed with tetrahydrofuran (80 ml). The mixture was cooled to -78°C, and 2M lithium diisopropylamide/tetrahydrofuran-heptane-ethylbenzene solution (24.67 ml) was added dropwise. The mixture was stirred at - 78°C for 30 min. To the mixture was added N-methoxy-N-methylacetamide (5.25 ml), and the mixture was stirred at -78°C for 35 min. To the reaction mixture was added saturated aqueous ammonium chloride solution, and the mixture was allowed to warm to room temperature, and extracted twice with ethyl acetate. The organic layer was washed with saturated aqueous sodium chloride solution and dried over sodium sulfate. Sodium sulfate was filtered off, and the filtrate was concentrated under reduced pressure. The obtained residue was purified by silica gel column chromatography (hexane:ethyl acetate=12:88 to ethyl acetate). To the obtained crude product was added toluene (12 ml), and the mixture was stirred at room temperature. The precipitated solid was collected by filtration to give the title compound (2.04 g).
¹H-NMR (400 MHz, DMSO-D₆) 1.27 (d, J=6.70Hz, 3H), 2.63 (s, 3H), 4.49 - 4.61 (m, 2H), 5.66 - 5.78 (m, 1H), 7.94 (s, 1H)

### Step 7

### (R)-1,1,1-trifluoro-2-((S)-6-methyl-3-(trifluoromethyl)-5,6-dihydroimidazo[1,5-a][1,2,4]triazolo[3,4-c]pyrazin-8-yl)propan-2-ol

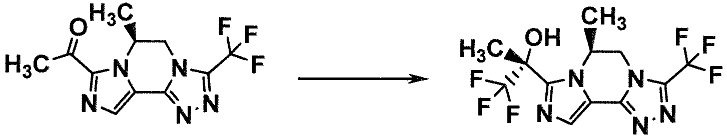

(S)-1-(6-Methyl-3-(trifluoromethyl)-5,6-dihydroimidazo[1,5-a][1,2,4]triazolo[3,4-c]pyrazin-8-yl)ethan-1-one (2.04 g) obtained in the previous step was mixed with tetrahydrofuran (40.8 ml). Under ice-cooling, cesium fluoride (0.217 g) and (trifluoromethyl)trimethylsilane (2.111 ml) were added, and the mixture was stirred at 0°C for 25 min. Under ice-cooling, methanol (24.48 ml) and potassium carbonate (1.186 g) were added, and the mixture was stirred at room temperature for 1 hr. To the reaction mixture was added silica gel (40 ml), the mixture was filtered through a silica gel (20 ml) pad, and the filtrate was concentrated under reduced pressure. The obtained residue was purified by silica gel column chromatography (hexane:ethyl acetate=16:84 to ethyl acetate). To the obtained crude product were added toluene (20 ml) and ethyl acetate (4 ml), and the mixture was stirred at 80°C. The precipitated solid was collected by filtration. To the obtained solid were added ethyl acetate (6 ml) and hexane (4 ml), and the mixture was stirred at room temperature. The precipitated solid was collected by filtration to give the title compound (1.171 g). The steric configuration of the title compound was determined by X-ray crystal structure analysis.
¹H-NMR (400 MHz, DMSO-D₆) 1.28 (d, J=6.47Hz, 3H), 1.86 (s, 3H), 4.48 (dd, J=13.64, 3.93Hz, 1H), 4.55 (d, J=13.64Hz, 1H), 5.45 - 5.51 (m, 1H), 7.49 (s, 1H), 7.75 (s, 1H)

### Production Example 4

### Synthesis of 1,1,1-trifluoro-2-(5-methyl-5,6-dihydroimidazo[5,1-a]isoquinolin-3-yl)propan-2-ol (compound of Example 1)

### Step 1

### 1-(2-bromophenyl)propan-2-ol

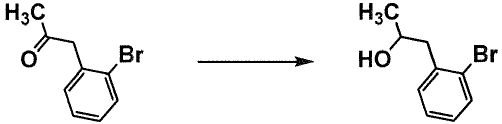

1-(2-Bromophenyl)propan-2-one (2 g) was mixed with methanol (10 ml). Under ice-cooling, to the mixture was added sodium borohydride (0.533 g), and the mixture was stirred at room temperature for 3 hr. To the reaction mixture was added saturated aqueous ammonium chloride solution, and the mixture was extracted twice with ethyl acetate. The organic layer was washed with saturated aqueous sodium chloride solution and dried over sodium sulfate. Sodium sulfate was filtered off, and the filtrate was concentrated under reduced pressure. The obtained residue was purified by silica gel chromatography (hexane:ethyl acetate=95:5 to 60:40) to give the title compound (1.937 g).
¹H-NMR (400 MHz, CDCl₃) 1.27 (d, J=6.01Hz, 3H), 1.46 (d, J=3.93Hz, 1H), 2.83 (dd, J=13.52, 7.98Hz, 1H), 2.96 (dd, J=13.52, 4.74Hz, 1H), 4.07 - 4.19 (m, 1H), 7.04 - 7.13 (m, 1H), 7.21 - 7.28 (m, 2H), 7.54 (d, J=8.09Hz, 1H)

### Step 2

### 1-(2-bromophenyl)propan-2-yl methanesulfonate

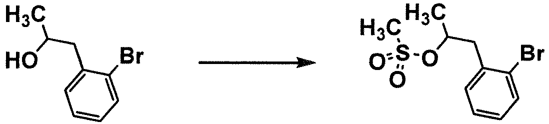

Methanesulfonic anhydride (2.11 g) and dichloromethane (10 ml) were mixed. Under ice-cooling, to the mixture were added 1-(2-bromophenyl)propan-2-ol (1.737 g) obtained in the previous step and triethylamine (3.38 ml), and the mixture was stirred at room temperature for 4 hr. The reaction mixture was concentrated under reduced pressure and purified by silica gel chromatography (hexane:ethyl acetate=95:5 to 60:40) to give the title compound (2.142 g).
¹H-NMR (400 MHz, CDCl₃) 1.51 (d, J=6.24Hz, 3H), 2.50 (s, 3H), 3.00 - 3.14 (m, 2H), 4.94 - 5.06 (m, 1H), 7.08 - 7.17 (m, 1H), 7.23 - 7.30 (m, 2H), 7.56 (d, J=7.86Hz, 1H)

### Step 3

### ethyl 1-(1-(2-bromophenyl)propan-2-yl)-1H-imidazole-2-carboxylate

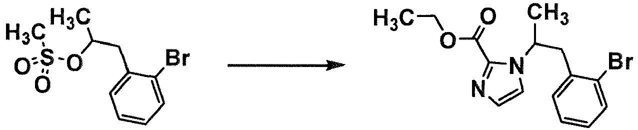

Ethyl 1H-imidazole-2-carboxylate (3.54 g) and cesium carbonate (3.29 g) were mixed with dimethylformamide (11.85 ml). The reaction solution was heated to 80°C, 1-(2-bromophenyl)propan-2-yl methanesulfonate (1.54 g) obtained in the previous step was added, and the mixture was stirred overnight. To the reaction mixture was added water, and the mixture was extracted twice with ethyl acetate. The organic layer was successively washed three times with water and once with saturated aqueous sodium chloride solution, and dried over sodium sulfate. Sodium sulfate was filtered off, and the filtrate was concentrated under reduced pressure. The obtained residue was purified by silica gel chromatography (hexane:ethyl acetate=88:12 to ethyl acetate) to give the title compound (0.383 g).
¹H-NMR (400 MHz, CDCl₃) 1.33 (t, J=7.17Hz, 3H), 1.54 (d, J=6.94Hz, 3H), 3.04 (dd, J=13.64, 7.86Hz, 1H), 3.27 (dd, J=13.64, 6.24Hz, 1H), 4.27 (q, J=7.17Hz, 2H), 5.74 - 5.86 (m, 1H), 6.87 (dd, J=7.51, 1.73Hz, 1H), 7.04 (td, J=7.63, 1.70Hz, 1H), 7.12 (td, J=7.46, 1.31Hz, 1H), 7.17 (s, 1H), 7.50 (dd, J=7.98, 1.27Hz, 1H)

### Step 4

### ethyl 5-methyl-5,6-dihydroimidazo[5,1-a]isoquinoline-3-carboxylate

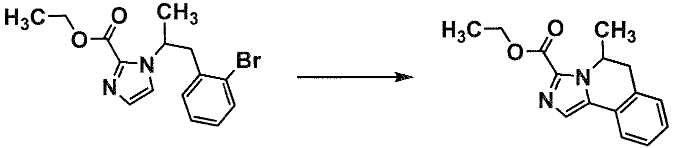

Ethyl 1-(1-(2-bromophenyl)propan-2-yl)-1H-imidazole-2-carboxylate (383 mg) obtained in the previous step, palladium(II) acetate (74.3 mg), potassium carbonate (305 mg) and di-1-adamantyl-n-butylphosphine (178 mg) were mixed with dimethylacetamide (3.7 ml). Under an argon atmosphere, the mixture was stirred at 120°C for 7 hr. Under ice-cooling, water was added, and the precipitated solid was collected by filtration. The filtrate was extracted twice with ethyl acetate. The organic layer was successively washed twice with water and once with saturated aqueous sodium chloride solution and dried over sodium sulfate. Sodium sulfate was filtered off, and the filtrate was concentrated under reduced pressure. The obtained residue and the solid collected by filtration were each purified by silica gel chromatography (hexane:ethyl acetate=88:12 to ethyl acetate) to give the title compound (145 mg).
¹H-NMR (400 MHz, CDCl₃) 1.26 (d, J=6.70Hz, 3H), 1.44 (t, J=7.05Hz, 3H), 2.84 (d, J=15.95Hz, 1H), 3.36 (dd, J=15.95, 6.24Hz, 1H), 4.35 - 4.50 (m, 2H), 5.59 - 5.69 (m, 1H), 7.23 - 7.33 (m, 3H), 7.49 (s, 1H), 7.59 (d, J=6.94Hz, 1H)

### Step 5

### N-methoxy-N,5-dimethyl-5,6-dihydroimidazo[5,1-a]isoquinoline-3-carboxamide

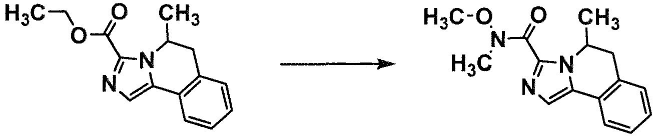

Ethyl 5-methyl-5,6-dihydroimidazo[5,1-a]isoquinoline-3-carboxylate (82.4 mg) obtained in the previous step was mixed with ethanol (1.6 ml). Under ice-cooling, 2N sodium hydroxide aqueous solution was added, and the mixture was stirred at room temperature for 4 hr. The reaction mixture was concentrated, and azeotroped with toluene. The obtained residue was mixed with dimethylformamide. Under ice-cooling, N,O-dimethylhydroxylamine hydrochloride (94 mg), HATU (183 mg) and diisopropylethylamine (0.253 ml) were added, and the mixture was stirred at room temperature overnight. To the reaction mixture was added saturated aqueous sodium hydrogen carbonate solution, and the mixture was extracted twice with ethyl acetate. The organic layer was washed successively with water and saturated aqueous sodium chloride solution and dried over sodium sulfate. Sodium sulfate was filtered off, and the filtrate was concentrated under reduced pressure. The obtained residue was purified by silica gel chromatography (hexane:ethyl acetate=88:12 to ethyl acetate) to give the title compound (80 mg).
¹H-NMR (400 MHz, CDCl₃) 1.29 (d, J=6.58Hz, 3H), 2.82 (dd, J=15.84, 1.49Hz, 1H), 3.35 (dd, J=15.84, 5.98Hz, 1H), 3.61 (s, 3H), 3.89 (s, 3H), 5.38 - 5.50 (m, 1H), 7.11 - 7.34 (m, 3H), 7.43 (s, 1H), 7.59 (d, J=7.47Hz, 1H)

### Step 6

### 1-(5-methyl-5,6-dihydroimidazo[5,1-a]isoquinolin-3-yl)ethan-1-one

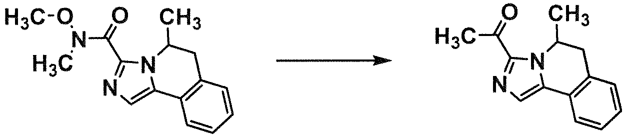

N-Methoxy-N,5-dimethyl-5,6-dihydroimidazo[5,1-a]isoquinoline-3-carboxamide (80 mg) obtained in the previous step was mixed with tetrahydrofuran (2 ml). Under ice-cooling, to the mixture was added dropwise 1M methylmagnesium bromide/tetrahydrofuran solution (0.59 ml), and the mixture was stirred for 1 hr. To the reaction mixture was added saturated aqueous ammonium chloride solution, and the mixture was allowed to warm to room temperature and extracted twice with ethyl acetate. The organic layer was washed with saturated aqueous sodium chloride solution and dried over sodium sulfate. Sodium sulfate was filtered off, and the filtrate was concentrated under reduced pressure. The obtained residue was purified by silica gel column chromatography (hexane:ethyl acetate=12:88 to ethyl acetate) to give the title compound (46.3 mg).
¹H-NMR (400 MHz, CDCl₃) 1.24 (d, J=6.70Hz, 3H), 2.68 (s, 3H), 2.82 (dd, J=15.95, 1.39Hz, 1H), 3.33 (dd, J=15.95, 6.47Hz, 1H), 5.61 - 5.73 (m, 1H), 7.23 - 7.33 (m, 3H), 7.48 (s, 1H), 7.57 - 7.64 (m, 1H)

### Step 7

### 1,1,1-trifluoro-2-(5-methyl-5,6-dihydroimidazo[5,1-a]isoquinolin-3-yl)propan-2-ol

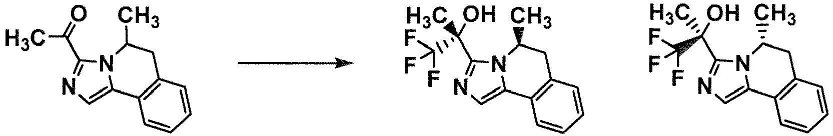

1-(5-Methyl-5,6-dihydroimidazo[5,1-a]isoquinolin-3-yl)ethan-1-one (46.3 mg) obtained in the previous step was mixed with dimethylformamide (1 ml). Under ice-cooling, cesium fluoride (7.42 mg) and (trifluoromethyl)trimethylsilane (54.1 µl) were added, and the mixture was stirred at room temperature for 30 min. Under ice-cooling, methanol (0.332 ml) and potassium carbonate (40.5 mg) were added, and the mixture was stirred at room temperature for 30 min. To the reaction mixture was added saturated aqueous ammonium chloride solution, and the mixture was extracted twice with ethyl acetate. The organic layer was successively washed twice with water and once with saturated aqueous sodium chloride solution and dried over sodium sulfate. Sodium sulfate was filtered off, and the filtrate was concentrated under reduced pressure. The obtained residue was successively purified by silica gel column chromatography (hexane:ethyl acetate=12:88 to ethyl acetate) and thin layer silica gel chromatography (hexane:ethyl acetate=2:1) to give the title compound (50.8 mg) as a racemate.
¹H-NMR (400 MHz, CDCl₃) 1.21 (d, J=6.47Hz, 3H), 1.95 (s, 3H), 2.77 (dd, J=15.49, 1.62Hz, 1H), 3.28 (dd, J=15.49, 5.32Hz, 1H), 3.52 (s, 1H), 5.10 - 5.20 (m, 1H), 7.17 - 7.30 (m, 5H), 7.33 (s, 1H), 7.54 (d, J=7.40Hz, 1H)

### Production Example 5

### Synthesis of 2-(3-cyclopropyl-5-methyl-4,5-dihydroimidazo[1,5-a]isoxazolo[5,4-c]pyridin-7-yl)-1,1,1-trifluoropropan-2-ol (compound of Example 34) and 2-(3-cyclopropyl-5-methyl-4,5-dihydroimidazo[1,5-a]isoxazolo[3,4-c]pyridin-7-yl)-1,1,1-trifluoropropan-2-ol (compound of Example 35)

### Step 1

### methyl 4-hydroxypentanoate

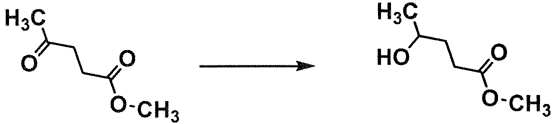

Methyl 4-oxopentanoate (14 g) was mixed with methanol (112 ml). The mixture was cooled to -78°C, sodium borohydride (4.07 g) was added, and the mixture was stirred at -50°C for 2 hr. To the reaction mixture was added saturated aqueous ammonium chloride solution, and the mixture was extracted with ethyl acetate. The organic layer was washed with saturated aqueous sodium chloride solution and dried over sodium sulfate. Sodium sulfate was filtered off, and the filtrate was concentrated under reduced pressure. The obtained residue was purified by silica gel chromatography (hexane:ethyl acetate=12:88 to ethyl acetate) to give the title compound as a crude product (11.15 g). The crude product was used for the next step without further purification.

### Step 2

### methyl 1-(5-methoxy-5-oxopentan-2-yl)-1H-imidazole-5-carboxylate

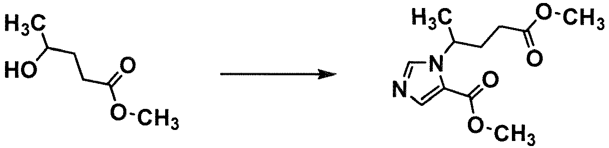

Methyl 1H-imidazole-5-carboxylate (3 g) and toluene (24 ml) were mixed. To the mixture were added methyl 4-hydroxypentanoate as a crude product (4.85 g) obtained in the previous step, and trioctylphosphine (10.58 g). The mixture was heated to 80°C, diisopropyl azodicarboxylate (5.77 g) was added dropwise, and the mixture was stirred for 3 hr. The reaction mixture was concentrated under reduced pressure, and purified by silica gel chromatography (ethyl acetate:hexane=50:50 to ethyl acetate, thereafter ethyl acetate:methanol=90:10) to give the title compound (2.6 g). ¹H-NMR (400 MHz, CDCl₃) 1.52 (d, J=6.94Hz, 3H), 2.05 - 2.35 (m, 4H), 3.63 (s, 3H), 3.83 (s, 3H), 5.17 - 5.30 (m, 1H), 7.70 - 7.74 (m, 2H)

### Step 3

### methyl 5-methyl-8-oxo-5,6,7,8-tetrahydroimidazo[1,5-a]pyridine-7-carboxylate

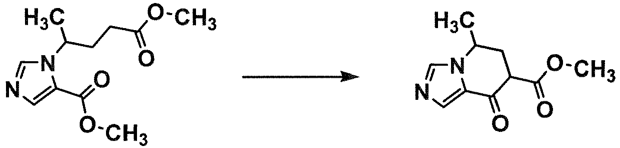

Methyl 1-(5-methoxy-5-oxopentan-2-yl)-1H-imidazole-5-carboxylate (1.48 g) obtained in the previous step was mixed with tetrahydrofuran (45 ml). To the mixture was added potassium tert-butoxide (0.76 g), and the mixture was stirred at room temperature for 3 hr. To the reaction mixture was added saturated aqueous ammonium chloride solution, and the mixture was extracted twice with ethyl acetate. The organic layer was washed with saturated aqueous sodium chloride solution and dried over sodium sulfate. Sodium sulfate was filtered off, and the filtrate was concentrated under reduced pressure to give the title compound as a crude product (1.04 g). The crude product was used for the next step without further purification.

### Step 4

### 5-methyl-6,7-dihydroimidazo[1,5-a]pyridin-8(5H)-one

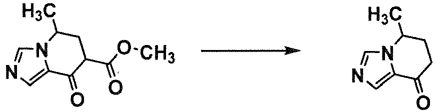

The crude product (1 g) of methyl 5-methyl-8-oxo-5,6,7,8-tetrahydroimidazo[1,5-a]pyridine-7-carboxylate obtained in the previous step was mixed with dimethyl sulfoxide (6.82 ml). To the mixture were added sodium chloride (0.421 g) and water (1.73 ml), and the mixture was stirred at 160°C for 3 hr. The reaction mixture was concentrated under reduced pressure to evaporate water, and purified twice by cation exchange column chromatography (methanol to 1N ammonia/methanol solution) to give the title compound (0.714 g).
¹H-NMR (400 MHz, DMSO-D₆) 1.54 (d, J=6.70Hz, 3H), 1.86 - 2.02 (m, 1H), 2.16 - 2.28 (m, 1H), 2.52 - 2.59 (m, 2H), 4.35 - 4.50 (m, 1H), 7.63 (d, J=0.69Hz, 1H), 8.05 (s, 1H)

### Step 5

### 7-(cyclopropanecarbonyl)-5-methyl-6,7-dihydroimidazo[1,5-a]pyridin-8(5H)-one

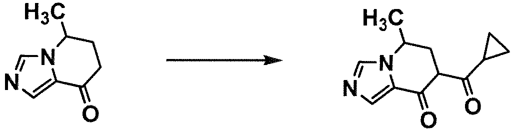

5-Methyl-6,7-dihydroimidazo[1,5-a]pyridin-8(5H)-one (100 mg) obtained in the previous step was mixed with tetrahydrofuran (1.5 ml). Under ice-cooling, to the mixture was added dropwise 1.1M lithium bis(trimethylsilyl)amide/n-hexane solution (0.908 ml), and the mixture was stirred for 40 min. To the reaction mixture was added cyclopropanecarbonyl chloride (77 mg), and the mixture was stirred at 0°C for 5 min and at room temperature for 25 min. To the reaction mixture was added saturated aqueous ammonium chloride solution, and the mixture was extracted twice with ethyl acetate. The organic layer was washed with saturated aqueous sodium chloride solution, and dried over magnesium sulfate. Magnesium sulfate was filtered off, and the filtrate was concentrated under reduced pressure. The obtained residue was purified by silica gel chromatography (ethyl acetate to ethyl acetate:methanol=90:10) to give the title compound as a crude product (91.1 mg). The crude product was used for the next step without further purification.

### Step 6

### Mixture of 3-cyclopropyl-5-methyl-4,5-dihydroimidazo[1,5-a]isoxazolo[5,4-c]pyridine and 3-cyclopropyl-5-methyl-4,5-dihydroimidazo[1,5-a]isoxazolo[3,4-c]pyridine

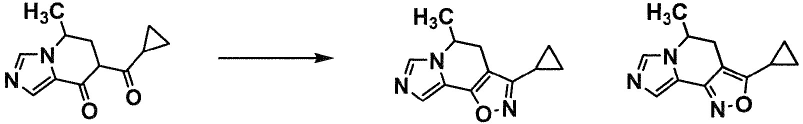

The crude product (90 mg) of 7-(cyclopropanecarbonyl)-5-methyl-6,7-dihydroimidazo[1,5-a]pyridin-8(5H)-one obtained in the previous step was mixed with acetic acid (0.9 ml). To the mixture was added hydroxylamine hydrochloride (86 mg), and the mixture was stirred at 90°C for 40 min. After allowing to cool to room temperature, to the reaction mixture was added sulfuric acid (81 mg), and the mixture was stirred at 100°C for 2 hr and at 110°C for 4 hr. After allowing to cool to room temperature, to the reaction mixture was added saturated aqueous ammonium chloride solution, and the mixture was extracted twice with ethyl acetate. The organic layer was washed with saturated aqueous sodium chloride solution, and dried over magnesium sulfate. Magnesium sulfate was filtered off, and the filtrate was concentrated under reduced pressure. The obtained residue was successively purified by silica gel chromatography (ethyl acetate to ethyl acetate:methanol=90:10) and preparative thin layer chromatography (ethyl acetate:methanol=90:10) to give the title compound as a crude product (45.2 mg). The crude product was used for the next step without further purification.

### Step 7

### Mixture of 1-(3-cyclopropyl-5-methyl-4,5-dihydroimidazo[1,5-a]isoxazolo[5,4-c]pyridin-7-yl)ethan-1-one and 1-(3-cyclopropyl-5-methyl-4,5-dihydroimidazo[1,5-a]isoxazolo[3,4-c]pyridin-7-yl)ethan-1-one

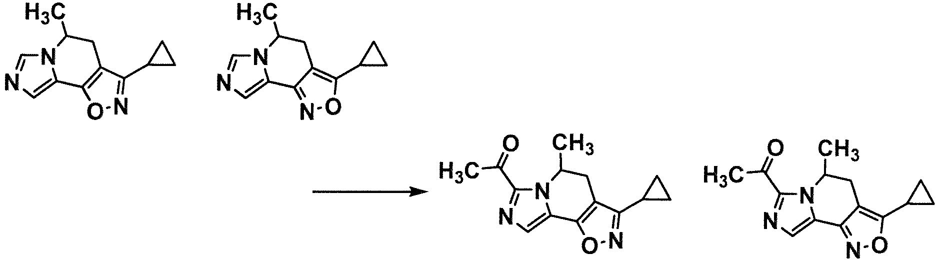

The crude product (43.2 mg) of 3-cyclopropyl-5-methyl-4,5-dihydroimidazo[1,5-a]isoxazolo[5,4-c]pyridine and 3-cyclopropyl-5-methyl-4,5-dihydroimidazo[1,5-a]isoxazolo[3,4-c]pyridine obtained in the previous step was mixed with tetrahydrofuran (0.864 ml). The mixture was cooled to -78°C, and 2M lithium diisopropylamide solution (0.251 ml) was added dropwise. The mixture was stirred at -78°C for 40 min. To the mixture was added N-methoxy-N-methylacetamide (62.1 mg), and the mixture was stirred at -78°C for 1 hr. To the reaction mixture was added saturated aqueous ammonium chloride solution, and the mixture was allowed to warm to room temperature and extracted twice with ethyl acetate. The organic layer was washed with saturated aqueous sodium chloride solution, and dried over magnesium sulfate. Magnesium sulfate was filtered off, and the filtrate was concentrated under reduced pressure. The obtained residue was purified by silica gel column chromatography (hexane:ethyl acetate=75:25 to 50:50) to give the title compound as a crude product (18.8 mg). The crude product was used for the next step without further purification.

### Step 8

### 2-(3-cyclopropyl-5-methyl-4,5-dihydroimidazo[1,5-a]isoxazolo[5,4-c]pyridin-7-yl)-1,1,1-trifluoropropan-2-ol (compound of Example 34) and 2-(3-cyclopropyl-5-methyl-4,5-dihydroimidazo[1,5-a]isoxazolo[3,4-c]pyridin-7-yl)-1,1,1-trifluoropropan-2-ol (compound of Example 35)

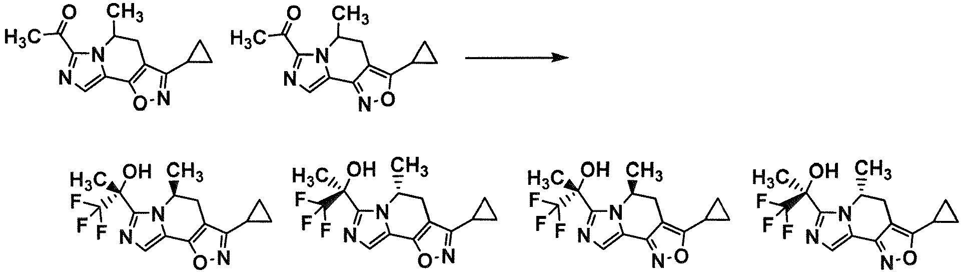

The crude product (17 mg) of 1-(3-cyclopropyl-5-methyl-4,5-dihydroimidazo[1,5-a]isoxazolo[5,4-c]pyridin-7-yl)ethan-1-one and 1-(3-cyclopropyl-5-methyl-4,5-dihydroimidazo[1,5-a]isoxazolo[3,4-c]pyridin-7-yl)ethan-1-one obtained in the previous step was mixed with tetrahydrofuran (0.34 ml). Under ice-cooling, cesium fluoride (7.42 mg) and (trifluoromethyl)trimethylsilane (54.1 µl) were added, and the mixture was stirred at room temperature for 1 hr. Under ice-cooling, methanol (0.332 ml) and potassium carbonate (40.5 mg) were added, and the mixture was stirred for 1.5 hr while allowing to warm to room temperature. To the reaction mixture was added saturated aqueous ammonium chloride solution, the mixture was partitioned, and the organic layer was purified by silica gel column chromatography (hexane:ethyl acetate=12:88 to ethyl acetate) to give the compound of Example 34 (5.2 mg) and the compound of Example 35 (8.4 mg) each as a racemate. The structure of the isoxazole moiety was determined by NMR. 2-(3-cyclopropyl-5-methyl-4,5-dihydroimidazo[1,5-a]isoxazolo[5,4-c]pyridin-7-yl)-1,1,1-trifluoropropan-2-ol ¹H-NMR (400 MHz, CDCl₃) 1.02 - 1.04 (m, 4H), 1.30 (d, J=3.29Hz, 3H), 2.00 (s, 3H), 2.63 - 2.67 (m, 1H), 3.05 - 3.11 (m, 1H), 3.33 - 3.37 (m, 1H), 5.30 - 5.36 (m, 1H), 7.41 (s, 1H) 2-(3-cyclopropyl-5-methyl-4,5-dihydroimidazo[1,5-a]isoxazolo[3,4-c]pyridin-7-yl)-1,1,1-trifluoropropan-2-ol ¹H-NMR (400 MHz, CDCl₃) 1.06 - 1.16 (m, 4H), 1.31 (d, J=6.58Hz, 3H), 1.85 (s, 3H), 2.79 (dd, J=15.55, 1.79Hz, 1H), 2.96 - 3.02 (m, 1H), 3.87 - 4.04 (m, 1H), 5.42 - 5.44 (m, 1H), 7.60 (s, 1H)

### Production Example 6

### Synthesis of 1,1,1-trifluoro-2-(6-methyl-2-(trifluoromethyl)-5,6-dihydroimidazo[1',5':1,2]pyrido[3,4-d]pyrimidin-8-yl)propan-2-ol (compound of Example 6)

### Step 1

### 7-((dimethylamino)methylene)-5-methyl-6,7-dihydroimidazo[1,5-a]pyridin-8(5H)-one

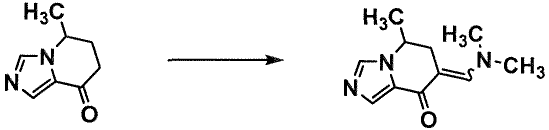

5-Methyl-6,7-dihydroimidazo[1,5-a]pyridin-8(5H)-one (250 mg) was mixed with 1,4-dioxane (1.25 ml). To the mixture was added tert-butoxy bis(dimethylamino)methane (435 mg), and the mixture was stirred at 110°C for 1 hr. The reaction mixture was concentrated under reduced pressure to give the title compound as a crude product. The crude product was used for the next step without further purification.

### Step 2

### 6-methyl-2-(trifluoromethyl)-5,6-dihydroimidazo[1',5':1,2]pyrido[3,4-d]pyrimidine

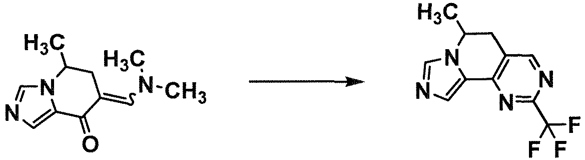

The crude product of 7-((dimethylamino)methylene)-5-methyl-6,7-dihydroimidazo[1,5-a]pyridin-8(5H)-one obtained in the previous step was mixed with ethanol (0.95 ml). To the mixture was added trifluoroacetamidine (142 mg), and the mixture was stirred at room temperature for 15 min. To the mixture was added sodium ethoxide (86 mg), and the mixture was stirred overnight with heating under reflux. The reaction mixture was allowed to cool to room temperature, saturated aqueous ammonium chloride solution was added, and the mixture was extracted twice with ethyl acetate. The organic layer was washed with saturated aqueous sodium chloride solution and dried over sodium sulfate. Sodium sulfate was filtered off, and the filtrate was concentrated under reduced pressure. The obtained residue was purified by silica gel column chromatography (hexane:ethyl acetate=95:5 to 60:40) to give the title compound (172.6 mg).
¹H-NMR (400 MHz, CDCl₃) 1.62 (d, J=6.47Hz, 3H), 2.94 (dd, J=16.18, 8.32Hz, 1H), 3.25 (dd, J=16.18, 5.09Hz, 1H), 4.44 - 4.58 (m, 1H), 7.76 (s, 1H), 8.00 (s, 1H),
8.64 (s, 1H)

### Step 3

### 1-(6-methyl-2-(trifluoromethyl)-5,6-dihydroimidazo[1',5':1,2]pyrido[3,4-d]pyrimidin-8-yl)ethan-1-one

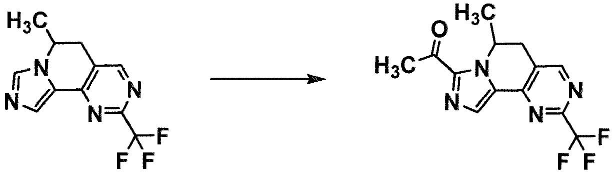

6-Methyl-2-(trifluoromethyl)-5,6-dihydroimidazo[1',5':1,2]pyrido[3,4-d]pyrimidine (50 mg) obtained in the previous step was mixed with tetrahydrofuran (0.5 ml). The mixture was cooled to -78°C, and 2M lithium diisopropylamide solution (0.118 ml) was added dropwise. The mixture was stirred at -78°C for 1 hr. To the mixture was added N-methoxy-N-methylacetamide (40.6 mg), and the mixture was stirred at -78°C for 1 hr. To the reaction mixture was added saturated aqueous ammonium chloride solution, and the mixture was allowed to warm to room temperature and extracted twice with ethyl acetate. The organic layer was washed with saturated aqueous sodium chloride solution and dried over sodium sulfate. Sodium sulfate was filtered off, and the filtrate was concentrated under reduced pressure. The obtained residue was purified by silica gel column chromatography (hexane:ethyl acetate=12:88 to ethyl acetate) to give the title compound (17.2 mg).
¹H-NMR (400 MHz, CDCl₃) 1.30 (d, J=6.70Hz, 3H), 3.00 (dd, J=16.30, 1.16Hz, 1H), 3.38 (dd, J=16.30, 6.59Hz, 1H), 5.77 - 5.86 (m, 1H), 8.04 (s, 1H), 8.74 (s, 1H)

### Step 4

### 1,1,1-trifluoro-2-(6-methyl-2-(trifluoromethyl)-5,6-dihydroimidazo[1',5':1,2]pyrido[3,4-d]pyrimidin-8-yl)propan-2-ol

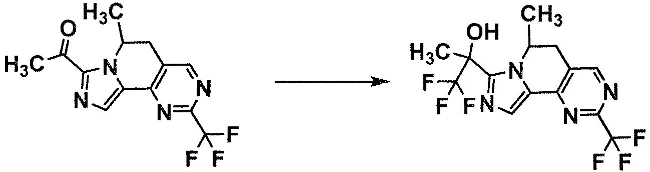

1-(6-Methyl-2-(trifluoromethyl)-5,6-dihydroimidazo[1',5':1,2]pyrido[3,4-d]pyrimidin-8-yl)ethan-1-one (17.2 mg) obtained in the previous step was mixed with tetrahydrofuran (0.172 ml). Under ice-cooling, to the mixture were added cesium fluoride (1.764 mg) and (trifluoromethyl)trimethylsilane (16.51 mg), and the mixture was stirred at room temperature for 1 hr. Under ice-cooling, to the mixture was added (trifluoromethyl)trimethylsilane (16.51 mg), and the mixture was stirred at room temperature for 20 min. Under ice-cooling, to the mixture was added (trifluoromethyl)trimethylsilane (16.51 mg), and the mixture was stirred at room temperature for 20 min. Under ice-cooling, to the reaction mixture were added methanol (0.103 ml) and potassium carbonate (9.63 mg), and the mixture was stirred at room temperature for 30 min. To the mixture was added saturated aqueous ammonium chloride solution, and the mixture was extracted twice with ethyl acetate. The organic layer was washed successively with water and saturated aqueous sodium chloride solution and dried over sodium sulfate. Sodium sulfate was filtered off, and the filtrate was concentrated under reduced pressure. The obtained residue was purified by preparative thin layer silica gel chromatography (hexane:ethyl acetate=1:1). To the obtained crude product was added ethyl acetate/hexane=1/1, and the mixture was stirred at room temperature. The precipitated solid was collected by filtration to give the title compound (7.1 mg) as a racemate. ¹H-NMR (400 MHz, DMSO-D₆) 1.20 (d, J=6.47Hz, 3H), 1.84 (s, 3H), 3.14 (d, J=16.41Hz, 1H), 3.23 (dd, J=16.41, 5.32Hz, 1H), 5.34 - 5.44 (m, 1H), 7.40 (s, 1H), 7.81 (s, 1H), 8.92 (s, 1H) [0357]

### Production Example 7

### Synthesis of 2-(1,5-dimethyl-3-(trifluoromethyl)-4,5-dihydro-1H-imidazo[1,5-a]pyrazolo[3,4-c]pyridin-7-yl)-1,1,1-trifluoropropan-2-ol (compound of Example 24)

### Step 1

### 1,5-dimethyl-3-(trifluoromethyl)-4,5-dihydro-1H-imidazo[1,5-a]pyrazolo[3,4-c]pyridine

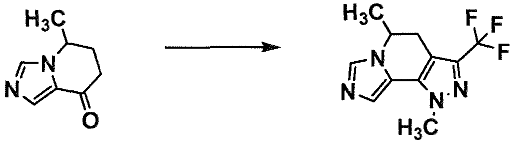

5-Methyl-6,7-dihydroimidazo[1,5-a]pyridin-8(5H)-one (150 mg) was mixed with tetrahydrofuran (1.5 ml). To the mixture was added ethyl trifluoroacetate (312 mg). Under ice-cooling, to the mixture was added sodium hydride (88 mg), and the mixture was stirred at room temperature for 10 min and at 60°C for 1 hr. After allowing to cool to room temperature, to the reaction mixture were added methylhydrazine (138 mg) and ethanol (1.5 ml). The reaction mixture was stirred at 95°C for 3 hr. The reaction mixture was concentrated under reduced pressure, and azeotroped with acetic acid. To the obtained residue were added acetic acid (1.5 ml) and methylhydrazine (138 mg), and the mixture was stirred at room temperature for 1 hr. The reaction mixture was concentrated, saturated aqueous sodium hydrogen carbonate solution was added, and the mixture was extracted twice with ethyl acetate. The organic layer was washed with saturated aqueous sodium hydrogen carbonate solution and dried over sodium sulfate. Sodium sulfate was filtered off, and the filtrate was concentrated under reduced pressure. The obtained residue was purified by silica gel column chromatography (ethyl acetate:methanol=95:5 to 60:40) to give the title compound (170.4 mg). The substitution position of the methyl group in the pyrazole moiety was determined by two-dimensional NMR.
¹H-NMR (400 MHz, DMSO-D₆) 1.44 (d, J=6.47Hz, 3H), 2.72 (dd, J=15.95, 7.86Hz, 1H), 3.09 (dd, J=15.95, 5.55Hz, 1H), 4.01 (s, 3H), 4.41 - 4.52 (m, 1H), 7.46 (s, 1H), 7.96 (s, 1H)

### Step 2

### 1-(1,5-dimethyl-3-(trifluoromethyl)-4,5-dihydro-1H-imidazo[1,5-a]pyrazolo[3,4-c]pyridin-7-yl)ethan-1-one

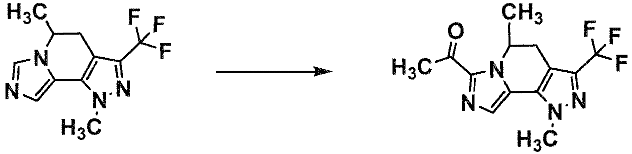

1,5-Dimethyl-3-(trifluoromethyl)-4,5-dihydro-1H-imidazo[1,5-a]pyrazolo[3,4-c]pyridine (170.4 mg) obtained in the previous step was mixed with tetrahydrofuran (5.112 ml). The mixture was cooled to -78°C, and 2M lithium diisopropylamide solution (0.831 ml) was added dropwise. The mixture was stirred at -78°C for 30 min. To the mixture was added N-methoxy-N-methylacetamide (206 mg), and the mixture was stirred at -78°C for 1 hr. To the reaction mixture was added saturated aqueous ammonium chloride solution, and the mixture was allowed to warm to room temperature and extracted twice with ethyl acetate. The organic layer was washed with saturated aqueous sodium chloride solution and dried over sodium sulfate. Sodium sulfate was filtered off, and the filtrate was concentrated under reduced pressure. The obtained residue was purified by silica gel column chromatography (hexane:ethyl acetate=12:88 to ethyl acetate) to give the title compound (150 mg).

### Step 3

### 2-(1,5-dimethyl-3-(trifluoromethyl)-4,5-dihydro-1H-imidazo[1,5-a]pyrazolo[3,4-c]pyridin-7-yl)-1,1,1-trifluoropropan-2-ol

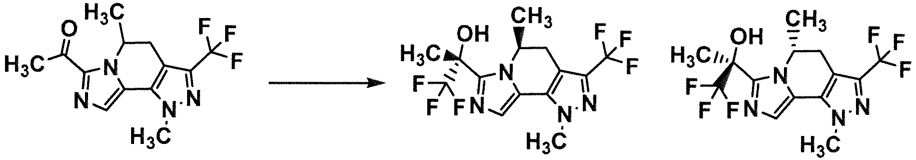

1-(1,5-Dimethyl-3-(trifluoromethyl)-4,5-dihydro-1H-imidazo[1,5-a]pyrazolo[3,4-c]pyridin-7-yl)ethan-1-one (150 mg) obtained in the previous step was mixed with tetrahydrofuran (1.5 ml). Under ice-cooling, to the mixture were added cesium fluoride (1.764 mg) and (trifluoromethyl)trimethylsilane (143 mg), and the mixture was stirred at room temperature for 45 min. Under ice-cooling, to the mixture was added (trifluoromethyl)trimethylsilane (143 mg), and the mixture was stirred at room temperature for 15 min. Under ice-cooling, to the reaction mixture were added methanol (0.103 ml) and potassium carbonate (9.63 mg), and the mixture was stirred at room temperature for 1 hr. To the mixture was added saturated aqueous ammonium chloride solution, and the mixture was extracted twice with ethyl acetate. The organic layer was washed with saturated aqueous sodium chloride solution and dried over sodium sulfate. Sodium sulfate was filtered off, and the filtrate was concentrated under reduced pressure. The obtained residue was purified by silica gel column chromatography (hexane:ethyl acetate=12:88 to ethyl acetate) to give the title compound (127.7 mg) as a racemate.
¹H-NMR (400 MHz, DMSO-D₆) 1.16 (d, J=6.58Hz, 3H), 1.84 (s, 3H), 2.90 (d, J=16.14Hz, 1H), 2.97 (dd, J=16.14, 5.38Hz, 1H), 4.04 (s, 3H), 5.32 - 5.43 (m, 1H), 7.27 (s, 1H), 7.46 (s, 1H)

### Production Example 8

### Synthesis of 1,1,1-trifluoro-2-(5-methyl-3-(trifluoromethyl)-4,5-dihydro-1H-imidazo[1,5-a]pyrazolo[3,4-c]pyridin-7-yl)propan-2-ol (compound of Example 50)

### Step 1

### 5-methyl-3-(trifluoromethyl)-4,5-dihydro-1H-imidazo[1,5-a]pyrazolo[3,4-c]pyridine

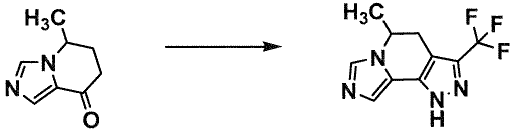

5-Methyl-6,7-dihydroimidazo[1,5-a]pyridin-8(5H)-one (200 mg) was mixed with tetrahydrofuran (8 ml). To the mixture was added ethyl trifluoroacetate (227 mg). To the mixture was added sodium hydride (80 mg), and the mixture was stirred at room temperature for 1 hr and at 70°C for 1.5 hr. After allowing to cool to room temperature, to the reaction mixture was added ethyl trifluoroacetate (37.8 mg), and the mixture was stirred at 70°C for 30 min. After allowing to cool to room temperature, to the reaction mixture was added acetic acid (80 mg), and the mixture was concentrated under reduced pressure. To the obtained residue were added acetic acid (2 ml), hydrazine monohydrate (233 mg) and sulfuric acid (261 mg), and the mixture was stirred at 100°C for 2 hr. The reaction mixture was purified by silica gel column chromatography (ethyl acetate to ethyl acetate:methanol=80:20) to give the title compound as a crude product (316.8 mg). The crude product was used for the next step without further purification.

### Step 2

### Mixture of 1-(4-methoxybenzyl)-5-methyl-3-(trifluoromethyl)-4,5-dihydro-1H-imidazo[1,5-a]pyrazolo[3,4-c]pyridine and 2-(4-methoxybenzyl)-5-methyl-3-(trifluoromethyl)-4,5-dihydro-1H-imidazo[1,5-a]pyrazolo[3,4-c]pyridine

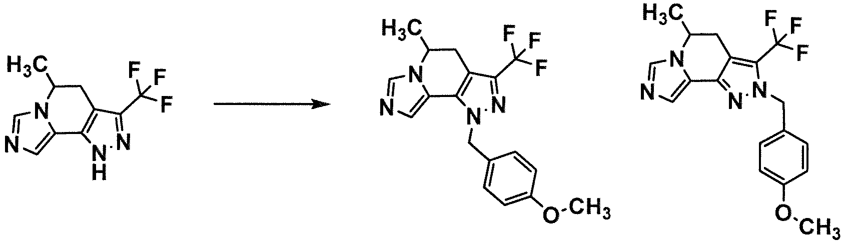

The crude product (82 mg) of 5-methyl-3-(trifluoromethyl)-4,5-dihydro-1H-imidazo[1,5-a]pyrazolo[3,4-c]pyridine obtained in the previous step was mixed with dimethylformamide (0.82 ml). Under ice-cooling, to the mixture was added sodium hydride (16.25 mg), and the mixture was stirred for 30 min. Under ice-cooling, to the reaction mixture was added 4-methoxybenzyl chloride (58.3 mg), and the mixture was stirred at room temperature overnight. To the reaction mixture was added saturated aqueous ammonium chloride solution, and the mixture was extracted with ethyl acetate. The organic layer was washed with saturated aqueous sodium chloride solution, and dried over magnesium sulfate. Magnesium sulfate was filtered off, and the filtrate was concentrated under reduced pressure. The obtained residue was purified by silica gel column chromatography (hexane:ethyl acetate=12:88 to ethyl acetate) to give the title compound as a crude product (67.1 mg). The crude product was used for the next step without further purification.

### Step 3

### Mixture of 1-(1-(4-methoxybenzyl)-5-methyl-3-(trifluoromethyl)-4,5-dihydro-1H-imidazo[1,5-a]pyrazolo[3,4-c]pyridin-7-yl)ethan-1-one and 1-(2-(4-methoxybenzyl)-5-methyl-3-(trifluoromethyl)-4,5-dihydro-2H-imidazo[1,5-a]pyrazolo[3,4-c]pyridin-7-yl)ethan-1-one

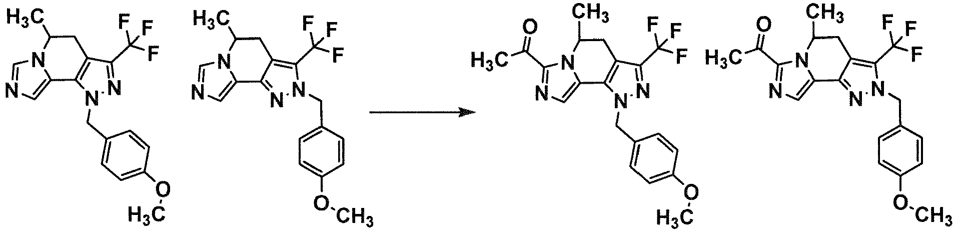

The crude product (61.1 mg) of 1-(4-methoxybenzyl)-5-methyl-3-(trifluoromethyl)-4,5-dihydro-1H-imidazo[1,5-a]pyrazolo[3,4-c]pyridine and 2-(4-methoxybenzyl)-5-methyl-3-(trifluoromethyl)-4,5-dihydro-1H-imidazo[1,5-a]pyrazolo[3,4-c]pyridine obtained in the previous step was mixed with tetrahydrofuran (0.611 ml). The mixture was cooled to -78°C, and 2M lithium diisopropylamide solution (0.211 ml) was added dropwise. The mixture was stirred at -78°C for 45 min. To the mixture was added N-methoxy-N-methylacetamide (52.2 mg), and the mixture was stirred at -78°C for 40 min. To the reaction mixture was added saturated aqueous ammonium chloride solution, and the mixture was allowed to warm to room temperature and partitioned. The organic layer was purified by silica gel column chromatography (hexane:ethyl acetate=20:80 to ethyl acetate) to give the title compound as a crude product (48 mg). The crude product was used for the next step without further purification.

### Step 4

### Mixture of 1,1,1-trifluoro-2-(1-(4-methoxybenzyl)-5-methyl-3-(trifluoromethyl)-4,5-dihydro-1H-imidazo[1,5-a]pyrazolo[3,4-c]pyridin-7-yl)propan-2-ol and 1,1,1-trifluoro-2-(2-(4-methoxybenzyl)-5-methyl-3-(trifluoromethyl)-4,5-dihydro-2H-imidazo[1,5-a]pyrazolo[3,4-c]pyridin-7-yl)propan-2-ol

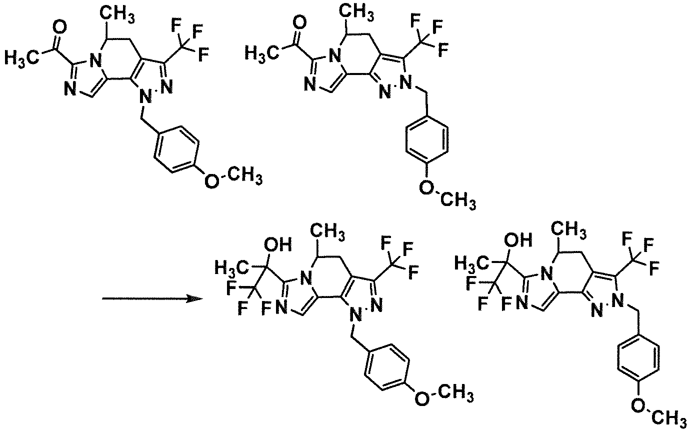

The crude product (45 mg) of 1-(1-(4-methoxybenzyl)-5-methyl-3-(trifluoromethyl)-4,5-dihydro-1H-imidazo[1,5-a]pyrazolo[3,4-c]pyridin-7-yl)ethan-1-one and 1-(2-(4-methoxybenzyl)-5-methyl-3-(trifluoromethyl)-4,5-dihydro-2H-imidazo[1,5-a]pyrazolo[3,4-c]pyridin-7-yl)ethan-1-one obtained in the previous step was mixed with tetrahydrofuran (0.9 ml). Under ice-cooling, to the mixture were added cesium fluoride (5.07 mg) and (trifluoromethyl)trimethylsilane (47.5 mg), and the mixture was stirred at room temperature for 2 hr. Under ice-cooling, to the reaction mixture were added methanol (0.45 ml) and potassium carbonate (77 mg), and the mixture was stirred for 2 hr. To the mixture was added saturated aqueous ammonium chloride solution, and the mixture was extracted with ethyl acetate. The organic layer was dried over magnesium sulfate. Magnesium sulfate was filtered off, and the filtrate was concentrated under reduced pressure. The obtained residue was purified by preparative thin layer silica gel chromatography (hexane:ethyl acetate=50:50) to give the title compound as a crude product (11.8 mg). The crude product was used for the next step without further purification.

### Step 5

### 1,1,1-trifluoro-2-(5-methyl-3-(trifluoromethyl)-4,5-dihydro-1H-imidazo[1,5-a]pyrazolo[3,4-c]pyridin-7-yl)propan-2-ol

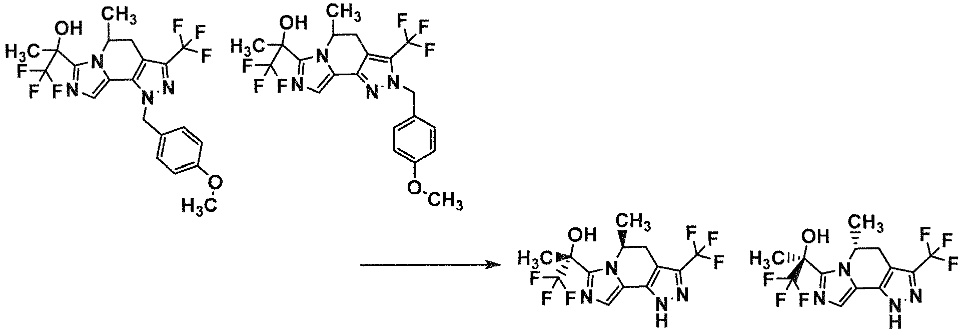

The crude product (11.5 mg) of 1,1,1-trifluoro-2-(1-(4-methoxybenzyl)-5-methyl-3-(trifluoromethyl)-4,5-dihydro-1H-imidazo[1,5-a]pyrazolo[3,4-c]pyridin-7-yl)propan-2-ol and 1,1,1-trifluoro-2-(2-(4-methoxybenzyl)-5-methyl-3-(trifluoromethyl)-4,5-dihydro-2H-imidazo[1,5-a]pyrazolo[3,4-c]pyridin-7-yl)propan-2-ol obtained in the previous step was mixed with dichloromethane (0.46 ml). To the mixture was added trifluoroacetic acid (0.0467 ml), and the mixture was stirred at room temperature for 2 days and at 60°C for 2 hr. To the mixture was added trifluoroacetic acid (0.3 ml), and the mixture was stirred at 70°C for 2 hr and at 80°C overnight. The reaction mixture was concentrated under reduced pressure, and ethyl acetate and saturated aqueous sodium hydrogen carbonate solution were added. The mixture was extracted twice with ethyl acetate, and the organic layer was dried over magnesium sulfate. Magnesium sulfate was filtered off, and the filtrate was concentrated under reduced pressure. The obtained residue was purified by preparative thin layer silica gel chromatography (hexane:ethyl acetate=1:2) to give the title compound (6.5 mg) as a racemate.
¹H-NMR (400 MHz, DMSO-D₆) 1.17 (d, J=6.58Hz, 3H), 1.84 (s, 3H), 2.87 - 3.02 (m, 2H), 3.41 - 3.48 (m, 2H), 5.30 - 5.45 (m, 1H), 7.23 (s, 1H), 14.06 (s, 1H)

### Production Example 9

### Synthesis of (R)-1,1,1-trifluoro-2-((S)-5-methyl-9-(trifluoromethyl)-5,6-dihydropyrazolo[1,5-a][1,2,4]triazolo[3,4-c]pyrazin-3-yl)propan-2-ol (compound of Example 13)

### Step 1

### tert-butyl (R)-2-(3,3,3-trifluoro-2-hydroxy-2-methylpropanoyl)hydrazine-1-carboxylate

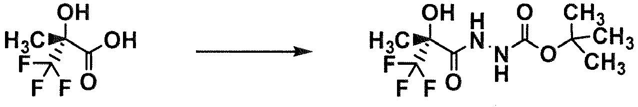

tert-Butoxycarbonylhydrazine (1.17 g) was mixed with acetonitrile (15 ml). To the mixture was added (R)-3,3,3-trifluoro-2-hydroxy-2-methylpropionic acid (1 g). Under ice-cooling, to the mixture were added 1-hydroxybenzotriazole monohydrate (0.581 g) and 1-(3-dimethylaminopropyl)-3-ethylcarbodiimide hydrochloride (1.455 g), and the mixture was stirred at room temperature overnight. The reaction mixture was concentrated under reduced pressure, ethyl acetate was added to the obtained residue, and the mixture was washed successively with 0.5N hydrochloric acid and saturated aqueous sodium hydrogen carbonate solution. The organic layer was dried over sodium sulfate. Sodium sulfate was filtered off, and the filtrate was concentrated under reduced pressure to give the title compound (1.66 g).
¹H-NMR (400 MHz, DMSO-D₆) 1.27 - 1.42 (m, 9H), 1.46 (s, 3H), 6.99 (s, 1H), 8.31 (s, 0.25H), 8.76 (s, 0.75H), 9.64 - 9.96 (m, 1H)

### Step 2

### (R)-3,3,3-trifluoro-2-hydroxy-2-methylpropanehydrazide

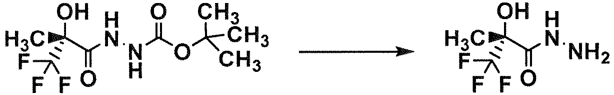

tert-Butyl (R)-2-(3,3,3-trifluoro-2-hydroxy-2-methylpropanoyl)hydrazine-1-carboxylate (0.6 g) obtained in the previous step was mixed with ethyl acetate (1.114 ml). Under ice-cooling, to the mixture was added trifluoroacetic acid (2.78 ml), and the mixture was stirred at room temperature for 5 hr. The reaction mixture was concentrated under reduced pressure, and purified by cation exchange column chromatography (methanol to 1N ammonia/methanol solution) to give the title compound (155.8 mg).
¹H-NMR (400 MHz, DMSO-D₆) 1.44 (s, 3H), 4.32 (s, 2H), 6.82 (s, 1H), 9.28 (brs, 1H)

### Step 3

### methyl (S)-1-(2-((tert-butoxycarbonyl)amino)propyl)-3-(trifluoromethyl)-1H-pyrazole-5-carboxylate

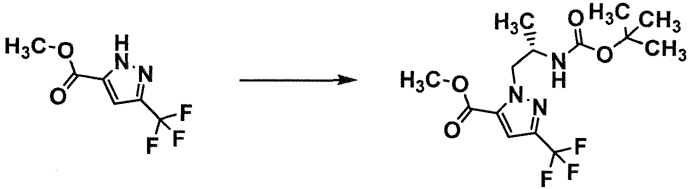

Methyl 3-(trifluoromethyl)-1H-pyrazole-5-carboxylate (200 mg), N-(tert-butoxycarbonyl)-L-alaninol (271 mg) and triphenylphosphine (405 mg) were mixed with tetrahydrofuran (2 ml). The mixture was heated to 80°C, and diisopropyl azodicarboxylate (0.3 ml) was added dropwise. The reaction mixture was stirred at 80°C for 2 hr and at room temperature overnight. The reaction mixture was concentrated under reduced pressure, and the obtained residue was purified by silica gel column chromatography (hexane:ethyl acetate=92:8 to 44:66) to give the title compound (388.9 mg).
¹H-NMR (400 MHz, CDCl₃) 1.18 (d, J=6.70Hz, 3H), 1.31 (s, 9H), 3.90 (s, 3H), 4.14 - 4.29 (m, 1H), 4.37 - 4.52 (m, 1H), 4.55 - 4.79 (m, 2H), 7.06 (s, 1H)

### Step 4

### (S)-6-methyl-2-(trifluoromethyl)-6,7-dihydropyrazolo[1,5-a]pyrazin-4(5H)-one

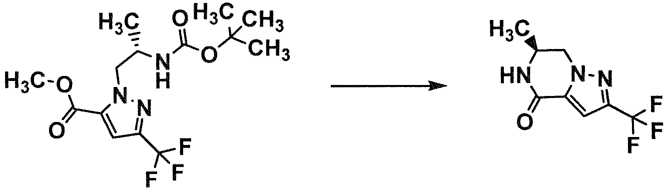

Methyl (S)-1-(2-((tert-butoxycarbonyl)amino)propyl)-3-(trifluoromethyl)-1H-pyrazole-5-carboxylate (362 mg) obtained in the previous step was mixed with chloroform (2 ml). Under ice-cooling, to the mixture was added trifluoroacetic acid (2 ml), and the mixture was stirred at room temperature for 3 hr. The reaction mixture was concentrated under reduced pressure, and azeotroped with toluene. To the obtained residue were added methanol (3.62 ml) and sodium carbonate (437 mg), and the mixture was stirred at room temperature for 3 hr. To the reaction mixture was added water, and the mixture was stirred at room temperature for 30 min. The precipitated solid was collected by filtration to give the title compound (193.4 mg). ¹H-NMR (400 MHz, DMSO-D₆) 1.22 (d, J=6.47Hz, 3H), 3.97 - 4.16 (m, 2H), 4.49 (dd, J=12.60, 4.05Hz, 1H), 7.17 (s, 1H), 8.50 (s, 1H)

### Step 5

### (S)-6-methyl-2-(trifluoromethyl)-6,7-dihydropyrazolo[1,5-a]pyrazine-4(5H)-thione

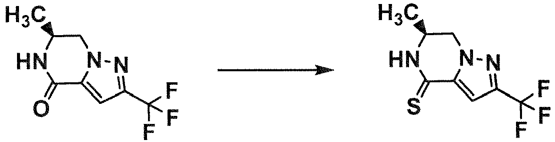

(S)-6-Methyl-2-(trifluoromethyl)-6,7-dihydropyrazolo[1,5-a]pyrazin-4(5H)-one (193.4 mg) obtained in the previous step was mixed with tetrahydrofuran (3.868 ml). To the mixture was added 2,4-bis(4-methoxyphenyl)-1,3,2,4-dithiadiphosphetane-2,4-disulfide (250 mg), and the mixture was stirred at 70°C for 7 hr. The reaction mixture was concentrated under reduced pressure, and the obtained residue was purified by silica gel column chromatography (hexane:ethyl acetate=92:8 to 44:66) to give the title compound (199.8 mg).
¹H-NMR (400 MHz, CDCl₃) 1.47 (d, J=6.47Hz, 3H), 4.03 - 4.23 (m, 2H), 4.48 (dd, J=12.25, 3.47Hz, 1H), 7.29 (s, 1H), 7.62 (brs, 1H)

### Step 6

### (S)-6-methyl-4-(methylthio)-2-(trifluoromethyl)-6,7-dihydropyrazolo[1,5-a]pyrazine hydroiodide

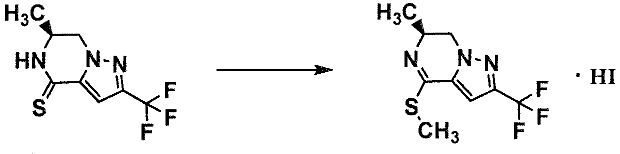

(S)-6-Methyl-2-(trifluoromethyl)-6,7-dihydropyrazolo[1,5-a]pyrazine-4(5H)-thione (80 mg) obtained in the previous step was mixed with acetone (0.8 ml). Under ice-cooling, to the mixture was added methyl iodide (57.9 mg), and the mixture was stirred at room temperature overnight. Under ice-cooling, to the mixture was added methyl iodide (57.9 mg), and the mixture was stirred at room temperature for 7 hr. The reaction mixture was concentrated under reduced pressure to give the title compound as a crude product (128 mg). The crude product was used for the next step without further purification.

### Step 7

### (R)-1,1,1-trifluoro-2-((S)-5-methyl-9-(trifluoromethyl)-5,6-dihydropyrazolo[1,5-a] [1,2,4]triazolo[3,4-c]pyrazin-3-yl)propan-2-ol

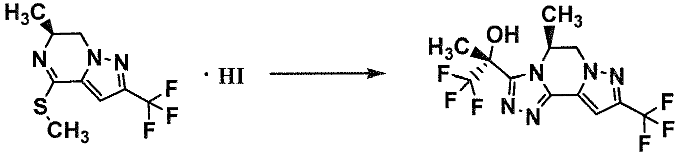

The crude product (128 mg) of (S)-6-methyl-4-(methylthio)-2-(trifluoromethyl)-6,7-dihydropyrazolo[1,5-a]pyrazine hydroiodide obtained in the previous step and (R)-3,3,3-trifluoro-2-hydroxy-2-methylpropanehydrazide (61 mg) obtained in Step 2 were mixed with isopropanol (1 ml). To the mixture was added acetic acid (0.039 ml), and the mixture was stirred at 100°C for 1.5 hr. The mixture was allowed to cool to room temperature, to the reaction mixture were added sodium acetate (56 mg) and water (0.256 ml), and the mixture was stirred at 100°C overnight. The reaction mixture was successively purified by cation exchange column chromatography (methanol) and silica gel column chromatography (hexane:ethyl acetate=88:12 to ethyl acetate) to give the title compound (32.2 mg).
¹H-NMR (400 MHz, DMSO-D₆) 1.30 (d, J=6.58Hz, 3H), 1.91 (s, 3H), 4.62 (d, J=13.75Hz, 1H), 4.70 (dd, J=13.75, 4.04Hz, 1H), 5.31 - 5.42 (m, 1H), 7.47 (s, 1H), 7.71 (s, 1H)

### Production Example 10

### Synthesis of (R)-2-((S)-9-(difluoromethoxy)-5-methyl-5,6-dihydroimidazo[1,5-a]pyrazolo[5,1-c]pyrazin-3-yl)-1,1,1-trifluoropropan-2-ol (compound of Example 23)

### Step 1

### ethyl 5-hydroxy-1H-pyrazole-3-carboxylate

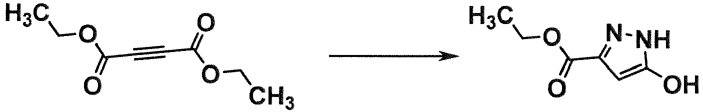

Diethyl acetylenedicarboxylate (250 ml) was mixed with acetonitrile (797 ml). Under ice-cooling, hydrazine monohydrate (80 ml) and acetic acid (17.9 ml) were successively added dropwise, and the mixture was stirred at room temperature for 24 hr. The reaction mixture was stirred under ice-cooling for 2 hr, and the precipitated solid was collected by filtration to give the title compound (156.71 g). ¹H-NMR (400 MHz, DMSO-D₆) 1.26 (t, J=7.05Hz, 3H), 3.94 - 4.53 (m, 2H), 5.39 - 6.26 (m, 1H), 9.47 - 11.36 (brm, 1H), 12.73 (s, 1H)

### Step 2

### ethyl 5-hydroxy-1-(tetrahydro-2H-pyran-2-yl)-1H-pyrazole-3-carboxylate

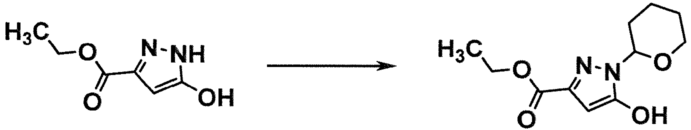

Ethyl 5-hydroxy-1H-pyrazole-3-carboxylate (300 g) obtained in the previous step was mixed with acetonitrile (720 ml). Pyridinium p-toluenesulfonate (24.14 g) was added at room temperature. To the mixture was added dropwise 3,4-dihydro-2H-pyran (170 g), and the mixture was stirred at room temperature for 2 days. The reaction mixture was stirred under ice-cooling for 2 hr, and the precipitated solid was collected by filtration to give the title compound (410 g).
¹H-NMR (400 MHz, DMSO-D₆) 1.25 (t, J=7.05Hz, 3H), 1.42 - 1.77 (m, 4H), 1.88 - 1.99 (m, 1H), 2.12 - 2.26 (m, 1H), 3.49 - 3.61 (m, 1H), 3.85 - 3.94 (m, 1H), 4.13 - 4.29 (m, 2H), 5.27 - 5.35 (m, 1H), 5.73 (s, 1H), 11.63 (s, 1H)

### Step 3

### ethyl 5-(benzyloxy)-1-(tetrahydro-2H-pyran-2-yl)-1H-pyrazole-3-carboxylate

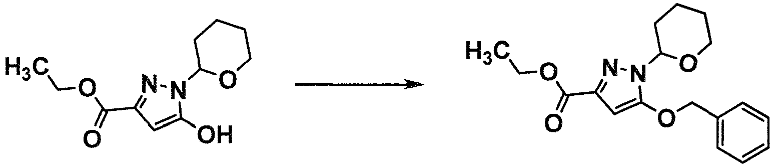

Ethyl 5-hydroxy-1-(tetrahydro-2H-pyran-2-yl)-1H-pyrazole-3-carboxylate (205 g) obtained in the previous step was mixed with N-methylpyrrolidone (615 ml). Under water-cooling, potassium carbonate (142 g) was added, benzyl bromide (175 g) was added dropwise, and the mixture was stirred at room temperature for 2 hr. To the mixture was added dropwise acetic acid (25.6 g), and the mixture was stirred at room temperature overnight. To the reaction mixture were added water (1.23 L), and the mixture was extracted with isopropyl acetate (2.05 L). The organic layer was washed twice with 10% aqueous sodium chloride solution (1.025 L), and concentrated under reduced pressure. The obtained residue was azeotroped with isopropyl acetate (2.05 L). To the obtained solid was added isopropyl acetate (615 ml), and the mixture was stirred with heating at 55°C to dissolve the solid. The solution was allowed to cool to 40°C, and seed crystals were inoculated. To the obtained suspension was added dropwise n-heptane (2.46 L), and the mixture was allowed to cool to room temperature, and stirred at room temperature for 2 hr. The mixture was stirred under ice-cooling for 2 hr, and the precipitated solid was collected by filtration to give the title compound (235.15 g).
¹H-NMR (400 MHz, DMSO-D₆) 1.26 (t, J=7.05Hz, 3H), 1.45 - 1.56 (m, 2H), 1.57 - 1.80 (m, 2H), 1.89 - 2.00 (m, 1H), 2.11 - 2.26 (m, 1H), 3.52 - 3.62 (m, 1H), 3.84 - 3.92 (m, 1H), 4.19 - 4.28 (m, 2H), 5.19 - 5.31 (m, 2H), 5.35 - 5.42 (m, 1H), 6.22 (s, 1H), 7.30 - 7.51 (m, 5H)

In this step, the crystal of the title compound can be obtained without using seed crystals.

### Step 4

### 5-(benzyloxy)-N-methoxy-N-methyl-1-(tetrahydro-2H-pyran-2-yl)-1H-pyrazole-3-carboxamide

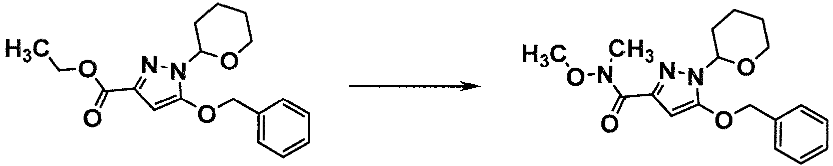

Ethyl 5-(benzyloxy)-1-(tetrahydro-2H-pyran-2-yl)-1H-pyrazole-3-carboxylate (235.15 g) obtained in the previous step was mixed with 1,2-dimethoxyethane (941 ml). To the mixture was added dropwise 2N sodium hydroxide aqueous solution (428 ml), and the mixture was stirred at room temperature for 3.5 hr. Under water-cooling, to the mixture were added N-methoxy-N-methylamine hydrochloride (83 g) and HOBt-H₂O (21.8 g), WSC-HCl (164 g) was added in 4 portions, and the mixture was stirred at room temperature overnight. To the reaction mixture was added 5% sodium hydrogen carbonate aqueous solution (941 ml), and the mixture was extracted with toluene (1411 ml). The organic layer was washed with 5% aqueous sodium chloride solution (941 ml), and concentrated under reduced pressure. The obtained residue was azeotroped with toluene (2.35 L). To the obtained residue was added toluene to prepare a solution of the title compound containing toluene in 5 times the weight of the starting material.

### Step 5

### 5-(benzyloxy)-1-(tetrahydro-2H-pyran-2-yl)-1H-pyrazole-3-carbaldehyde

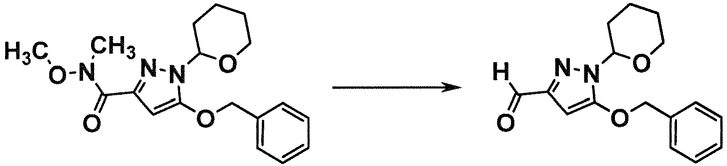

Under ice-cooling, to the 5-(benzyloxy)-N-methoxy-N-methyl-1-(tetrahydro-2H-pyran-2-yl)-1H-pyrazole-3-carboxamide/toluene solution obtained in the previous step was added dropwise 70% sodium bis(2-methoxyethoxy)aluminum hydride/toluene solution (141 ml), and the mixture was stirred for 2.5 hr. Under water-cooling, to the reaction mixture was added dropwise isopropanol (54.8 ml)/toluene (118 ml) solution. The mixture was allowed to warm to room temperature, Rochelle salt (201 g)/water (941 ml) was added, and the mixture was stirred for 1 hr. The solution was partitioned, and the organic layer was washed twice with 5% aqueous sodium chloride solution (941 ml) and concentrated under reduced pressure. The obtained residue was solvent-substituted with 1,2-dimethoxyethane (2.35 L). To the obtained residue was added 1,2-dimethoxyethane to prepare a solution of the title compound containing 1,2-dimethoxyethane in 13 times the weight of the starting material.

### Step 6

### 5-(benzyloxy)-1H-pyrazole-3-carbaldehyde

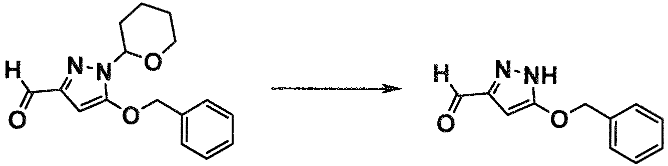

Under ice-cooling, to 5-(benzyloxy)-1-(tetrahydro-2H-pyran-2-yl)-1H-pyrazole-3-carbaldehyde/1,2-dimethoxyethane solution obtained in the previous step was added 1N hydrochloric acid (1423 ml), and the mixture was stirred at room temperature overnight. The precipitated solid was collected by filtration to give the title compound (132.04 g). ¹H-NMR (400 MHz, DMSO-D₆) 5.18 (s, 2H), 6.36 (s, 1H), 7.26 - 7.49 (m, 5H), 9.70 (s, 1H), 13.34 (brs, 1H)

### Step 7

### (S)-2-(5-(3-(benzyloxy)-1H-pyrazol-5-yl)-1H-imidazol-1-yl)propan-1-ol

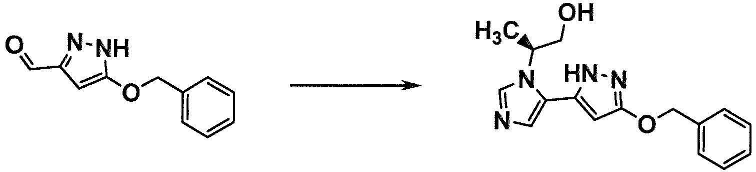

5-(Benzyloxy)-1H-pyrazole-3-carbaldehyde (261.97 g) obtained in the previous step was mixed with dimethylformamide (1040 ml). (S)-2-Aminopropan-1-ol (102 g) was added at room temperature, and the mixture was stirred at room temperature overnight. To the mixture was added potassium carbonate (358 g), and p-toluenesulfonylmethyl isocyanide (304 g) was added in 3 portions, and the mixture was stirred at room temperature overnight. To the reaction mixture was added potassium tert-butoxide (72.7 g), and the mixture was stirred at room temperature overnight. To the reaction mixture were added 2-methyltetrahydrofuran (1572 ml) and KC FLOCK (W-300G, Nippon Paper Industries Co., Ltd., 131 g), and the mixture was filtered using KC FLOCK as an adjuvant. To the filtrate was added 2-methyltetrahydrofuran (1572 ml), and the mixture was stirred at room temperature and filtered. The filtrates were combined and washed 3 times with 10% aqueous sodium chloride solution (1048 ml). The first aqueous layer was extracted with 2-methyltetrahydrofuran (2620 ml) and washed 3 times with 10% aqueous sodium chloride solution (1048 ml). The organic layers were combined, concentrated under reduced pressure, and azeotroped successively with toluene and 2-methyltetrahydrofuran. To the obtained residue was added 2-methyltetrahydrofuran to prepare a solution of the title compound containing 2-methyltetrahydrofuran in 4 times the weight of the starting material.

### Step 8

### (S)-9-(benzyloxy)-5-methyl-5,6-dihydroimidazo[1,5-a]pyrazolo[5,1-c]pyrazine hydrochloride

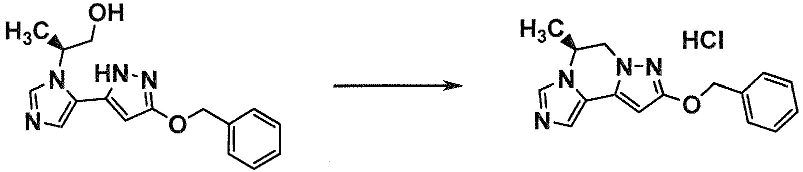

(S)-2-(5-(3-(Benzyloxy)-1H-pyrazol-5-yl)-1H-imidazol-1-yl)propan-1-ol/2-methyltetrahydrofuran solution obtained in the previous step was mixed with triphenylphosphine (408 g). Under heating at 73°C, 40% diisopropyl azodicarboxylate/toluene solution (763 ml) was added dropwise, and the mixture was stirred for 1 hr. Under heating at 60°C, 2N hydrochloric acid/ethanol solution (842 ml) was added dropwise, and the mixture was stirred at room temperature overnight. The precipitated solid was collected by filtration to give the title compound (244.13 g).
¹H-NMR (400 MHz, DMSO-D₆) 1.59 (d, J=6.47Hz, 3H), 4.05 - 4.19 (m, 1H), 4.43 - 4.53 (m, 1H), 4.85 - 4.99 (m, 1H), 5.19 (s, 2H), 6.23 (s, 1H), 7.26 - 7.48 (m, 5H), 8.01 (d, J=1.16Hz, 1H), 9.29 (s, 1H)

### Step 9

### (S)-9-(benzyloxy)-5-methyl-5,6-dihydroimidazo[1,5-a]pyrazolo[5,1-c]pyrazine

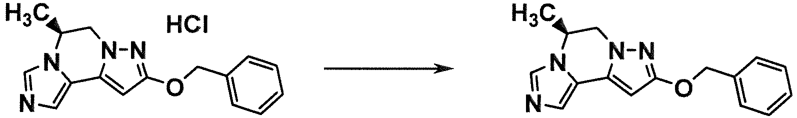

(S)-9-(Benzyloxy)-5-methyl-5,6-dihydroimidazo[1,5-a]pyrazolo[5,1-c]pyrazine hydrochloride (224 g) obtained in the previous step was mixed with 2-methyltetrahydrofuran (896 ml) and water (448 ml). To the mixture was added 4N sodium hydroxide aqueous solution (194 ml), and the mixture was stirred for 1 hr. The solution was partitioned, and the organic layer was washed with 20% aqueous sodium chloride solution (896 ml) and concentrated under reduced pressure. The obtained residue was azeotroped successively with toluene (1 L) and tetrahydrofuran (1.1 L). To the obtained residue was added tetrahydrofuran to prepare a solution of the title compound containing tetrahydrofuran in 5.4 times the weight of the starting material.

### Step 10

### (S)-1-(9-(benzyloxy)-5-methyl-5,6-dihydroimidazo[1,5-a]pyrazolo[5,1-c]pyrazin-3-yl)ethan-1-one

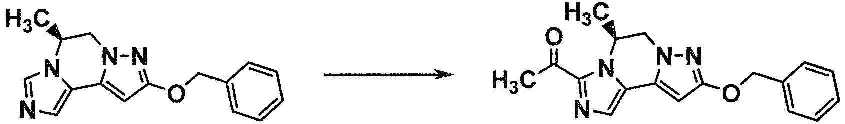

Under cooling at -78°C, to (S)-9-(benzyloxy)-5-methyl-5,6-dihydroimidazo[1,5-a]pyrazolo[5,1-c]pyrazine/tetrahydrofuran solution obtained in the previous step was added dropwise 1M lithium diisopropylamide/hexane-tetrahydrofuran solution (634 ml), and the mixture was stirred for 1 hr. To the mixture was added N-methoxy-N-methylacetamide (173 ml), and the mixture was stirred at -78°C for 1.5 hr. To the mixture was added dropwise 1M lithium diisopropylamide/hexane-tetrahydrofuran solution (667 ml), and the mixture was stirred for 2.5 hr. The reaction mixture was added dropwise to acetic acid (405 ml)/ethanol (672 ml) solution cooled to 0°C. The precipitated solid was collected by filtration. The obtained solid was mixed with ethanol/water=1/2 (2016 ml), and the mixture was stirred at room temperature. The precipitated solid was collected by filtration to give the title compound (205.85 g).
¹H-NMR (400 MHz, CDCl₃) 1.44 (d, J=6.58Hz, 3H), 2.69 (s, 3H), 4.19 (dd, J=13.45, 1.20Hz, 1H), 4.29 (dd, J=13.45, 4.63Hz, 1H), 5.24 (s, 2H), 5.75 - 5.84 (m, 1H), 5.98 (s, 1H), 7.32 - 7.50 (m, 6H)

### Step 11

### (S)-1-(9-hydroxy-5-methyl-5,6-dihydroimidazo[1,5-a]pyrazolo[5,1-c]pyrazin-3-yl)ethan-1-one

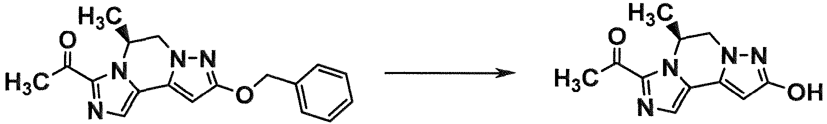

(S)-1-(9-(Benzyloxy)-5-methyl-5,6-dihydroimidazo[1,5-a]pyrazolo[5,1-c]pyrazin-3-yl)ethan-1-one (205.7 g) obtained in the previous step was mixed with concentrated hydrochloric acid (563 ml). The mixture was stirred at 48°C overnight and then stirred at room temperature overnight. The reaction mixture was washed with toluene (1.029 L). The obtained aqueous layer was diluted with ethanol (206 ml) and water (411 ml), and 4N sodium hydroxide aqueous solution (1276 ml) and 10% trisodium citrate aqueous solution (1076 ml) were added. After stirring under ice-cooling for 2 hr, the precipitated solid was collected by filtration to give the title compound (144.84 g).
¹H-NMR (400 MHz, DMSO-D₆) 1.28 (d, J=6.73Hz, 3H), 2.57 (s, 3H), 4.12 (d, J=13.46Hz, 1H), 4.22 (dd, J=13.46, 4.49Hz, 1H), 5.53 - 5.65 (m, 1H), 5.94 (s, 1H), 7.48 (s, 1H), 10.04 (s, 1H)

### Step 12

### (S)-1-(9-(difluoromethoxy)-5-methyl-5,6-dihydroimidazo[1,5-a]pyrazolo[5,1-c]pyrazin-3-yl)ethan-1-one

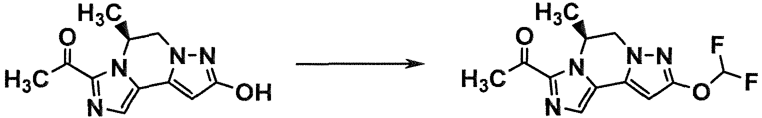

(S)-1-(9-Hydroxy-5-methyl-5,6-dihydroimidazo[1,5-a]pyrazolo[5,1-c]pyrazin-3-yl)ethan-1-one (1 g) obtained in the previous step was mixed with acetonitrile (10 ml). To the mixture was added 8M potassium hydroxide aqueous solution (168 ml). Under ice-cooling, to the mixture was added dropwise diethyl (bromodifluoromethyl)phosphonate (1.533 ml), and the mixture was stirred under water-cooling for 1 hr. To the reaction mixture were added water (20 ml) and ethyl acetate (20 ml) at room temperature, and the mixture was extracted twice with ethyl acetate. The organic layer was concentrated under reduced pressure, and the obtained residue was purified by silica gel column chromatography (hexane:ethyl acetate=3:1 to 1:1) to give the title compound (561 mg).
¹H-NMR (400 MHz, DMSO-D₆) 1.27 (d, J=6.70Hz, 3H), 2.58 (s, 3H), 4.25 - 4.43 (m, 2H), 5.59 - 5.69 (m, 1H), 6.50 (s, 1H), 7.30 (t, J=73.06Hz, 1H), 7.57 (s, 1H)

### Step 13

### (R)-2-((S)-9-(difluoromethoxy)-5-methyl-5,6-dihydroimidazo[1,5-a]pyrazolo[5,1-c]pyrazin-3-yl)-1,1,1-trifluoropropan-2-ol

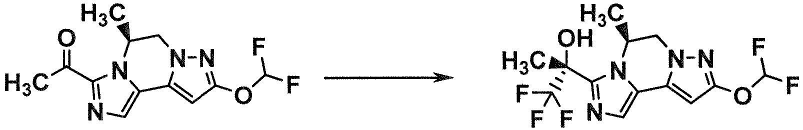

(S)-1-(9-(Difluoromethoxy)-5-methyl-5,6-dihydroimidazo[1,5-a]pyrazolo[5,1-c]pyrazin-3-yl)ethan-1-one (561 mg) obtained in the previous step was mixed with tetrahydrofuran (5.6 ml). To the mixture was added cesium fluoride (0.06 g). Under ice-cooling, (trifluoromethyl)trimethylsilane (0.587 ml) was added dropwise, and the mixture was stirred at room temperature for 1.5 hr. To the reaction mixture were added methanol (3.366 ml) and potassium carbonate (0.33 g), and the mixture was stirred at room temperature for 1 hr. The reaction mixture was concentrated under reduced pressure, and the obtained residue was purified by silica gel column chromatography (ethyl acetate:hexane=1:3 to 1:1) to give the title compound (529 mg). ¹H-NMR (400 MHz, DMSO-D₆) 1.27 (d, J=6.70Hz, 3H), 1.81 (s, 3H), 4.20 - 4.30 (m, 2H), 5.31 - 5.40 (m, 1H), 6.31 (s, 1H), 7.27 (t, J=73.29Hz, 1H), 7.31 (s, 1H), 7.32 (s, 1H)

The compounds of Examples 1 to 103 were obtained according to methods similar to the above-mentioned Production Method 1 to Production Method 10 and Production Examples 1 to 10, or other known methods as necessary. The structural formulas and property data of the Example compounds are shown in the following Tables. The Remarks in the Tables show the following contents.
1 (Examples 1, 3, 5, 6, 24, 25, 34, 35 and 50)
   racemates
2 (Example 26) mixture of two kinds of diastereomers
3 (Example 97)
   steric configuration of cyclopropane moiety is trans, mixture of two kinds of diastereomers

**[Table 1-1]**

| Example | Structure | ¹H-NMR | Mass M+1 | Mass M-1 | Remarks |
|---|---|---|---|---|---|
| 1 | 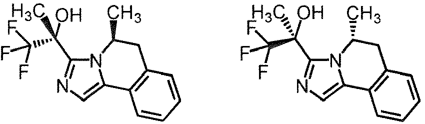 | (400 MHz, CDCl3) 1.21 (d, J=6.47Hz, 3H), 1.95 (s, 3H), 2.77 (dd, J=15.49, 1.62Hz, 1H), 3.28 (dd, J=15.49, 5.32Hz, 1H), 3.52 (s, 1H), 5.10 - 5.20 (m, 1H), 7.17 - 7.30 (m, 5H), 7.33 (s, 1H), 7.54 (d, J=7.40Hz, 1H) | 297 | - | 1 |
| 2 | 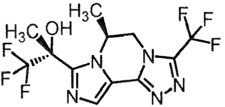 | (400 MHz, DMSO-D6) 1.28 (d, J=6.47Hz, 3H), 1.86 (s, 3H), 4.48 (dd, J=13.64, 3.93Hz, 1H), 4.55 (d, J=13.64Hz, 1H), 5.45 - 5.51 (m, 1H), 7.49 (s, 1H), 7.75 (s, 1H) | 356 | 354 | |
| 3 | 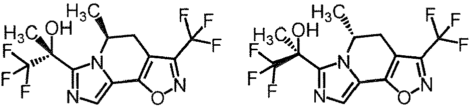 | (400 MHz, DMSO-D6) 1.21 (d, J=6.70Hz, 3H), 1.86 (s, 3H), 3.09 - 3.18 (m, 2H), 5.44 - 5.50 (m, 1H), 7.40 (s, 1H), 7.68 (s, 1H) | 356 | 354 | 1 |
| 4 | 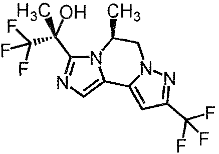 | (400 MHz, DMSO-D6) 1.24 (d, J=6.47Hz, 3H), 1.82 (s, 3H), 4.40 - 4.56 (m, 2H), 5.37 - 5.46 (m, 1H), 7.07 (s, 1H), 7.36 (s, 1H), 7.40 (s, 1H) | 355 | - | |
| 5 | 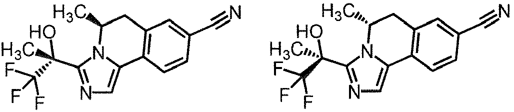 | (400 MHz, DMSO-D6) 1.12 (d, J=6.47Hz, 3H), 1.81 (s, 3H), 2.98 (dd, J=16.07, 1.62Hz, 1H), 3.11 (dd, J=16.07, 5.43Hz, 1H), 5.20 - 5.31 (m, 1H), 7.21 (s, 1H), 7.59 (s, 1H), 7.73 (d, J=8.09Hz, 1H), 7.79 (s, 1H), 7.82 (d, J=8.09Hz, 1H) | 322 | - | 1 |

**[Table 1-2]**

| Example | Structure | ¹ H-NMR | Mass M+1 | Mass M-1 | Remarks |
|---|---|---|---|---|---|
| 6 | 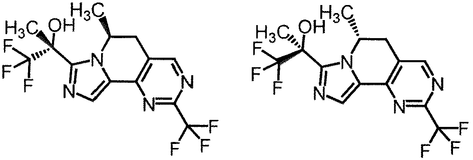 | (400 MHz, DMSO-D6) 1.20 (d, J=6.47Hz, 3H), 1.84 (s, 3H), 3.14 (d, J=16.41Hz, 1H), 3.23 (dd, J=16.41, 5.32Hz, 1H), 5.34 - 5.44 (m, 1H), 7.40 (s, 1H), 7.81 (s, 1H), 8.92 (s, 1H) | 367 | - | 1 |
| 7 | 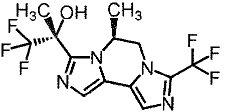 | (400 MHz, DMSO-D6) 1.20 (d, J=6.70Hz, 3H), 1.81 (s, 3H), 4.31 (dd, J=13.76, 3.81Hz, 1H), 4.51 (d, J=13.76Hz, 1H), 5.33 - 5.44 (m, 1H), 7.31 (s, 1H), 7.37 (s, 1H), 7.45 (s, 1H) | 355 | - | |
| 8 | 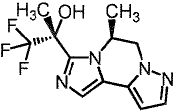 | (400 MHz, DMSO-D6) 1.21 (d, J=6.47Hz, 3H), 1.81 (s, 3H), 4.28 (dd, J=13.64, 3.93Hz, 1H), 4.42 (dd, J=13.64, 1.16Hz, 1H), 5.30 - 5.41 (m, 1H), 6.53 (d, J=2.08Hz, 1H), 7.26 (s, 1H), 7.27 (s, 1H), 7.51 (d, J=2.08Hz, 1H) | 287 | - | |
| 9 | 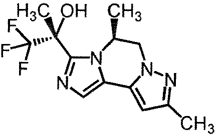 | (400 MHz, DMSO-D6) 1.22 (d, J=6.47Hz, 3H), 1.80 (s, 3H), 2.18 (s, 3H), 4.19 (dd, J=13.52, 4.05Hz, 1H), 4.29 (dd, J=13.52, 1.04Hz, 1H), 5.26 - 5.36 (m, 1H), 6.30 (s, 1H), 7.20 (s, 1H), 7.24 (s, 1H) | 301 | 299 | |

**[Table 1-3]**

| Example | Structure | ¹H-NMR | Mass M+1 | Mass M-1 | Remarks |
|---|---|---|---|---|---|
| 10 | 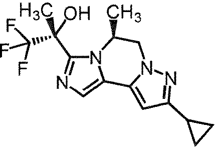 | (400 MHz, DMSO-D6) 0.62 - 0.69 (m, 2H), 0.82 - 0.90 (m, 2H), 1.21 (d, J=6.70Hz, 3H), 1.80 (s, 3H), 1.83 - 1.91 (m, 1H), 4.18 (dd, J=13.64, 3.93Hz, 1H), 4.28 (dd, J=13.64, 1.04Hz, 1H), 5.25 - 5.36 (m, 1H), 6.22 (s, 1H), 7.19 (s, 1H), 7.24 (s, 1H) | 327 | 325 | |
| 11 | 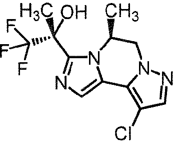 | (400 MHz, DMSO-D6) 1.23 (d, J=6.70Hz, 3H), 1.82 (s, 3H), 4.34 (dd, J=13.64, 3.81Hz, 1H), 4.41 (dd, J=13.64, 1.39Hz, 1H), 5.34 - 5.44 (m, 1H), 7.35 (s, 1H), 7.38 (s, 1H), 7.67 (s, 1H) | 321 | 319 | |
| 12 | 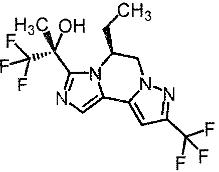 | (400 MHz, CDCl3) 0.96 (t, J=7.47Hz, 3H), 1.51 - 1.55 (m, 1H), 1.75 - 1.77 (m, 1H), 2.02 (s, 3H), 2.94 (s, 1H), 4.35 (d, J=13.75Hz, 1H), 4.61 (d, J=13.75Hz, 1H), 5.11 - 5.13 (m, 1H), 6.68 (s, 1H), 7.32 (s, 1H) | 369 | 367 | |
| 13 | 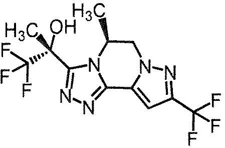 | (400 MHz, DMSO-D6) 1.30 (d, J=6.58Hz, 3H), 1.91 (s, 3H), 4.62 (d, J=13.75Hz, 1H), 4.70 (dd, J=13.75, 4.04Hz, 1H), 5.31 - 5.42 (m, 1H), 7.47 (s, 1H), 7.71 (s, 1H) | 356 | - | |

**[Table 1-4]**

| Example | Structure | ¹H-NMR | Mass M+1 | Mass M-1 | Remarks |
|---|---|---|---|---|---|
| 14 | 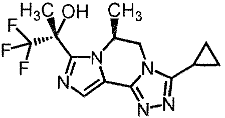 | (400 MHz, DMSO-D6) 1.30 (d, J=6.58Hz, 3H), 1.91 (s, 3H), 4.62 (d, J=13.75Hz, 1H), 4.70 (dd, J=13.75, 4.04Hz, 1H), 5.31 - 5.42 (m, 1H), 7.47 (s, 1H), 7.71 (s, 1H) | 328 | - | |
| 15 | 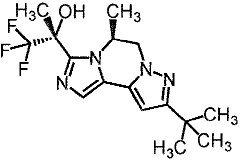 | (400 MHz, CDCl3) 1.34 (s, 9H), 1.37 (d, J=6.58Hz, 3H), 1.99 (s, 3H), 3.11 (s, 1H), 4.28 - 4.36 (m, 2H), 5.31 - 5.34 (m, 1H), 6.30 (s, 1H), 7.22 (s, 1H) | 343 | - | |
| 16 | 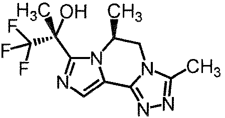 | (400 MHz, DMSO-D6) 1.20 (d, J=6.47Hz, 3H), 1.83 (s, 3H), 2.38 (s, 3H), 4.09 (dd, J=13.52, 4.05Hz, 1H), 4.33 (dd, J=13.52, 1.16Hz, 1H), 5.34 - 5.45 (m, 1H), 7.35 (s, 1H), 7.48 (s, 1H) | 302 | - | |
| 17 | 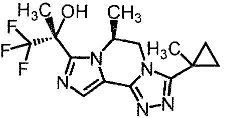 | (400 MHz, DMSO-D6) 0.71 - 0.88 (m, 3H), 1.07 - 1.14 (m, 1H), 1.21 (d, J=6.47Hz, 3H), 1.35 (s, 3H), 1.83 (s, 3H), 4.25 (dd, J=13.52, 4.05Hz, 1H), 4.46 (dd, J=13.52, 1.16Hz, 1H), 5.35 - 5.46 (m, 1H), 7.35 (s, 1H), 7.49 (s, 1H) | 342 | - | |
| 18 | 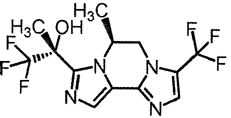 | (400 MHz, DMSO-D6) 1.26 (d, J=6.47Hz, 3H), 1.83 (s, 3H), 4.29 - 4.41 (m, 2H), 5.36 - 5.48 (m, 1H), 7.37 (s, 1H), 7.48 (s, 1H), 7.63 - 7.65 (m, 1H) | 355 | - | |

**[Table 1-5]**

| Example | Structure | ¹ H-NMR | Mass M+1 | Mass M-1 | Remarks |
|---|---|---|---|---|---|
| 19 | 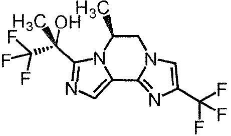 | (400 MHz, DMSO-D6) 1.23 (d, J=6.70Hz, 3H), 1.83 (s, 3H), 4.26 (dd, J=13.64, 3.93Hz, 1H), 4.38 (dd, J=13.64, 1.16Hz, 1H), 5.35 - 5.46 (m, 1H), 7.33 (s, 1H), 7.44 (s, 1H), 7.95 - 7.98 (m, 1H) | 355 | - | |
| 20 | 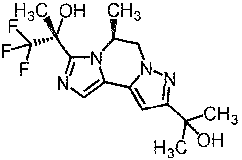 | (400 MHz, CDCl3) 1.61 (s, 6H), 2.00 (s, 3H), 2.45 (s, 1H), 3.08 (s, 1H), 4.30 - 4.36 (m, 2H), 5.35 - 5.36 (m, 1H), 6.38 (s, 1H), 7.25 (s, 1H) | 345 | - | |
| 21 | 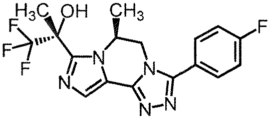 | (400 MHz, DMSO-D6) 1.17 (d, J=6.47Hz, 3H), 1.85 (s, 3H), 4.38 (dd, J=13.52, 1.04Hz, 1H), 4.52 (dd, J=13.52, 4.05Hz, 1H), 5.37 - 5.48 (m, 1H), 7.37 - 7.48 (m, 3H), 7.60 (s, 1H), 7.76 - 7.85 (m, 2H) | 382 | - | |
| 22 | 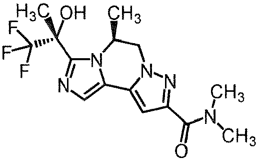 | (400 MHz, CDCl3) 2.06 (s, 3H), 3.11 (s, 3H), 3.33 (s, 3H), 4.26 (d, J=13.75Hz, 1H), 4.36 - 4.40 (m, 2H), 5.39 - 5.40 (m, 1H), 6.43 (s, 1H), 7.33 (s, 1H) | 358 | 356 | |

**[Table 1-6]**

| Example | Structure | ¹H-NMR | Mass M+1 | Mass M-1 | Remarks |
|---|---|---|---|---|---|
| 23 | 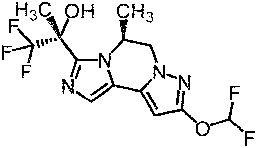 | (400 MHz, DMSO-D6) 1.27 (d, J=6.70Hz, 3H), 1.81 (s, 3H), 4.20 - 4.30 (m, 2H), 5.31 - 5.40 (m, 1H), 6.31 (s, 1H), 7.27 (t, J=73.29Hz, 1H), 7.31 (s, 1H), 7.32 (s, 1H) | 353 | 351 | |
| 24 | 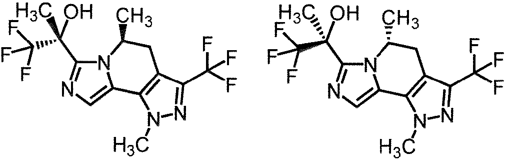 | (400 MHz, DMSO-D6) 1.16 (d, J=6.58Hz, 3H), 1.84 (s, 3H), 2.90 (d, J=16.14Hz, 1H), 2.97 (dd, J=16.14, 5.38Hz, 1H), 4.04 (s, 3H), 5.32 - 5.43 (m, 1H), 7.27 (s, 1H), 7.46 (s, 1H) | 369 | 367 | 1 |
| 25 | 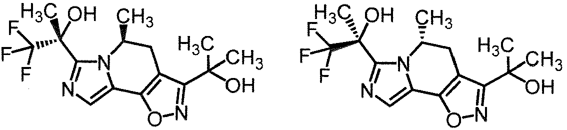 | (400 MHz, DMSO-D6) 1.20 (d, J=6.47Hz, 3H), 1.46 (s, 3H), 1.50 (s, 3H), 1.82 (s, 3H), 2.94 (dd, J=16.53, 5.90Hz, 1H), 3.05 (d, J=16.41Hz, 1H), 5.29 - 5.39 (m, 1H), 5.44 (s, 1H), 7.27 (s, 1H), 7.37 (s, 1H) | 346 | 344 | 1 |
| 26 | 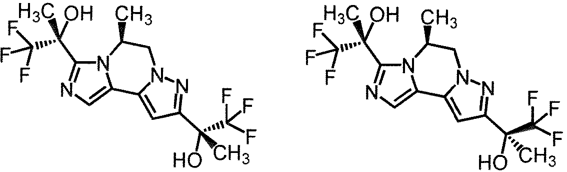 | (400 MHz, CDCl3) 1.37 (d, J=6.58Hz, 3H), 1.73 (d, J=3.89Hz, 3H), 2.01 (s, 3H), 2.99 (s, 1H), 4.31 - 4.43 (m, 2H), 5.39 - 5.40 (m, 1H), 6.46 (d, J=2.69Hz, 1H), 7.29 (s, 1H) | 399 | - | 2 |

**[Table 1-7]**

| Example | Structure | ¹H-NMR | Mass M+1 | Mass M-1 | Remarks |
|---|---|---|---|---|---|
| 27 | 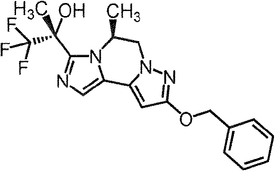 | (400 MHz, DMSO-D6) 1.28 (d, J=6.47Hz, 3H), 1.80 (s, 3H), 4.11 - 4.24 (m, 2H), 5.15 (s, 2H), 5.27 - 5.36 (m, 1H), 6.03 (s, 1H), 7.23 (s, 1H), 7.26 (s, 1H), 7.28 - 7.48 (m, 5H) | 393 | - | |
| 28 | 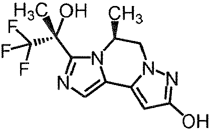 | (400 MHz, DMSO-D6) 1.26 (d, J=6.47Hz, 3H), 1.80 (s, 3H), 4.04 - 4.10 (m, 2H), 5.23 - 5.33 (m, 1H), 5.74 (s, 1H), 7.19 (s, 1H), 7.23 (brs, 1H), 9.85 (brs, 1H) | 303 | - | |
| 29 | 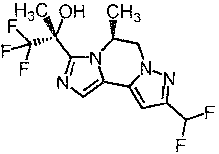 | (400 MHz, CDCl3) 1.37 (d, J=6.58Hz, 3H), 2.01 (s, 3H), 3.09 (s, 1H), 4.34 - 4.44 (m, 2H), 5.41 - 5.43 (m, 1H), 6.62 - 6.78 (m, 2H), 7.30 (d, J=4.78Hz, 1H) | 337 | - | |
| 30 | 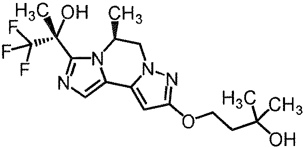 | (400 MHz, DMSO-D6) 1.13 (s, 6H), 1.27 (d, J=6.47Hz, 3H), 1.76 - 1.84 (m, 5H), 4.12 - 4.21 (m, 4H), 4.32 (brs, 1H), 5.25 - 5.35 (m, 1H), 5.96 (s, 1H), 7.22 (s, 1H), 7.25 (s, 1H) | 389 | - | |

**[Table 1-8]**

| Example | Structure | ¹ H-NMR | Mass M+1 | Mass M-1 | Remarks |
|---|---|---|---|---|---|
| 31 | 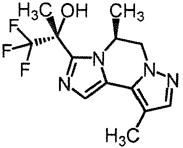 | (400 MHz, DMSO-D6) 1.19 (d, J=6.47Hz, 3H), 1.81 (s, 3H), 2.14 (s, 3H), 4.23 (dd, J=13.64, 3.93Hz, 1H), 4.34 (dd, J=13.64, 1.27Hz, 1H), 5.29 - 5.39 (m, 1H), 7.23 (s, 1H), 7.25 (s, 1H), 7.33 (s, 1H) | 301 | - | |
| 32 | 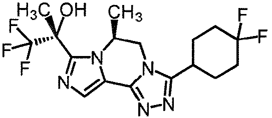 | (400 MHz, DMSO-D6) 1.20 (d, J=6.70Hz, 3H), 1.67 - 2.27 (m, 11H), 3.02 - 3.12 (m, 1H), 4.18 (dd, J=13.41, 3.93Hz, 1H), 4.43 (d, J=13.41Hz, 1H), 5.34 - 5.49 (m, 1H), 7.35 (s, 1H), 7.51 (s, 1H) | 406 | - | |
| 33 | 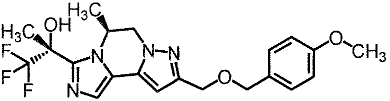 | (400 MHz, CDCl3) 1.36 (d, J=6.58Hz, 3H), 2.00 (s, 3H), 2.97 (s, 1H), 3.81 (s, 3H), 4.35 (dd, J=7.92, 2.84Hz, 2H), 4.55 (d, J=3.29Hz, 4H), 6.47 (s, 1H), 6.89 (d, J=8.67Hz, 2H), 7.25 (s, 1H), 7.31 (d, J=8.67Hz, 2H) | 437 | 435 | |
| 34 | 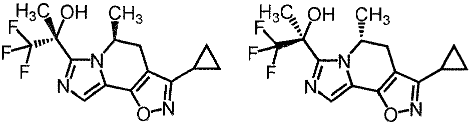 | (400 MHz, CDCl3) 1.02 - 1.04 (m, 4H), 1.30 (d, J=3.29Hz, 3H), 2.00 (s, 3H), 2.63 - 2.67 (m, 1H), 3.05 - 3.11 (m, 1H), 3.33 - 3.37 (m, 1H), 5.30 - 5.36 (m, 1H), 7.41 (s, 1H) | 328 | - | 1 |
| 35 | 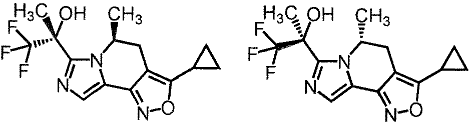 | (400 MHz, CDCl3) 1.06 - 1.16 (m, 4H), 1.31 (d, J=6.58Hz, 3H), 1.85 (s, 3H), 2.79 (dd, J=15.55, 1.79Hz, 1H), 2.96 - 3.02 (m, 1H), 3.87 - 4.04 (m, 1H), 5.42 - 5.44 (m, 1H), 7.60 (s, 1H) | 328 | - | 1 |

**[Table 1-9]**

| Example | Structure | ¹ H-NMR | Mass M+1 | Mass M-1 | Remarks |
|---|---|---|---|---|---|
| 36 | 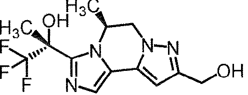 | (400 MHz, DMSO-D6) 1.22 (d, J=6.28Hz, 3H), 1.80 (s, 3H), 4.32 (d, J=6.13Hz, 2H), 4.39 - 4.43 (m, 2H), 5.29 - 5.37 (m, 1H), 6.46 (s, 1H), 7.25 (s, 1H) | 317 | 631 (Mx2-1) | |
| 37 | 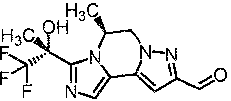 | (400 MHz, DMSO-D6) 1.25 (d, J=6.58Hz, 3H), 1.83 (s, 3H), 4.48 - 4.61 (m, 2H), 5.44 - 5.46 (m, 1H), 7.08 (s, 1H), 7.37 (s, 1H), 7.43 (s, 1H), 9.90 (s, 1H) | 315 | - | |
| 38 | 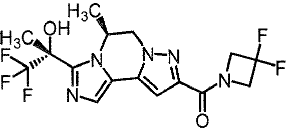 | (400 MHz, DMSO-D6) 1.24 (d, J=6.58Hz, 3H), 1.83 (s, 3H), 4.44 - 4.46 (m, 4H), 4.88 - 4.91 (m, 2H), 5.42 - 5.44 (m, 1H), 7.01 (s, 1H), 7.35 (s, 1H), 7.39 (s, 1H) | 406 | 404 | |
| 39 | 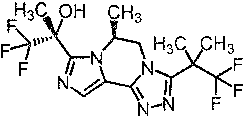 | (400 MHz, DMSO-D6) 1.20 (d, J=6.70Hz, 3H), 1.67 (s, 3H), 1.69 (s, 3H), 1.83 (s, 3H), 4.28 (dd, J=13.64, 3.70Hz, 1H), 4.71 (d, J=12.48Hz, 1H), 5.31 - 5.42 (m, 1H), 7.38 (s, 1H), 7.57 (s, 1H) | 398 | - | |
| 40 | 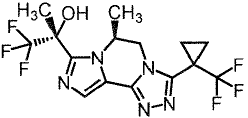 | (400 MHz, DMSO-D6) 1.21 (d, J=6.47Hz, 3H), 1.24 - 1.32 (m, 1H), 1.45 - 1.59 (m, 3H), 1.84 (s, 3H), 4.25 (dd, J=13.64, 3.93Hz, 1H), 4.46 (d, J=12.72Hz, 1H), 5.40 - 5.50 (m, 1H), 7.39 (s, 1H), 7.58 (s, 1H) | 396 | - | |

**[Table 1-10]**

| Example | Structure | ¹H-NMR | Mass M+1 | Mass M-1 | Remarks |
|---|---|---|---|---|---|
| 41 | 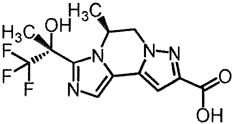 | (400 MHz, DMSO-D6) 1.16 - 1.21 (m, 3H), 1.82 (s, 3H), 4.26 - 4.41 (m, 1H), 4.41 - 4.54 (m, 1H), 5.31 - 5.46 (m, 1H), 6.80 (s, 1H), 7.33 (s, 1H) | 331 | 659 (Mx2-1) | |
| 42 | 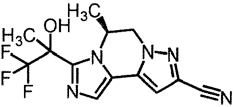 | (400 MHz, CDCl3) 1.36 (d, J=6.58Hz, 3H), 2.02 (s, 3H), 2.98 (s, 1H), 4.43 (d, J=2.69Hz, 2H), 5.45 - 5.47 (m, 1H), 6.81 (s, 1H), 7.35 (s, 1H) | 312 | 621 (Mx2-1) | |
| 43 | 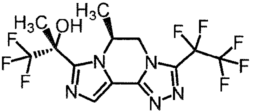 | (400 MHz, DMSO-D6) 1.27 (d, J=6.58Hz, 3H), 1.86 (s, 3H), 4.50 - 4.58 (m, 2H), 5.47 - 5.49 (m, 1H), 7.49 (s, 1H), 7.76 (s, 1H) | 406 | 405 | |
| 44 | 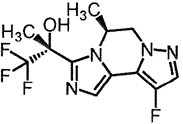 | (400 MHz, DMSO-D6) 1.25 (d, J=6.58Hz, 3H), 1.83 (s, 3H), 4.33 - 4.35 (m, 2H), 5.36 - 5.39 (m, 1H), 7.24 (s, 1H), 7.35 (s, 1H), 7.63 (d, J=4.19Hz, 1H) | 305 | 349 (M-1+46) | |
| 45 | 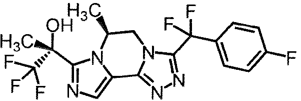 | (400 MHz, DMSO-D6) 1.21 (d, J=6.24Hz, 3H), 1.84 (s, 3H), 4.34 - 4.42 (m, 1H), 4.49 - 4.56 (m, 1H), 5.40 - 5.48 (m, 1H), 7.36 - 7.45 (m, 3H), 7.65 - 7.74 (m, 3H) | 432 | 430 | |

**[Table 1-11]**

| Example | Structure | ¹ H-NMR | Mass M+1 | Mass M-1 | Remarks |
|---|---|---|---|---|---|
| 46 | 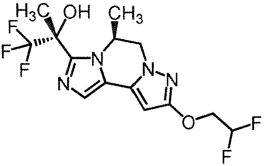 | (400 MHz, DMSO-D6) 1.27 (d, J=6.47Hz, 3H), 1.80 (s, 3H), 4.17 - 4.21 (m, 2H), 4.38 (td, J=14.80, 3.80Hz, 2H), 5.30 - 5.33 (m, 1H), 6.07 (s, 1H), 6.35 (tt, J=54.56, 3.93Hz, 1H), 7.25 (s, 1H), 7.29 (s, 1H) | 367 | 365 | |
| 47 | 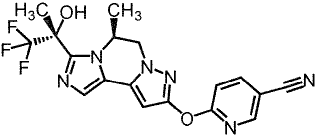 | (400 MHz, DMSO-D6) 1.30 (d, J=6.47Hz, 3H), 1.82 (s, 3H), 4.25 - 4.33 (m, 2H), 5.32 - 5.43 (m, 1H), 6.44 (s, 1H), 7.29 (dd, J=8.67, 0.69Hz, 1H), 7.31 (s, 1H), 7.33 (s, 1H), 8.34 (dd, J=8.67, 2.43Hz, 1H), 8.72 (dd, J=2.43, 0.69Hz, 1H) | 405 | 403 | |
| 48 | 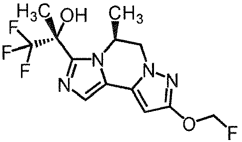 | (400 MHz, DMSO-D6) 1.27 (d, J=6.47Hz, 3H), 1.81 (s, 3H), 4.15 - 4.27 (m, 2H), 5.28 - 5.38 (m, 1H), 5.83 (d, J=53.64Hz, 2H), 6.19 (s, 1H), 7.29 (s, 2H) | 335 | 667 (Mx2-1) | |
| 49 | 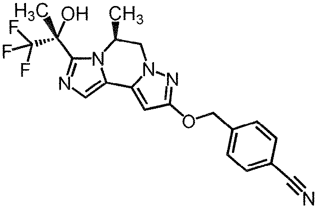 | (400 MHz, DMSO-D6) 1.27 (d, J=6.47Hz, 3H), 1.80 (s, 3H), 4.11 - 4.22 (m, 2H), 5.24 - 5.36 (m, 3H), 6.06 (s, 1H), 7.24 (s, 1H), 7.27 (s, 1H), 7.62 (d, J=8.32Hz, 2H), 7.85 (d, J=8.32Hz, 2H) | 418 | - | |

**[Table 1-12]**

| Example | Structure | ¹ H-NMR | Mass M+1 | Mass M-1 | Remarks |
|---|---|---|---|---|---|
| 50 | 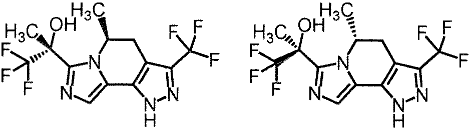 | (400 MHz, DMSO-D6) 1.17 (d, J=6.58Hz, 3H), 1.84 (s, 3H), 2.87 - 3.02 (m, 2H), 3.41 - 3.48 (m, 2H), 5.30 - 5.45 (m, 1H), 7.23 (s, 1H), 14.06 (s, 1H) | 355 | - | 1 |
| 51 | 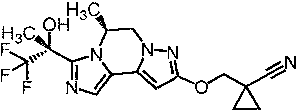 | (400 MHz, DMSO-D6) 1.18 - 1.18 (m, 2H), 1.28 (d, J=6.58Hz, 3H), 1.33 - 1.37 (m, 2H), 1.80 - 1.84 (m, 3H), 4.13 (s, 2H), 4.15 - 4.19 (m, 2H), 5.28 - 5.38 (m, 1H), 6.06 (s, 1H), 7.26 (s, 1H), 7.29 (s, 1H) | 382 | - | |
| 52 | 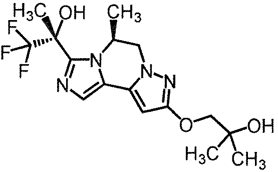 | (400 MHz, DMSO-D6) 1.16 (s, 6H), 1.27 (d, J=6.47Hz, 3H), 1.80 (s, 3H), 3.82 (s, 2H), 4.09 - 4.19 (m, 2H), 4.57 (s, 1H), 5.25 - 5.36 (m, 1H), 5.98 (s, 1H), 7.22 (s, 1H), 7.26 (s, 1H) | 375 | - | |
| 53 | 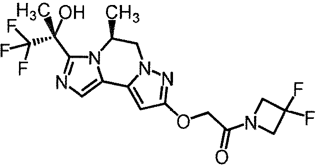 | (DMSO-D6) δ: 1.26 (d, J = 6.47 Hz, 3H), 1.80 (s, 3H), 4.10-4.21 (m, 2H), 4.27-4.39 (m, 2H), 4.62-4.74 (m, 4H), 5.26-5.35 (m, 1H), 6.04 (s, 1H), 7.25 (s, 1H), 7.27 (s, 1H). | 436 | - | |

**[Table 1-13]**

| Example | Structure | ¹ H-NMR | Mass M+1 | Mass M-1 | Remarks |
|---|---|---|---|---|---|
| 54 | 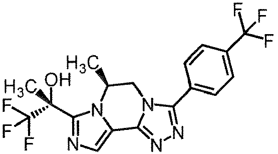 | (400 MHz, DMSO-D6) 1.20 (d, J=6.58Hz, 3H), 1.87 (s, 3H), 4.48 - 4.58 (m, 2H), 5.45 (s, 1H), 7.41 (s, 1H), 7.65 (s, 1H), 7.96 - 8.01 (m, 4H) | 432 | - | |
| 55 | 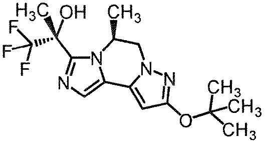 | (400 MHz, CDCl3) 1.36 (d, J=7.17Hz, 3H), 1.42 (s, 9H), 1.97 (s, 3H), 4.11 - 4.15 (m, 1H), 4.26 - 4.29 (m, 1H), 5.33 - 5.27 (m, 1H), 5.90 (s, 1H), 7.19 (s, 1H) | 359 | - | |
| 56 | 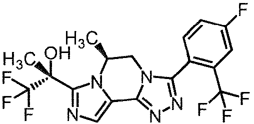 | (400 MHz, DMSO-D6) 1.17 (d, J=6.58Hz, 3H), 1.86 (s, 3H), 3.97 (d, J=13.45Hz, 1H), 4.24 (dd, J=13.60, 4.04Hz, 1H), 5.38 - 5.39 (m, 1H), 7.39 (s, 1H), 7.65 (s, 1H), 7.79 - 7.84 (m, 2H), 7.96 (dd, J=9.12, 2.24Hz, 1H) | 450 | - | |
| 57 | 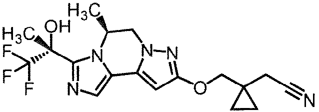 | (400 MHz, DMSO-D6) 0.67 (dt, J=23.02, 5.38Hz, 4H), 1.18 (d, J=5.98Hz, 2H), 1.28 (d, J=6.28Hz, 3H), 1.82 (s, 3H), 4.01 (s, 2H), 4.14 - 4.18 (m, 2H), 6.01 (s, 1H), 7.24 (s, 1H), 7.28 (s, 1H) | 396 | 394 | |
| 58 | 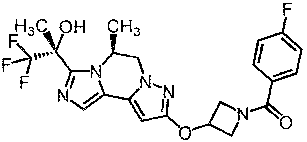 | (400 MHz, DMSO-D6) 1.26 (d, J=6.47Hz, 3H), 1.80 (s, 3H), 3.96 - 4.09 (m, 1H), 4.09 - 4.23 (m, 2H), 4.28 - 4.37 (m, 1H), 4.38 - 4.50 (m, 1H), 4.58 - 4.69 (m, 1H), 5.09 - 5.17 (m, 1H), 5.27 - 5.35 (m, 1H), 6.03 (s, 1H), 7.22 - 7.32 (m, 4H), 7.69 - 7.76 (m, 2H) | 480 | - | |

**[Table 1-14]**

| Example | Structure | ¹ H-NMR | Mass M+1 | Mass M-1 | Remarks |
|---|---|---|---|---|---|
| 59 | 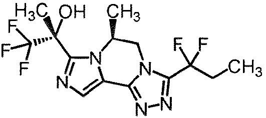 | (400 MHz, DMSO-D6) 1.09 (t, J=7.51Hz, 3H), 1.23 (d, J=6.47Hz, 3H), 1.84 (s, 3H), 2.40 - 2.58 (m, 2H), 4.38 (dd, J=13.64, 3.93Hz, 1H), 4.57 (d, J=13.64Hz, 1H), 5.38 - 5.48 (m, 1H), 7.41 (s, 1H), 7.65 (s, 1H) | 366 | - | |
| 60 | 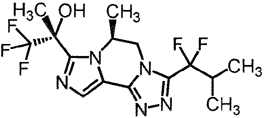 | (400 MHz, DMSO-D6) 1.08 - 1.13 (m, 6H), 1.23 (d, J=6.47Hz, 3H), 2.65 - 2.82 (m, 1H), 4.38 (dd, J=13.87, 3.93Hz, 1H), 4.56 (d, J=13.87Hz, 1H), 5.37 - 5.47 (m, 1H), 7.41 (s, 1H), 7.64 (s, 1H) | 380 | - | |
| 61 | 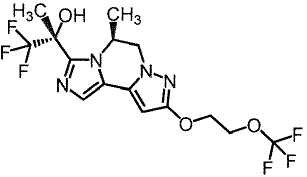 | (400 MHz, DMSO-D6) 1.27 (d, J=6.47Hz, 3H), 1.80 (s, 3H), 4.10 - 4.22 (m, 2H), 4.29 - 4.42 (m, 4H), 5.26 - 5.37 (m, 1H), 6.03 (s, 1H), 7.24 (s, 1H), 7.27 (s, 1H) | 415 | 459 (M-1+46) | |
| 62 | 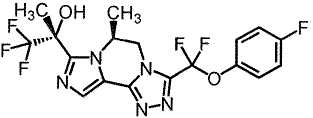 | (400 MHz, DMSO-D6) 1.23 (d, J=6.47Hz, 3H), 1.85 (s, 3H), 4.47 (dd, J=13.87, 3.93Hz, 1H), 4.68 (d, J=13.87Hz, 1H), 5.42 - 5.53 (m, 1H), 7.28 - 7.37 (m, 2H), 7.41 - 7.48 (m, 3H), 7.69 (s, 1H) | 448 | - | |
| 63 | 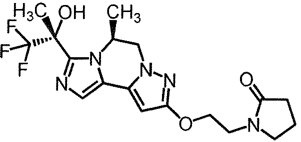 | (400 MHz, CDCl3) 1.37 (d, J=6.47Hz, 3H), 1.97 (s, 3H), 1.98 - 2.05 (m, 3H), 2.35 - 2.39 (m, 2H), 3.53 - 3.58 (m, 2H), 3.63 - 3.67 (m, 2H), 4.02 - 4.09 (m, 2H), 4.20 - 4.29 (m, 3H), 5.32 - 5.37 (m, 1H), 5.79 (s, 1H), 7.20 (s, 1H) | 414 | 412 | |

**[Table 1-15]**

| Example | Structure | ¹H-NMR | Mass M+1 | Mass M-1 | Remarks |
|---|---|---|---|---|---|
| 64 | 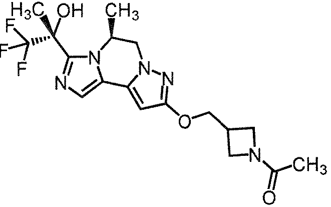 | (400 MHz, DMSO-D6) 1.27 (d, J=6.70Hz, 3H), 1.73 (s, 3H), 1.80 (s, 3H), 2.90 - 3.01 (m, 1H), 3.58 - 3.64 (m, 1H), 3.88 - 3.92 (m, 2H), 4.13 - 4.24 (m, 5H), 5.28 - 5.35 (m, 1H), 6.01 (s, 1H), 7.22 (s, 1H), 7.27 (s, 1H) | 414 | - | |
| 65 | 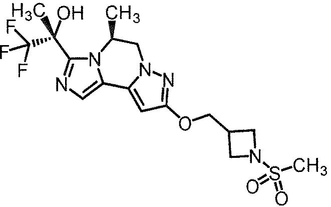 | (400 MHz, DMSO-D6) 1.28 (d, J=6.58Hz, 3H), 1.82 (s, 3H), 2.98 - 3.04 (m, 4H), 3.73 - 3.78 (m, 2H), 3.94 - 4.00 (m, 2H), 4.15 - 4.20 (m, 2H), 4.23 - 4.27 (m, 2H), 5.38 - 5.30 (m, 1H), 6.02 (s, 1H), 7.25 (s, 1H), 7.30 (s, 1H) | 450 | - | |
| 66 | 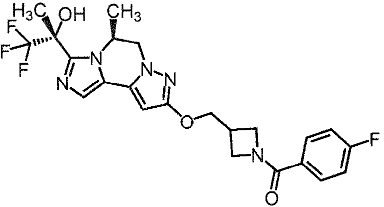 | (400 MHz, DMSO-D6) 1.26 (d, J=6.47Hz, 3H), 1.80 (s, 3H), 3.01 - 3.09 (m, 1H), 3.81 - 3.90 (m, 1H), 4.09 - 4.19 (m, 4H), 4.25 - 4.30 (m, 2H), 4.37 - 4.45 (m, 1H), 5.28 - 5.32 (m, 1H), 6.00 (s, 1H), 7.20 - 7.29 (m, 4H), 7.73 - 7.67 (m, 2H) | 494 | - | |

**[Table 1-16]**

| Example | Structure | ¹H-NMR | Mass M+1 | Mass M-1 | Remarks |
|---|---|---|---|---|---|
| 67 | 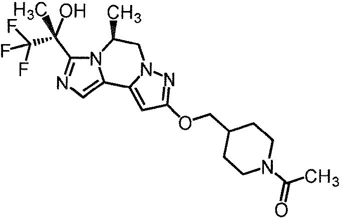 | (400 MHz, DMSO-D6) 1.01 - 1.27 (m, 5H), 1.71 - 1.78 (m, 5H), 1.93 - 2.02 (m, 4H), 2.51 - 2.55 (m, 1H), 2.98 - 3.05 (m, 1H), 3.78 - 3.86 (m, 1H), 3.92 - 3.94 (m, 2H), 4.10 - 4.18 (m, 2H), 4.33 - 4.42 (m, 1H), 5.28 - 5.33 (m, 1H), 5.98 (s, 1H), 7.21 (s, 1H), 7.27 (s, 1H) | 442 | - | |
| 68 | 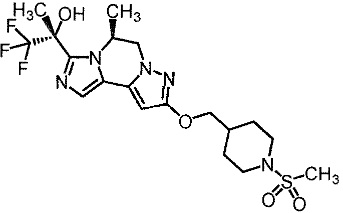 | (400 MHz, DMSO-D6) 1.21 - 1.35 (m, 5H), 1.78 - 1.87 (m, 6H), 2.66 - 2.73 (m, 2H), 2.83 (s, 3H), 3.53 - 3.60 (m, 2H), 3.96 (d, J=6.24Hz, 2H), 4.10 - 4.20 (m, 2H), 5.26 - 5.35 (m, 1H), 5.98 (s, 1H), 7.22 (s, 1H), 7.27 (s, 1H) | 478 | - | |
| 69 | 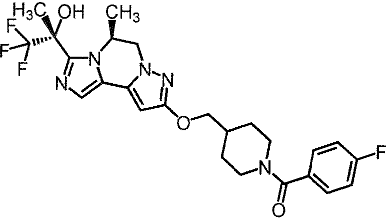 | (400 MHz, DMSO-D6) 1.20 - 1.30 (m, 5H), 1.75 - 1.83 (m, 5H), 1.98 - 2.06 (m, 1H), 2.81 - 3.04 (m, 2H), 3.50 - 3.67 (m, 1H), 3.96 (d, J=6.47Hz, 2H), 4.10 - 4.19 (m, 2H), 4.41 - 4.56 (m, 1H), 5.27 - 5.34 (m, 1H), 5.98 (s, 1H), 7.20 - 7.28 (m, 4H), 7.47 - 7.41 (m, 2H) | 522 | - | |

**[Table 1-17]**

| Example | Structure | ¹H-NMR | Mass M+1 | Mass M-1 | Remarks |
|---|---|---|---|---|---|
| 70 | 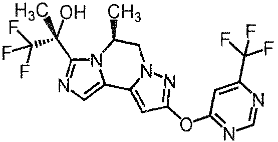 | (400 MHz, DMSO-D6) 1.31 (d, J=6.47Hz, 3H), 1.82 (s, 3H), 4.26 - 4.38 (m, 2H), 5.33 - 5.45 (m, 1H), 6.53 (s, 1H), 7.32 (s, 1H), 7.34 (s, 1H), 7.82 (s, 1H), 9.06 (s, 1H) | 449 | - | |
| 71 | 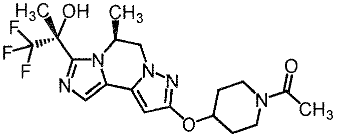 | (400 MHz, DMSO-D6) 1.27 (d, J=6.47Hz, 3H), 1.44 - 1.56 (m, 1H), 1.56 - 1.68 (m, 1H), 1.80 (s, 3H), 1.88 - 2.05 (m, 5H), 3.09 - 3.21 (m, 2H), 3.61 - 3.71 (m, 1H), 3.80 - 3.90 (m, 1H), 4.09 - 4.22 (m, 2H), 4.59 - 4.69 (m, 1H), 5.25 - 5.37 (m, 1H), 6.01 (s, 1H), 7.22 (s, 1H), 7.26 (s, 1H) | 428 | - | |
| 72 | 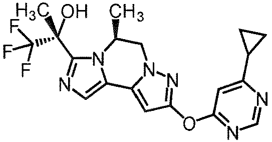 | (400 MHz, DMSO-D6) 0.98 - 1.08 (m, 4H), 1.31 (d, J=6.47Hz, 3H), 1.82 (s, 3H), 2.08 - 2.17 (m, 1H), 4.24 - 4.36 (m, 2H), 5.33 - 5.42 (m, 1H), 6.44 (s, 1H), 7.10 (d, J=0.92Hz, 1H), 7.31 (s, 1H), 7.32 (s, 1H), 8.61 (d, J=0.92Hz, 1H) | 421 | - | |
| 73 | 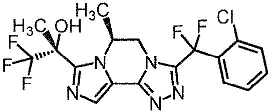 | (400 MHz, DMSO-D6) 1.28 (d, J=6.36Hz, 3H), 1.86 (s, 3H), 4.49 (dd, J=13.69, 3.91Hz, 1H), 4.68 (d, J=13.45Hz, 1H), 5.54 - 5.44 (m, 1H), 7.47 (s, 1H), 7.62 - 7.55 (m, 1H), 7.72 - 7.65 (m, 3H), 7.86 (d, J=7.34Hz, 1H) | 448 | - | |
| 74 | 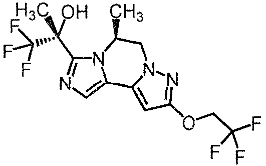 | (400 MHz, DMSO-D6) 1.28 (d, J=6.58Hz, 3H), 1.82 (s, 3H), 4.15 - 4.26 (m, 2H), 4.81 (q, J=8.97Hz, 2H), 5.29 - 5.40 (m, 1H), 6.15 (s, 1H), 7.28 (s, 1H), 7.30 (s, 1H) | 385 | 383 | |

**[Table 1-18]**

| Example | Structure | ¹ H-NMR | Mass M+1 | Mass M-1 | Remarks |
|---|---|---|---|---|---|
| 75 | 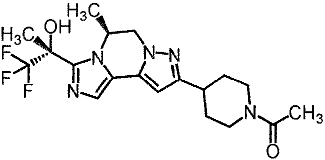 | (400 MHz, DMSO-D6) 1.43 - 1.55 (m, 3H), 1.88 - 1.92 (m, 2H), 2.01 (s, 3H), 2.67 - 2.70 (m, 1H), 2.84 - 2.87 (m, 1H), 3.13 - 3.16 (m, 1H), 3.84 (d, J=14.35Hz, 1H), 4.23 (dd, J=13.60, 4.04Hz, 1H), 4.34 - 4.37 (m, 2H), 5.34 - 5.35 (m, 1H), 6.40 (s, 1H), 7.23 (s, 1H), 7.26 (s, 1H) | 412 | - | |
| 76 | 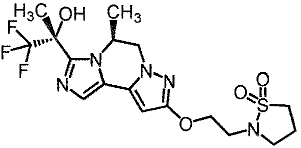 | (400 MHz, DMSO-D6) 1.28 (d, J=6.58Hz, 3H), 1.82 (s, 3H), 2.19 - 2.27 (m, 2H), 3.19 (t, J=7.62Hz, 2H), 3.28 - 3.33 (m, 4H), 4.13 - 4.18 (m, 2H), 4.23 (t, J=5.53Hz, 2H), 5.32 - 5.33 (m, 1H), 6.01 (s, 1H), 7.25 (s, 1H), 7.28 (s, 1H) | 450 | - | |
| 77 | 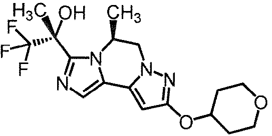 | (400 MHz, DMSO-D6) 1.28 (d, J=6.58Hz, 3H), 1.58 - 1.63 (m, 2H), 1.81 (s, 3H), 2.00 - 2.02 (m, 2H), 3.45 (tt, J=10.16, 2.29Hz, 2H), 3.84 - 3.85 (m, 2H), 4.12 - 4.20 (m, 2H), 4.59 - 4.65 (m, 1H), 5.31 - 5.32 (m, 1H), 6.01 (s, 1H), 7.23 (s, 1H), 7.27 (s, 1H) | 387 | - | |
| 78 | 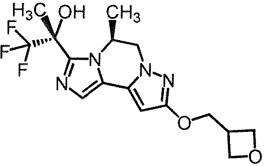 | (400 MHz, DMSO-D6) 1.29 (d, J=6.28Hz, 3H), 1.82 (s, 3H), 3.30 - 3.37 (m, 1H), 4.13 - 4.21 (m, 2H), 4.31 (d, J=6.58Hz, 2H), 4.41 (t, J=5.98Hz, 2H), 4.69 (dd, J=7.92, 6.13Hz, 2H), 5.32 - 5.34 (m, 1H), 6.01 (s, 1H), 7.24 (s, 1H), 7.28 (s, 1H) | 373 | - | |

**[Table 1-19]**

| Example | Structure | ¹ H-NMR | Mass M+1 | Mass M-1 | Remarks |
|---|---|---|---|---|---|
| 79 | 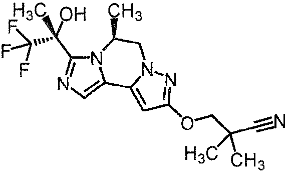 | (400 MHz, DMSO-D6) 1.29 (d, J=6.58Hz, 3H), 1.40 (s, 6H), 1.82 (s, 3H), 4.12 (s, 2H), 4.19 (s, 2H), 5.33 - 5.34 (m, 1H), 6.08 (s, 1H), 7.25 (s, 1H), 7.29 (s, 1H) | 384 | 382 | |
| 80 | 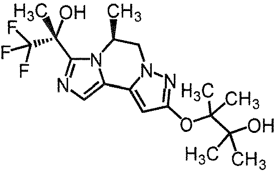 | (400 MHz, DMSO-D6) 1.19 (s, 6H), 1.25 (d, J=6.47Hz, 3H), 1.27 (s, 3H), 1.28 (s, 3H), 1.80 (s, 3H), 4.11 - 4.23 (m, 2H), 4.42 (s, 1H), 5.26 - 5.36 (m, 1H), 6.02 (s, 1H), 7.22 (s, 1H), 7.25 (s, 1 H) | 403 | 401 | |
| 81 | 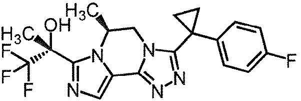 | (400 MHz, DMSO-D6) 1.06 (d, J=6.58Hz, 3H), 1.39 - 1.59 (m, 4H), 1.83 (s, 3H), 4.00 - 4.08 (m, 2H), 5.31 - 5.33 (m, 1H), 7.10 - 7.14 (m, 4H), 7.34 (s, 1H), 7.54 (s, 1H) | 422 | - | |
| 82 | 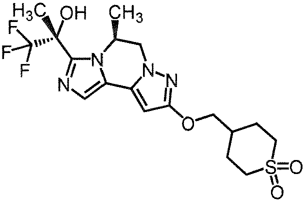 | (400 MHz, DMSO-D6) 1.27 (d, J=6.47Hz, 3H), 1.68 - 1.80 (m, 5H), 2.05 - 2.13 (m, 3H), 3.01 - 3.07 (m, 2H), 3.13 - 3.20 (m, 2H), 3.97 - 4.00 (m, 2H), 4.10 - 4.19 (m, 2H), 5.34 - 5.27 (m, 1H), 6.00 (s, 1H), 7.22 (s, 1H), 7.27 (s, 1H) | 449 | - | |

**[Table 1-20]**

| Example | Structure | ¹H-NMR | Mass M+1 | Mass M-1 | Remarks |
|---|---|---|---|---|---|
| 83 | 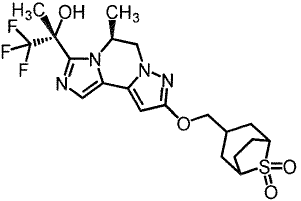 | (400 MHz, DMSO-D6) 1.26 (d, J=6.47Hz, 3H), 1.80 (s, 3H), 1.95 - 2.02 (m, 6H), 2.08 - 2.14 (m, 2H), 2.19 - 2.32 (m, 1H), 3.11 (s, 2H), 3.94 - 3.98 (m, 2H), 4.10 - 4.19 (m, 2H), 5.29 - 5.32 (m, 1H), 5.99 (s, 1H), 7.22 (s, 1H), 7.28 (s, 1H) | 475 | - | |
| 84 | 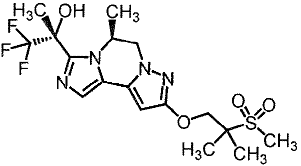 | (400 MHz, CDCl3) 1.39 (d, J=6.47Hz, 3H), 1.50 - 1.59 (m, 9H), 1.98 (s, 3H), 4.08 - 4.11 (m, 1H), 4.22 - 4.28 (m, 1H), 4.33 - 4.35 (m, 2H), 5.29 - 5.35 (m, 1H), 5.85 (s, 1H), 7.21 (s, 1H) | 437 | - | |
| 85 | 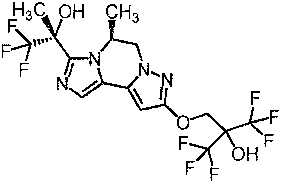 | (400 MHz, DMSO-D6) 1.27 (d, J=6.47Hz, 3H), 1.80 (s, 3H), 4.12 - 4.25 (m, 2H), 4.51 (s, 2H), 5.27 - 5.38 (m, 1H), 6.08 (s, 1H), 7.25 (s, 1H), 7.28 (s, 1H), 8.42 (s, 1H) | 483 | - | |
| 86 | 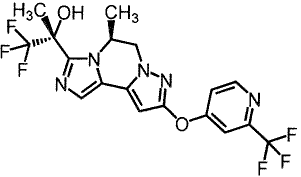 | (400 MHz, DMSO-D6) 1.32 (d, J=6.70Hz, 3H), 1.82 (s, 3H), 4.28 - 4.36 (m, 2H), 5.33 - 5.44 (m, 1H), 6.45 (s, 1H), 7.31 - 7.35 (m, 2H), 7.42 (dd, J=5.78, 2.31Hz, 1H), 7.63 (d, J=2.31 Hz, 1H), 8.67 (d, J=5.78Hz, 1H) | 448 | - | |

**[Table 1-21]**

| Example | Structure | ¹ H-NMR | Mass M+1 | Mass M-1 | Remarks |
|---|---|---|---|---|---|
| 87 | 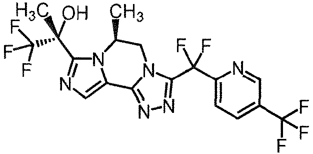 | (400 MHz, DMSO-D6) 1.26 (d, J=6.47Hz, 3H), 1.84 (s, 3H), 4.40 (dd, J=13.64, 3.93Hz, 1H), 4.63 (d, J=13.64Hz, 1H), 5.40 - 5.52 (m, 1H), 7.42 (s, 1H), 7.67 (s, 1H), 8.19 (d, J=8.44Hz, 1H), 8.54 (dd, J=8.44, 1.97Hz, 1H), 9.12 (s, 1H) | 483 | - | |
| 88 | 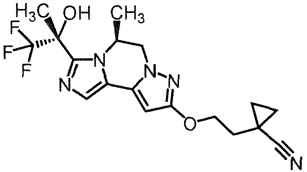 | (400 MHz, CDCl3) 0.94 (dd, J=7.03, 5.23Hz, 2H), 1.28 (dd, J=7.17, 5.08Hz, 2H), 1.41 (d, J=6.58Hz, 3H), 1.96 (t, J=6.43Hz, 2H), 1.99 (s, 3H), 3.24 (s, 1H), 4.11 (dd, J=11.06, 2.84Hz, 1H), 4.26 (dd, J=13.15, 3.89Hz, 1H), 4.39 (t, J=6.43Hz, 2H), 5.31 - 5.34 (m, 1H), 5.86 (s, 1H), 7.22 (s, 1H) | 396 | - | |
| 89 | 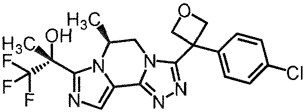 | (400 MHz, DMSO-D6) 0.99 (d, J=6.58Hz, 3H), 1.82 (s, 3H), 3.69 (d, J=12.56Hz, 1H), 4.00 (dd, J=13.90, 5.23Hz, 1H), 4.99 (d, J=6.28Hz, 1H), 5.10 (d, J=6.28Hz, 1H), 5.24 - 5.25 (m, 1H), 5.37 (dd, J=7.92, 6.13Hz, 2H), 7.36 - 7.39 (m, 3H), 7.46 - 7.49 (m, 2H), 7.60 (s, 1H) | 454 | - | |
| 90 | 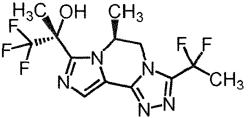 | (400 MHz, DMSO-D6) 1.23 (d, J=6.47Hz, 3H), 1.84 (s, 3H), 2.20 (t, J=19.54Hz, 3H), 4.38 (dd, J=13.87, 3.93Hz, 1H), 4.56 (d, J=13.87Hz, 1H), 5.39 - 5.50 (m, 1H), 7.41 (s, 1H), 7.66 (s, 1H) | 352 | - | |
| 91 | 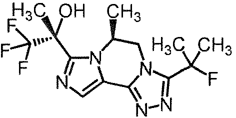 | (400 MHz, DMSO-D6) 1.22 (d, J=6.47Hz, 3H), 1.74 - 1.88 (m, 9H), 4.32 (dd, J=13.76, 3.81Hz, 1H), 4.61 (d, J=13.76Hz, 1H), 5.34 - 5.46 (m, 1H), 7.37 (s, 1H), 7.57 (s, 1H) | 348 | - | |

**[Table 1-22]**

| Example | Structure | ¹ H-NMR | Mass M+1 | Mass M-1 | Remarks |
|---|---|---|---|---|---|
| 92 | 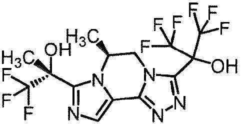 | (400 MHz, DMSO-D6) 1.19 - 1.22 (m, 3H), 1.85 (s, 3H), 4.24 - 4.26 (m, 1H), 4.86 (d, J=13.15Hz, 1H), 5.46 (s, 1H), 7.42 (s, 1H), 7.66 (s, 1H), 9.79 (brs, 1H) | 454 | - | |
| 93 | 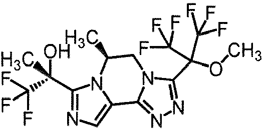 | (400 MHz, DMSO-D6) 1.25 (d, J=6.58Hz, 3H), 1.86 (s, 3H), 3.60 (s, 3H), 4.44 (dd, J=13.75, 3.59Hz, 1H), 4.66 (d, J=12.86Hz, 1H), 5.43 - 5.46 (m, 1H), 7.45 (s, 1H), 7.71 (s, 1H) | 468 | 466 | |
| 94 | 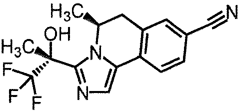 | (400 MHz, DMSO-D6) 1.12 (d, J=6.47Hz, 3H), 1.81 (s, 3H), 2.95 - 3.01 (m, 1H), 3.07 - 3.14 (m, 1H), 5.23 - 5.29 (m, 1H), 7.21 (s, 1H), 7.59 (s, 1H), 7.71 - 7.75 (m, 1H), 7.84 - 7.78 (m, 2H) | 322 | - | |
| 95 | 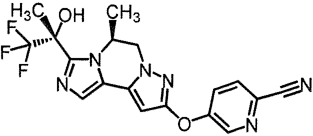 | (400 MHz, DMSO-D6) 1.31 (d, J=6.47Hz, 3H), 1.82 (s, 3H), 4.23 - 4.35 (m, 2H), 5.32 - 5.42 (m, 1H), 6.40 (s, 1H), 7.32 (s, 2H), 7.80 (dd, J=8.67, 2.89Hz, 1H), 8.07 (dd, J=8.67, 0.58Hz, 1H), 8.66 (dd, J=2.89, 0.58Hz, 1H) | 405 | 403 | |
| 96 | 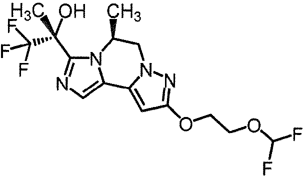 | (400 MHz, DMSO-D6) 1.27 (d, J=6.47Hz, 3H), 1.80 (s, 3H), 4.08 - 4.21 (m, 4H), 4.24 - 4.30 (m, 2H), 5.26 - 5.36 (m, 1H), 6.01 (s, 1H), 6.71 (t, J=75.83Hz, 1H), 7.23 (s, 1H), 7.26 (s, 1H) | 397 | 395 | |

**[Table 1-23]**

| Example | Structure | ¹ H-NMR | Mass M+1 | Mass M-1 | Remarks |
|---|---|---|---|---|---|
| 97 | 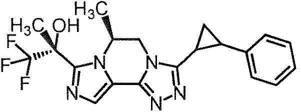 | (400 MHz, DMSO-D6) 1.23 (d, J=6.60Hz, 4H), 1.50 - 1.57 (m, 1H), 1.58 - 1.65 (m, 1H), 2.35 - 2.42 (m, 1H), 2.54 - 2.60 (m, 1H), 4.19 (dd, J=13.45, 3.91Hz, 1H), 4.43 (d, J=12.47Hz, 1H), 5.47 - 5.39 (m, 1H), 7.39 - 7.19 (m, 7H), 7.52 (s, 1H) | 404 | 402 | 3 |
| 98 | 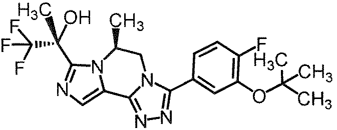 | (400 MHz, DMSO-D6) 1.18 (d, J=6.97Hz, 3H), 1.37 (s, 9H), 1.86 (s, 3H), 4.35 (d, J=12.72Hz, 1H), 4.56 (dd, J=13.57, 4.03Hz, 1H), 5.49 - 5.41 (m, 1H), 7.40 (s, 1H), 7.52 - 7.46 (m, 3H), 7.61 (s, 1H) | 454 | 452 | |
| 99 | 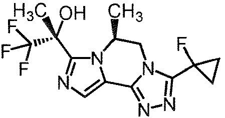 | (400 MHz, CDCl3) 1.37 - 1.65 (m, 7H), 2.03 (s, 3H), 4.14 (s, 1H), 4.26 - 4.32 (m, 1H), 4.49 - 4.56 (m, 1H), 5.49 - 5.55 (m, 1H), 7.74 (s, 1H) | 346 | - | |
| 100 | 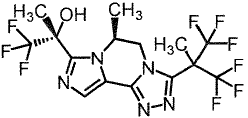 | (400 MHz, DMSO-D6) 1.19 (d, J=6.70Hz, 3H), 1.84 (s, 3H), 2.07 (s, 3H), 4.37 (dd, J=13.76, 3.58Hz, 1H), 4.70 (d, J=13.76Hz, 1H), 5.35 - 5.46 (m, 1H), 7.41 (s, 1H), 7.65 (s, 1H) | 452 | - | |
| 101 | 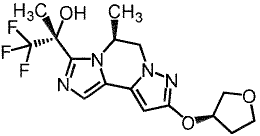 | (400 MHz, DMSO-D6) 1.28 (d, J=6.28Hz, 3H), 1.82 (s, 3H), 2.01 - 2.04 (m, 1H), 2.12 - 2.21 (m, 1H), 3.74 (td, J=8.37, 4.48Hz, 1H), 3.81 - 3.85 (m, 3H), 4.15 - 4.20 (m, 2H), 5.09 - 5.10 (m, 1H), 5.31 - 5.33 (m, 1H), 6.00 (s, 1H), 7.24 (s, 1H), 7.28 (s, 1H) | 373 | - | |

**[Table 1-24]**

| Example | Structure | ¹H-NMR | Mass M+1 | Mass M-1 | Remarks |
|---|---|---|---|---|---|
| 102 | 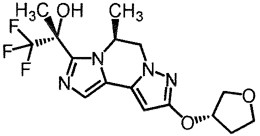 | (400 MHz, DMSO-D6) 1.28 (d, J=6.28Hz, 3H), 1.82 (s, 3H), 2.01 - 2.09 (m, 1H), 2.15 - 2.19 (m, 1H), 3.74 (td, J=8.30, 4.58Hz, 1H), 3.79 - 3.85 (m, 3H), 4.15 - 4.19 (m, 2H), 5.09 - 5.10 (m, 1H), 5.31 - 5.32 (m, 1H), 6.00 (s, 1H), 7.24 (s, 1H), 7.28 (s, 1H) | 373 | - | |
| 103 | 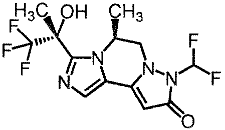 | (400 MHz, DMSO-D6) 1.57 (d, J=6.47Hz, 3H), 1.82 (s, 3H), 3.70 (dd, J=11.44, 3.35Hz, 1H), 3.98 (d, J=11.44Hz, 1H), 5.23 - 5.33 (m, 1H), 5.83 (d, J=1.62Hz, 1H), 7.40 (t, J=57.22Hz, 1H), 7.43 (s, 1H), 7.73 (s, 1H) | 353 | 351 | |

### Experimental Example 1

### (Inhibitory action of PDHK2 activity)

In the case of human PDHK2 (hPDHK2, NCBI Reference Database Accession number NM_002611.4), modified hPDHK2 cDNA wherein FLAG-Tag sequence was added to the N terminus of hPDHK2 cDNA clone (pReceiver-M01/PDK2-GeneCopoeia) as the base was prepared by polymerase chain reaction (PCR) and ligated to the NdeI/EcoRI site of pET-17b vector (Merck KGaA, Catalog Number 69663-3). The recombinant construct was transformed into Escherichia coli DH5α. The recombinant clones were identified, and plasmid DNA was isolated and subjected to the DNA sequence analysis. One clone which had the expected nucleic acid sequence was selected for expression work.

For expression of hPDHK2 activity, Escherichia coli strain BL21(DE3) cells (Merck KGaA, Catalog Number 69450-4) were transformed with the pET17b vector containing modified hPDHK2 cDNA. The Escherichia coli were grown to an optical density 0.6 (600 nmol/L) at 30°C. Protein expression was induced by the addition of 500 µmol/L isopropyl-β-thiogalactopyranoside. The Escherichia coli were cultured at 20°C for 17-18 hr and harvested by centrifugation. The harvested Escherichia coli was resuspended in a suspension buffer (20 mmol/L HEPES-NaOH, 500 mmol/L sodium chloride, 1% ethylene glycol, 0.1% Pluronic (registered trademark) F-68 (pH 8.0), complete, EDTA-free (Roche) (pH 8.0)), and disrupted by a microfluidizer M-110H (MIZUHO INDUSTRIAL CO., LTD.). The precipitate was removed by centrifugation and the supernatant was added to DDDDK-tagged Protein PURIFICATION GEL (MEDICAL & BIOLOGICAL LABORATORIES CO., LTD., Code No. 3329). DDDDK-tagged Protein PURIFICATION GEL was washed with a washing buffer (20 mmol/L HEPES-NaOH, 500 mmol/L sodium chloride, 1% ethylene glycol, 0.1% Pluronic F-68 (pH 8.0)) and the bound protein was eluted with elution buffer 1 (20 mmol/L HEPES-NaOH, 100 µg/mL peptide (amino acid sequence DYKDDDDK) (SEQ ID NO: 1), 500 mmol/L sodium chloride, 1% ethylene glycol, 0.1% Pluronic F-68 (pH 8.0)). The eluted fractions containing FLAG-Tagged protein were pooled, concentrated by an ultrafiltration method, added to a gel filtration column (HiLoad 26/60 Superdex 200 (GE Healthcare Life Sciences, Code No. 17-1070-01)), and eluted with elution buffer 2 (20 mmol/L HEPES-NaOH, 150 mmol/L sodium chloride, 0.5 mmol/L ethylenediaminetetraacetic acid (EDTA), 1% ethylene glycol, 0.1% Pluronic F-68 (pH 8.0)). The eluted fractions were pooled and preserved at -80°C.

PDH (porcine heart PDH complex, Sigma P7032) and 0.5 µg/mL hPDHK2 were mixed in an assay buffer (50 mmol/L 3-morpholinopropanesulfonic acid (pH 7.0), 20 mmol/L dipotassium hydrogen phosphate, 60 mmol/L potassium chloride, 2 mmol/L magnesium chloride, 0.4 mmol/L EDTA, 0.2% poloxamer, 2 mmol/L dithiothreitol) so that the final concentration of PDH was 0.025 U/mL, and the mixture was incubated at 4°C overnight to obtain a PDH/hPDHK2 complex solution.

PDH was mixed with the assay buffer so that the final concentration was 0.025 U/mL, and the mixture was incubated at 4°C overnight to prepare a PDH solution.

The test compounds were diluted with DMSO. To measure an inhibitory action of the test compound on the PDHK activity in the PDH/hPDHK2 complex solution, PDH/hPDHK2 complex solution (20 µL), test compound (1.5 µL) and 1.06 µmol/L ATP (diluted with assay buffer) (8.5 µL) were added to a 384 well microplate (Greiner Bio-One 781801) and PDHK reaction was performed at room temperature for 45 min (test compound well). DMSO (1.5 µL) was added to control wells instead of test compound. In addition, DMSO (1.5 µL) was added to blank wells instead of the test compound, and PDH solution was added instead of the PDH/hPDHK2 complex solution. To measure an inhibitory action of the test compound on the PDHK activity inherent in the PDH solution, a test compound was added and the PDH solution instead of the PDH/hPDHK2 complex solution was added to a blank + test compound well.

Then, 10 µL of substrates (5 mmol/L sodium pyruvate, 5 mmol/L Coenzyme A, 12 mmol/L NAD, 5 mmol/L thiamine pyrophosphate, diluted with assay buffer) were added. The mixture was incubated at room temperature for 90 min, and the residual PDH activity was measured.

The absorbance of each well at 340 nm was measured using a microplate reader to detect NADH produced by the PDH reaction. The PDH activity of each well was calculated from the changes in the absorbance before and after the PDH reaction. The PDH activity of the test compound-treated sample was calculated from the formula {PDH activity of test compound well - (PDH activity of blank + test compound well - PDH activity of blank well)}. The hPDHK2 inhibition rate (%) of the test compound was calculated from the formula [{ (PDH activity of the test compound-treated sample - PDH activity of control well)/ PDH activity of blank well - PDH activity of control well)} x 100]. IC₅₀ value was calculated according to a logistic regression method based on a test compound concentration and hPDHK2 inhibitory rate (%).

The results are shown in the following Tables. When IC₅₀ value could not be calculated, the inhibitory rate at the lowest or highest concentration of the test compound in the assay is shown. For example, the compound of Example 12 showed 35% hPDHK2 inhibitory rate at 0.1 µM.

**[Table 2-1]**

| Example No. | hPDHK2 IC₅₀ (µM) |
|---|---|
| 1 | 0.016 |
| 2 | 0.026 |
| 3 | 0.017 |
| 4 | 0.014 |
| 5 | 0.021 |
| 6 | 0.22 |
| 7 | 0.0075 |
| 8 | 0.0077 |
| 9 | 0.013 |
| 10 | 0.013 |
| 11 | 0.034 |
| 12 | >0.1 (35%) |
| 13 | 0.013 |
| 14 | 0.0063 |
| 15 | 0.014 |
| 16 | 0.012 |
| 17 | 0.0073 |
| 18 | 0.0065 |
| 19 | 0.011 |
| 20 | 0.022 |
| 21 | 0.0063 |
| 22 | 0.012 |
| 23 | 0.013 |
| 24 | >0.1 (31%) |
| 25 | 0.018 |
| 26 | 0.031 |
| 27 | 0.011 |
| 28 | 0.022 |
| 29 | 0.038 |
| 30 | 0.012 |

**[Table 2-2]**

| Example No. | hPDHK2 IC₅₀ (µM) |
|---|---|
| 31 | 0.081 |
| 32 | 0.0075 |
| 33 | 0.029 |
| 34 | 0.021 |
| 35 | 0.019 |
| 36 | 0.019 |
| 37 | 0.021 |
| 38 | 0.014 |
| 39 | 0.0081 |
| 40 | 0.011 |
| 41 | 0.021 |
| 42 | 0.024 |
| 43 | 0.020 |
| 44 | 0.017 |
| 45 | 0.0091 |
| 46 | 0.013 |
| 47 | 0.020 |
| 48 | 0.014 |
| 49 | 0.0088 |
| 50 | 0.027 |
| 51 | 0.017 |
| 52 | 0.021 |
| 53 | 0.013 |
| 54 | 0.0093 |
| 55 | 0.016 |
| 56 | 0.0070 |
| 57 | 0.0220 |
| 58 | 0.0088 |
| 59 | 0.010 |
| 60 | 0.0068 |

**[Table 2-3]**

| Example No. | hPDHK2 IC₅₀ (µM) |
|---|---|
| 61 | 0.014 |
| 62 | 0.0063 |
| 63 | 0.014 |
| 64 | 0.019 |
| 65 | 0.013 |
| 66 | 0.018 |
| 67 | 0.019 |
| 68 | 0.014 |
| 69 | 0.015 |
| 70 | 0.019 |
| 71 | 0.026 |
| 72 | 0.012 |
| 73 | 0.0074 |
| 74 | 0.012 |
| 75 | 0.019 |
| 76 | 0.013 |
| 77 | 0.017 |
| 78 | 0.017 |
| 79 | 0.015 |
| 80 | 0.013 |
| 81 | 0.0073 |
| 82 | 0.023 |
| 83 | 0.016 |
| 84 | 0.042 |
| 85 | 0.023 |
| 86 | 0.016 |
| 87 | 0.0087 |
| 88 | 0.017 |
| 89 | 0.0088 |
| 90 | 0.015 |

**[Table 2-4]**

| Example No. | hPDHK2 IC₅₀ (µM) |
|---|---|
| 91 | 0.011 |
| 92 | 0.016 |
| 93 | 0.021 |
| 94 | 0.0086 |
| 95 | 0.018 |
| 96 | 0.013 |
| 97 | 0.0062 |
| 98 | 0.012 |
| 99 | 0.0085 |
| 100 | 0.0095 |
| 101 | 0.0140 |
| 102 | 0.0160 |
| 103 | 0.026 |

As Formulation Examples of the present invention, the following preparations can be mentioned. However, the present invention is not limited by these Formulation Examples.

### Formulation Example 1: Production of capsule

| | |
|---|---|
| 1) compound of Example 1 | 30 mg |
| 2) crystalline cellulose | 10 mg |
| 3) lactose | 19 mg |
| 4) magnesium stearate | 1 mg |

1), 2), 3) and 4) are mixed and filled in a gelatin capsule.

### Formulation Example 2: Production of tablet

| | |
|---|---|
| 1) compound of Example 1 | 10 g |
| 2) lactose | 50 g |
| 3) cornstarch | 15 g |
| 4) carmellose calcium | 44 g |
| 5) magnesium stearate | 1 g |

The total amount of 1), 2), 3) and 30 g of 4) are kneaded with water, vacuum dried, and sieved. The sieved powder is mixed with 14 g of 4) and 1 g of 5), and the mixture is punched by a tableting machine. In this way, 1000 tablets each containing 10 mg of the compound of Example 1 per tablet are obtained.

### Formulation Example 3: Production of injection

| | |
|---|---|
| 1) compound of Example 1 | 5 mg |
| 2) D-mannitol | 5 g |
| 3) distilled water | 100 mL |

1) and 2) are dissolved in 3) and the solution is filled in a container for injection, sealed and sterilized.

### Industrial Applicability

Since the compound of the formula [I-a] or a pharmaceutically acceptable salt thereof has a PDHK inhibitory activity, it is useful as an active ingredient of a medicament for the treatment or prophylaxis of diabetes (type 1 diabetes, type 2 diabetes etc.), insulin resistance syndrome, metabolic syndrome, hyperglycemia, hyperlactacidemia, diabetic complications (diabetic neuropathy, diabetic retinopathy, diabetic nephropathy, cataract etc.), cardiac failure (acute cardiac failure, chronic cardiac failure), cardiomyopathy, myocardial ischemia, myocardial infarction, angina pectoris, dyslipidemia, atherosclerosis, peripheral arterial disease, intermittent claudication, chronic obstructive pulmonary disease, brain ischemia, cerebral apoplexy, mitochondrial disease, mitochondrial encephalomyopathy, cancer, pulmonary hypertension, Alzheimer disease, vascular dementia (large-vessel type or small-vessel type vascular dementia), glaucoma, diabetic retinopathy, retinopathy of prematurity, retinal vein occlusion, ischemic optic neuropathy, or chronic kidney disease.
The present invention is summarized by the followig embodiments:
1. A compound of the formula [I-a], or a pharmaceutically acceptable salt thereof: 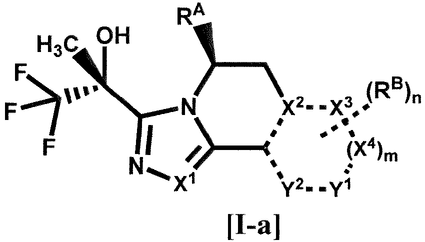 wherein
   a bond in a dotted line is a single bond or a double bond, X¹, X², X³ and X⁴ are each independently C or N, Y¹ and Y² are each independently C, N or O (wherein the total number of N and O for X², X³, X⁴, Y¹ or Y² is 0 to 3),
   R^{A} is C₁₋₄ alkyl,
   R^{B} is
      (1) halogen,
      (2) cyano,
      (3) hydroxy,
      (4) oxo,
      (5) -COR¹ {wherein R¹ is
         (A) hydrogen,
         (B) -OH,
         (C) -NR²R³ (wherein R² and R³ are each independently hydrogen or C₁₋₄ alkyl), or
         (D) a 4- to 6-membered saturated heterocyclyl having one nitrogen atom (wherein the saturated heterocyclyl is optionally substituted by 1 or 2 halogens)},
      (6) C₁₋₈ alkyl {wherein the C₁₋₈ alkyl is optionally substituted by 1 to 8 substituents independently selected from the group consisting of
         (A) halogen,
         (B) hydroxy,
         (C) phenyl optionally substituted by halogen,
         (D) pyridyl optionally substituted by haloC₁₋₄ alkyl, and
         (E) -OR⁴ (wherein R⁴ is
            (a) C₁₋₄ alkyl,
            (b) phenyl optionally substituted by halogen, or
            (c) benzyl optionally substituted by C₁₋₄ alkoxy)},
      (7) C₁₋₈ alkoxy {wherein the C₁₋₈ alkoxy is optionally substituted by 1 to 8 substituents independently selected from the group consisting of
         (A) halogen,
         (B) cyano,
         (C) hydroxy,
         (D) C₁₋₄ alkoxy optionally substituted by 1 to 3 halogens,
         (E) C₁₋₉ alkylsulfonyl,
         (F) C₃₋₆ cycloalkyl optionally substituted by one substituent selected from the group consisting of cyano and cyano C₁₋₄ alkyl,
         (G) phenyl optionally substituted by cyano,
         (H) -COCy¹ (wherein Cy¹ is a 4- to 6-membered saturated heterocyclyl having one nitrogen atom, and the saturated heterocyclyl is optionally substituted by 1 or 2 halogens), and
         (I) a 4- to 6-membered saturated heterocyclyl having 1 or 2 hetero atoms independently selected from a nitrogen atom, an oxygen atom and a sulfur atom (wherein the saturated heterocyclyl is optionally substituted by 1 to 4 substituents independently selected from
            (a) C₁₋₄ alkyl,
            (b) oxo,
            (c) C₁₋₄ alkylcarbonyl,
            (d) benzoyl optionally substituted by halogen, and
            (e) C₁₋₄ alkylsulfonyl,
         when the saturated heterocyclyl is substituted by two C₁₋₄ alkyls, the two C₁₋₄ alkyls are optionally bonded to each other to form a bridged ring together with the atoms bonded thereto) },
      (8) -Cy² {wherein Cy² is
         (A) C₃₋₆ cycloalkyl (wherein the C₃₋₆ cycloalkyl is optionally substituted by 1 or 2 substituents independently selected from
            (a) halogen,
            (b) C₁₋₄ alkyl,
            (c) haloC₁₋₄ alkyl, and
            (d) phenyl optionally substituted by halogen),
         (B) phenyl optionally substituted by 1 or 2 substituents independently selected from the group consisting of halogen, haloC₁₋₄ alkyl, and C₁₋₄ alkoxy, or
         (C) a 4- to 6-membered saturated heterocyclyl having one nitrogen atom or oxygen atom (wherein the saturated heterocyclyl is optionally substituted by one substituent selected from the group consisting of (a) phenyl optionally substituted by halogen and (b) C₁₋₄ alkylcarbonyl)}, or
      (9) -OCy³ {wherein Cy³ is
         (A) a 4- to 6-membered saturated heterocyclyl having one nitrogen atom or oxygen atom (wherein the saturated heterocyclyl is optionally substituted by one substituent selected from the group consisting of (a) benzoyl optionally substituted by halogen and (b) C₁₋₄ alkylcarbonyl), or
         (B) a 6-membered heteroaryl having 1 or 2 nitrogen atoms (wherein the heteroaryl is optionally substituted by 1 or 2 substituents independently selected from cyano, haloC₁₋₄ alkyl, and C₃₋₆ cycloalkyl) },
   m is 0 or 1, and
   n is 0, 1 or 2, when n is 2, each R^{B} may be the same or different.
2. The compound according to **item 1** or a pharmaceutically acceptable salt thereof, which is a compound of the formula [I-b] : 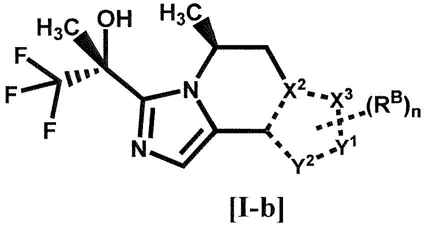 wherein each symbol is as defined in **item 1,** or a pharmaceutically acceptable salt thereof.
3. The compound according to **item 1 or 2** or a pharmaceutically acceptable salt thereof, which is a compound of the formula [I-c]: 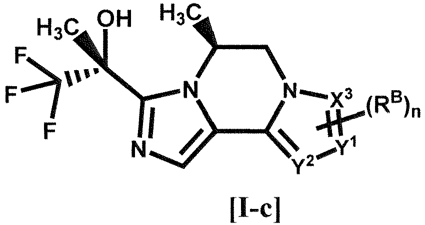 wherein each symbol is as defined in **item 1,** or a pharmaceutically acceptable salt thereof.
4. The compound according to any one of **items 1 to 3,** wherein n is 1, or a pharmaceutically acceptable salt thereof.
5. The compound according to any one of **items 1 to 4** or a pharmaceutically acceptable salt thereof, which is a compound of the formula [I-d]: 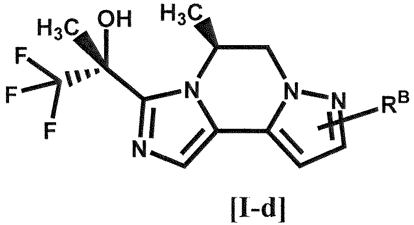 wherein the symbol is as defined in **item 1,** or a pharmaceutically acceptable salt thereof.
6. The compound according to any one of **items 1 to 4** or a pharmaceutically acceptable salt thereof, which is a compound of the formula [I-e]: 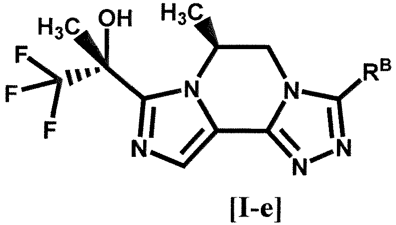 wherein the symbol is as defined in **item 1,** or a pharmaceutically acceptable salt thereof.
7. The compound according to any one of **items 1 to 6** or a pharmaceutically acceptable salt thereof, wherein R^{B} is
   (1) C₁₋₈ alkyl {wherein the C₁₋₈ alkyl is optionally substituted by 1 to 8 substituents independently selected from the group consisting of
      (A) halogen,
      (B) hydroxy,
      (C) phenyl optionally substituted by halogen,
      (D) pyridyl optionally substituted by haloC₁₋₄ alkyl, and
      (E) -OR⁴ (wherein R⁴ is
         (a) C₁₋₄ alkyl,
         (b) phenyl optionally substituted by halogen, or
         (c) benzyl optionally substituted by C₁₋₄ alkoxy)}, or
   (2) C₁₋₈ alkoxy {wherein the C₁₋₈ alkoxy is optionally substituted by 1 to 8 substituents independently selected from the group consisting of
      (A) halogen,
      (B) cyano,
      (C) hydroxy,
      (D) C₁₋₄ alkoxy optionally substituted by 1 to 3 halogens,
      (E) C₁₋₄ alkylsulfonyl,
      (F) C₃₋₆ cycloalkyl optionally substituted by one substituent selected from the group consisting of cyano and cyano C₁₋₄ alkyl,
      (G) phenyl optionally substituted by cyano,
      (H) -COCy¹ (wherein Cy¹ is a 4- to 6-membered saturated heterocyclyl having one nitrogen atom, and the saturated heterocyclyl is optionally substituted by 1 or 2 halogens), and
      (I) a 4- to 6-membered saturated heterocyclyl having 1 or 2 hetero atoms independently selected from a nitrogen atom, an oxygen atom and a sulfur atom (wherein the saturated heterocyclyl is optionally substituted by 1 to 4 substituents independently selected from
         (a) C₁₋₄ alkyl,
         (b) oxo,
         (c) C₁₋₄ alkylcarbonyl,
         (d) benzoyl optionally substituted by halogen, and
         (e) C₁₋₄ alkylsulfonyl,
   when the saturated heterocyclyl is substituted by two C₁₋₄ alkyls, the two C₁₋₄ alkyls are optionally bonded to each other to form a bridged ring together with the atoms bonded thereto)}.
8. A compound selected from the following formulas: 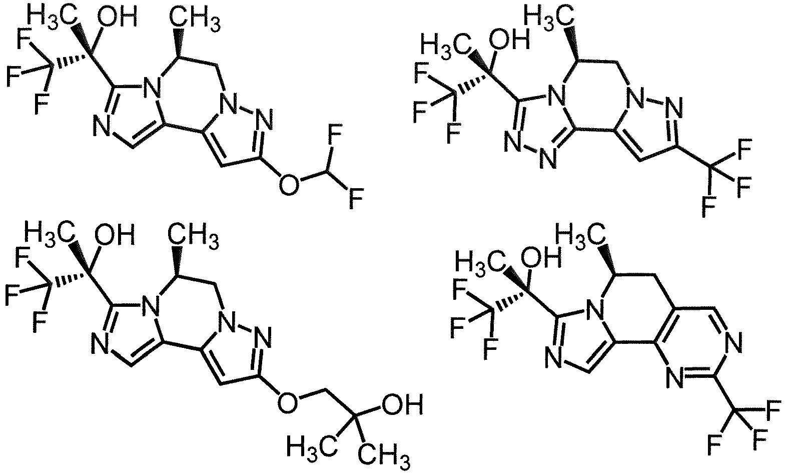 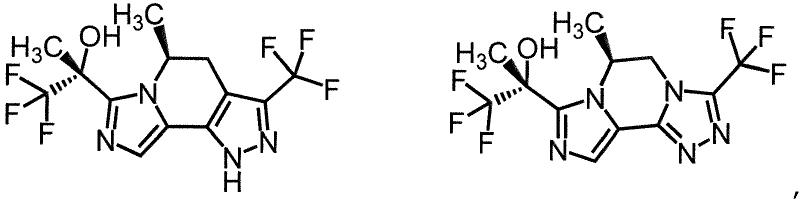 or a pharmaceutically acceptable salt thereof.
9. A pharmaceutical composition comprising the compound according to any one of **items 1 to 8** or a pharmaceutically acceptable salt thereof, and a pharmaceutically acceptable carrier.
10. A PDHK inhibitor comprising the compound according to any one of **items 1 to 8** or a pharmaceutically acceptable salt thereof.
11. A PDHK2 inhibitor comprising the compound according to any one of **items 1 to 8** or a pharmaceutically acceptable salt thereof.
12. An agent for the treatment or prophylaxis of diabetes, insulin resistance syndrome, metabolic syndrome, hyperglycemia, hyperlactacidemia, diabetic complication, cardiac failure, cardiomyopathy, myocardial ischemia, myocardial infarction, angina pectoris, dyslipidemia, atherosclerosis, peripheral arterial disease, intermittent claudication, chronic obstructive pulmonary disease, brain ischemia, cerebral apoplexy, mitochondrial disease, mitochondrial encephalomyopathy, cancer, pulmonary hypertension, Alzheimer disease, vascular dementia, glaucoma, diabetic retinopathy, retinopathy of prematurity, retinal vein occlusion, ischemic optic neuropathy or chronic kidney disease, the agent comprising the compound according to any one of **items 1 to 8** or a pharmaceutically acceptable salt thereof.
13. The agent according to **item 12,** wherein the diabetes is type 1 diabetes or type 2 diabetes.
14. The agent according to **item 12,** wherein the vascular dementia is a large-vessel type of vascular dementia or a small-vessel type of vascular dementia.
15. The agent according to **item 12,** wherein the cardiac failure is acute cardiac failure or chronic cardiac failure.
16. The agent according to **item 12,** wherein the pulmonary hypertension is pulmonary arterial hypertension.
17. A method for inhibiting PDHK, comprising administering a therapeutically effective amount of the compound according to any one of **items 1 to 8** or a pharmaceutically acceptable salt thereof to a mammal.
18. A method for treating or preventing a disease selected from the group consisting of diabetes, insulin resistance syndrome, metabolic syndrome, hyperglycemia, hyperlactacidemia, diabetic complication, cardiac failure, cardiomyopathy, myocardial ischemia, myocardial infarction, angina pectoris, dyslipidemia, atherosclerosis, peripheral arterial disease, intermittent claudication, chronic obstructive pulmonary disease, brain ischemia, cerebral apoplexy, mitochondrial disease, mitochondrial encephalomyopathy, cancer, pulmonary hypertension, Alzheimer disease, vascular dementia, glaucoma, diabetic retinopathy, retinopathy of prematurity, retinal vein occlusion, ischemic optic neuropathy and chronic kidney disease, the method comprising administering a therapeutically effective amount of the compound according to any one of **items 1 to 8** or a pharmaceutically acceptable salt thereof to a mammal.
19. The method according to **item 18,** wherein the diabetes is type 1 diabetes or type 2 diabetes.
20. The method according to **item 18,** wherein the vascular dementia is a large-vessel type of vascular dementia or a small-vessel type of vascular dementia.
21. The method according to **item 18,** wherein the cardiac failure is acute cardiac failure or chronic cardiac failure.
22. The method according to **item 18,** wherein the pulmonary hypertension is pulmonary arterial hypertension.
23. Use of the compound according to any one of **items 1 to 8** or a pharmaceutically acceptable salt thereof in the manufacture of a PDHK inhibitor.
24. Use of the compound according to any one of **items 1 to 8** or a pharmaceutically acceptable salt thereof in the manufacture of an agent for the treatment or prophylaxis of a disease selected from the group consisting of diabetes, insulin resistance syndrome, metabolic syndrome, hyperglycemia, hyperlactacidemia, diabetic complication, cardiac failure, cardiomyopathy, myocardial ischemia, myocardial infarction, angina pectoris, dyslipidemia, atherosclerosis, peripheral arterial disease, intermittent claudication, chronic obstructive pulmonary disease, brain ischemia, cerebral apoplexy, mitochondrial disease, mitochondrial encephalomyopathy, cancer, pulmonary hypertension, Alzheimer disease, vascular dementia, glaucoma, diabetic retinopathy, retinopathy of prematurity, retinal vein occlusion, ischemic optic neuropathy and chronic kidney disease.
25. The use according to **item 24,** wherein the diabetes is type 1 diabetes or type 2 diabetes.
26. The use according to **item 24,** wherein the vascular dementia is of a large-vessel type of vascular dementia or a small-vessel type of vascular dementia.
27. The use according to **item 24** wherein the cardiac failure is acute cardiac failure or chronic cardiac failure.
28. The use according to **item 24** wherein the pulmonary hypertension is pulmonary arterial hypertension.
29. The compound according to any one of **items 1 to 8** or a pharmaceutically acceptable salt thereof for use in the treatment or prophylaxis of a disease selected from the group consisting of diabetes, insulin resistance syndrome, metabolic syndrome, hyperglycemia, hyperlactacidemia, diabetic complication, cardiac failure, cardiomyopathy, myocardial ischemia, myocardial infarction, angina pectoris, dyslipidemia, atherosclerosis, peripheral arterial disease, intermittent claudication, chronic obstructive pulmonary disease, brain ischemia, cerebral apoplexy, mitochondrial disease, mitochondrial encephalomyopathy, cancer, pulmonary hypertension, Alzheimer disease, vascular dementia, glaucoma, diabetic retinopathy, retinopathy of prematurity, retinal vein occlusion, ischemic optic neuropathy and chronic kidney disease.
30. The compound according to **item 29** or a pharmaceutically acceptable salt thereof, wherein the diabetes is type 1 diabetes or type 2 diabetes.
31. The compound according to **item 29** or a pharmaceutically acceptable salt thereof, wherein the vascular dementia is a large-vessel type of vascular dementia or a small-vessel type of vascular dementia.
32. The compound according to **item 29** or a pharmaceutically acceptable salt thereof, wherein the cardiac failure is acute cardiac failure or chronic cardiac failure.
33. The compound according to **item 29** or a pharmaceutically acceptable salt thereof, wherein the pulmonary hypertension is pulmonary arterial hypertension.

## Claims

1. A compound of the formula [I-a], or a pharmaceutically acceptable salt thereof: 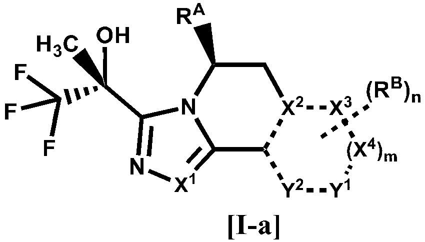 wherein
a bond in a dotted line is a single bond or a double bond,
X¹, X², X³ and X⁴ are each independently C or N, Y¹ and Y² are each independently C, N or O (wherein the total number of N and O for X², X³, X⁴, Y¹ or Y² is 0 to 3),
R^{A} is C₁₋₄ alkyl,
R^{B} is
(1) halogen,
(2) cyano,
(3) hydroxy,
(4) oxo,
(5) -COR¹ {wherein R¹ is
(A) hydrogen,
(B) -OH,
(C) -NR²R³ (wherein R² and R³ are each independently hydrogen or C₁₋₄ alkyl), or
(D) a 4- to 6-membered saturated heterocyclyl having one nitrogen atom (wherein the saturated heterocyclyl is optionally substituted by 1 or 2 halogens)},
(6) C₁₋₈ alkyl {wherein the C₁₋₈ alkyl is optionally substituted by 1 to 8 substituents independently selected from the group consisting of
(A) halogen,
(B) hydroxy,
(C) phenyl optionally substituted by halogen,
(D) pyridyl optionally substituted by haloC₁₋₄ alkyl, and
(E) -OR⁴ (wherein R⁴ is
(a) C₁₋₄ alkyl,
(b) phenyl optionally substituted by halogen, or
(c) benzyl optionally substituted by C₁₋₄ alkoxy)},
(7) C₁-₈ alkoxy {wherein the C₁₋₈ alkoxy is optionally substituted by 1 to 8 substituents independently selected from the group consisting of
(A) halogen,
(B) cyano,
(C) hydroxy,
(D) C₁₋₄ alkoxy optionally substituted by 1 to 3 halogens,
(E) C₁₋₄ alkylsulfonyl,
(F) C₃₋₆ cycloalkyl optionally substituted by one substituent selected from the group consisting of cyano and cyano C₁₋₄ alkyl,
(G) phenyl optionally substituted by cyano,
(H) -COCy¹ (wherein Cy¹ is a 4- to 6-membered saturated heterocyclyl having one nitrogen atom, and the saturated heterocyclyl is optionally substituted by 1 or 2 halogens), and
(I) a 4- to 6-membered saturated heterocyclyl having 1 or 2 hetero atoms independently selected from a nitrogen atom, an oxygen atom and a sulfur atom (wherein the saturated heterocyclyl is optionally substituted by 1 to 4 substituents independently selected from
(a) C₁₋₄ alkyl,
(b) oxo,
(c) C₁₋₄ alkylcarbonyl,
(d) benzoyl optionally substituted by halogen, and
(e) C₁₋₄ alkylsulfonyl,
when the saturated heterocyclyl is substituted by two C₁₋₄ alkyls, the two C₁₋₄ alkyls are optionally bonded to each other to form a bridged ring together with the atoms bonded thereto)},
(8) -Cy² {wherein Cy² is
(A) C₃₋₆ cycloalkyl (wherein the C₃₋₆ cycloalkyl is optionally substituted by 1 or 2 substituents independently selected from
(a) halogen,
(b) C₁₋₄ alkyl,
(c) haloC₁₋₄ alkyl, and
(d) phenyl optionally substituted by halogen),
(B) phenyl optionally substituted by 1 or 2 substituents independently selected from the group consisting of halogen, haloC₁₋₄ alkyl, and C₁₋₄ alkoxy, or
(C) a 4- to 6-membered saturated heterocyclyl having one nitrogen atom or oxygen atom (wherein the saturated heterocyclyl is optionally substituted by one substituent selected from the group consisting of (a) phenyl optionally substituted by halogen and (b) C₁₋₄ alkylcarbonyl)}, or
(9) -OCy³ {wherein Cy³ is
(A) a 4- to 6-membered saturated heterocyclyl having one nitrogen atom or oxygen atom (wherein the saturated heterocyclyl is optionally substituted by one substituent selected from the group consisting of (a) benzoyl optionally substituted by halogen and (b) C₁₋₄ alkylcarbonyl), or
(B) a 6-membered heteroaryl having 1 or 2 nitrogen atoms (wherein the heteroaryl is optionally substituted by 1 or 2 substituents independently selected from cyano, haloC₁₋₄ alkyl, and C₃₋₆ cycloalkyl)},
m is 0 or 1, and
n is 0, 1 or 2, when n is 2, each R^{B} may be the same or different.

2. The compound according to claim 1 or a pharmaceutically acceptable salt thereof, which is a compound of the formula [I-b]: 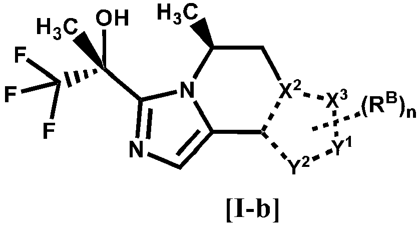 wherein each symbol is as defined in claim 1, or a pharmaceutically acceptable salt thereof.

3. The compound according to claim 1 or 2 or a pharmaceutically acceptable salt thereof, which is a compound of the formula [I-c]: 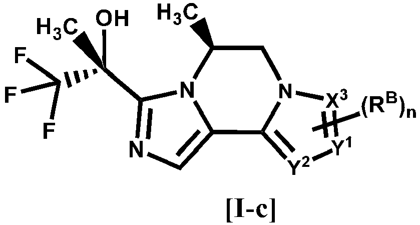 wherein each symbol is as defined in claim 1, or a pharmaceutically acceptable salt thereof.

4. The compound according to any one of claims 1 to 3, wherein n is 1, or a pharmaceutically acceptable salt thereof.

5. The compound according to any one of claims 1 to 4 or a pharmaceutically acceptable salt thereof, which is a compound of the formula [I-d]: 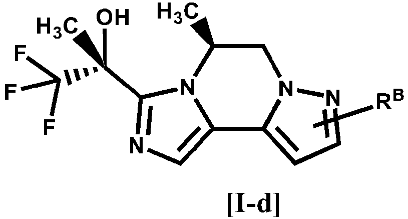 wherein the symbol is as defined in claim 1, or a pharmaceutically acceptable salt thereof.

6. The compound according to any one of claims 1 to 4 or a pharmaceutically acceptable salt thereof, which is a compound of the formula [I-e]: 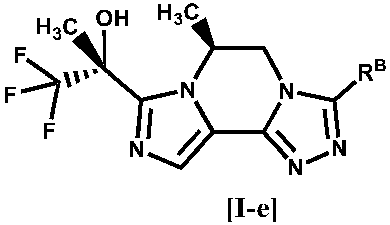 wherein the symbol is as defined in claim 1, or a pharmaceutically acceptable salt thereof.

7. The compound according to any one of claims 1 to 6 or a pharmaceutically acceptable salt thereof, wherein R^{B} is
(1) C₁-₈ alkyl {wherein the C₁₋₈ alkyl is optionally substituted by 1 to 8 substituents independently selected from the group consisting of
(A) halogen,
(B) hydroxy,
(C) phenyl optionally substituted by halogen,
(D) pyridyl optionally substituted by haloC₁₋₄ alkyl, and
(E) -OR⁴ (wherein R⁴ is
(a) C₁₋₄ alkyl,
(b) phenyl optionally substituted by halogen, or
(c) benzyl optionally substituted by C₁₋₄ alkoxy)}, or
(2) C₁-₈ alkoxy {wherein the C₁₋₈ alkoxy is optionally substituted by 1 to 8 substituents independently selected from the group consisting of
(A) halogen,
(B) cyano,
(C) hydroxy,
(D) C₁₋₄ alkoxy optionally substituted by 1 to 3 halogens,
(E) C₁₋₄ alkylsulfonyl,
(F) C₃₋₆ cycloalkyl optionally substituted by one substituent selected from the group consisting of cyano and cyano C₁₋₄ alkyl,
(G) phenyl optionally substituted by cyano,
(H) -COCy¹ (wherein Cy¹ is a 4- to 6-membered saturated heterocyclyl having one nitrogen atom, and the saturated heterocyclyl is optionally substituted by 1 or 2 halogens), and
(I) a 4- to 6-membered saturated heterocyclyl having 1 or 2 hetero atoms independently selected from a nitrogen atom, an oxygen atom and a sulfur atom (wherein the saturated heterocyclyl is optionally substituted by 1 to 4 substituents independently selected from
(a) C₁₋₄ alkyl,
(b) oxo,
(c) C₁₋₄ alkylcarbonyl,
(d) benzoyl optionally substituted by halogen, and
(e) C₁₋₄ alkylsulfonyl,
when the saturated heterocyclyl is substituted by two C₁₋₄ alkyls, the two C₁₋₄ alkyls are optionally bonded to each other to form a bridged ring together with the atoms bonded thereto)}.

8. A compound selected from the following formulas: 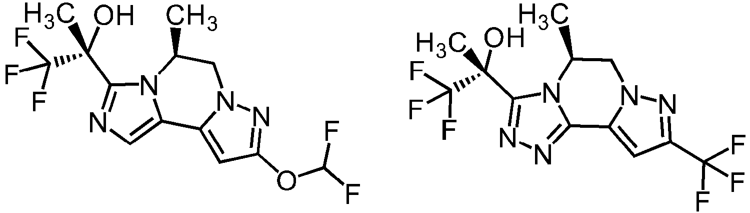 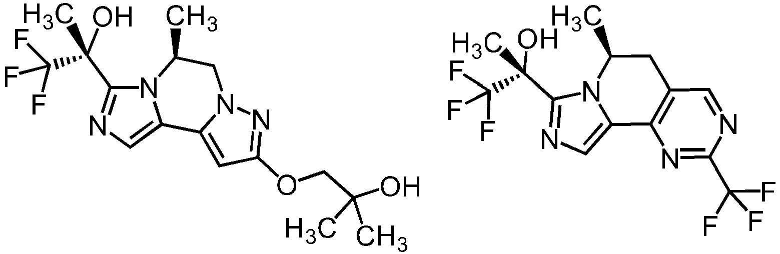 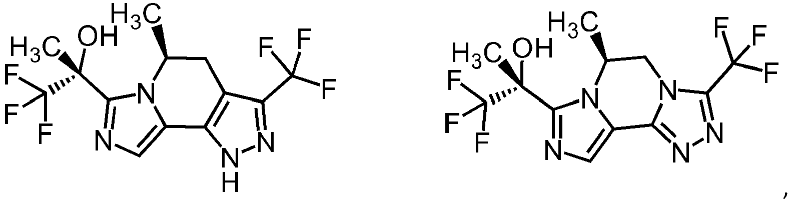 or a pharmaceutically acceptable salt thereof.

9. A pharmaceutical composition comprising the compound according to any one of claims 1 to 8 or a pharmaceutically acceptable salt thereof, and a pharmaceutically acceptable carrier.

10. The compound according to any one of claims 1 to 8 or a pharmaceutically acceptable salt thereof, for use as a PDHK inhibitor.

11. The compound according to any one of claims 1 to 8 or a pharmaceutically acceptable salt thereof, for use as a PDHK2 inhibitor.

12. An agent for use in the treatment or prophylaxis of diabetes, insulin resistance syndrome, metabolic syndrome, hyperglycemia, hyperlactacidemia, diabetic complication, cardiac failure, cardiomyopathy, myocardial ischemia, myocardial infarction, angina pectoris, dyslipidemia, atherosclerosis, peripheral arterial disease, intermittent claudication, chronic obstructive pulmonary disease, brain ischemia, cerebral apoplexy, mitochondrial disease, mitochondrial encephalomyopathy, cancer, pulmonary hypertension, Alzheimer disease, vascular dementia, glaucoma, diabetic retinopathy, retinopathy of prematurity, retinal vein occlusion, ischemic optic neuropathy or chronic kidney disease, the agent comprising the compound according to any one of claims 1 to 8 or a pharmaceutically acceptable salt thereof.

13. The agent for use according to claim 12, wherein the diabetes is type 1 diabetes or type 2 diabetes; or wherein the vascular dementia is a large-vessel type of vascular dementia or a small-vessel type of vascular dementia.

14. The agent for use according to claim 12, wherein the cardiac failure is acute cardiac failure or chronic cardiac failure; or wherein the pulmonary hypertension is pulmonary arterial hypertension.

15. The compound according to any one of claims 1 to 8 or a pharmaceutically acceptable salt thereof for use in the treatment or prophylaxis of a disease selected from the group consisting of diabetes, insulin resistance syndrome, metabolic syndrome, hyperglycemia, hyperlactacidemia, diabetic complication, cardiac failure, cardiomyopathy, myocardial ischemia, myocardial infarction, angina pectoris, dyslipidemia, atherosclerosis, peripheral arterial disease, intermittent claudication, chronic obstructive pulmonary disease, brain ischemia, cerebral apoplexy, mitochondrial disease, mitochondrial encephalomyopathy, cancer, pulmonary hypertension, Alzheimer disease, vascular dementia, glaucoma, diabetic retinopathy, retinopathy of prematurity, retinal vein occlusion, ischemic optic neuropathy and chronic kidney disease.

16. The compound for use according to claim 15 or a pharmaceutically acceptable salt thereof, wherein the diabetes is type 1 diabetes or type 2 diabetes; or wherein the vascular dementia is a large-vessel type of vascular dementia or a small-vessel type of vascular dementia.

17. The compound for use according to claim 15 or a pharmaceutically acceptable salt thereof, wherein the cardiac failure is acute cardiac failure or chronic cardiac failure; or wherein the pulmonary hypertension is pulmonary arterial hypertension.
